# EUROPEAN PATENT APPLICATION

(11) **EP 3 670 501 A1**
(43) Date of publication of application: **24.06.2020**
(21) Application number: 18213079.9
(22) Date of filing: 17.12.2018
(51) Int. Cl.: C07D 249/08, A01N 43/653

(54) **SUBSTITUTED [1,2,4]TRIAZOLE COMPOUNDS AS FUNGICIDES**

(71) Applicant: BASF SE, 67056 Ludwigshafen am Rhein (DE)
(72) Inventor: Lohmann, Jan Klaas, 67056 Ludwigshafen (DE); Craig, Ian Robert, 67056 Ludwigshafen (DE); Brahm, Lutz, 67117 Limburgerhof (DE); Fehr, Marcus, 67117 Limburgerhof (DE); Weber, Anja, 67056 Ludwigshafen (DE); Seet, Michael, 67056 Ludwigshafen (DE); Mueller, Bernd, 67056 Ludwigshafen (DE); Wiebe, Christine, 67056 Ludwigshafen (DE); Winter, Christian Harald, 67056 Ludwigshafen (DE); Grote, Thomas, 67056 Ludwigshafen (DE); Rudolf, Georg Christoph, 67056 Ludwigshafen (DE); Grammenos, Wassilios, 67056 Ludwigshafen (DE); Nett, Markus, 67056 Ludwigshafen (DE)
(74) Representative: BASF IP Association

(57) **Abstract**

The present invention relates to substituted [1,2,4]triazole compounds of formula (I) as well as to the N-oxides and the salts thereof. It further relates to the use of said compounds for combating phytopathogenic fungi, to methods for combating phytopathogenic fungi, and to seeds coated with at least one such compound. The invention also relates to processes for preparation of these compounds, intermediates, processes for preparation of such intermediates, and to compositions comprising at least one said substituted [1,2,4]triazole compound of formula (I).

## Description

The present invention relates to substituted [1,2,4]triazole compounds as well as to the N-oxides and the salts thereof. It further relates to the use of said compounds for combating phytopathogenic fungi, to methods for combating phytopathogenic fungi, and to seeds coated with at least one such compound. The invention also relates to processes for preparation of these compounds, intermediates, processes for preparation of such intermediates, and to compositions comprising at least one said substituted [1,2,4]triazole compound.

In many cases, in particular at low application rates, the fungicidal activity of known fungicidal compounds is unsatisfactory. Based on this, it was an object of the present invention to provide compounds having improved activity and/or a broader activity spectrum against phytopathogenic harmful fungi.

Surprisingly, this object is achieved by the substituted [1,2,4]triazol compounds of formula (I) having favorable fungicidal activity against phytopathogenic fungi.

Thus, the present invention relates to compounds of the formula (I) 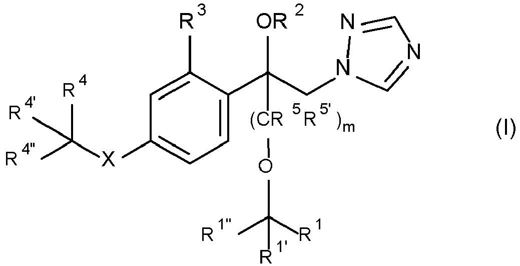 wherein
R¹, R^{1'} and R^{1"} are independently of one another selected from hydrogen, halogen, C₁-C₄-alkyl, C₁-C₄-halogenalkyl, C₂-C₄-alkenyl, C₂-C₄-halogenalkenyl, C₂-C₄-alkynyl and C₂-C₄-halogenalkynyl,
   provided that if any two of R¹, R^{1'} and R^{1"} are hydrogens, the third one is not hydrogen or not C₁-C₄-alkyl; and
   provided that if any two of R¹, R^{1'} and R^{1"} are halogens, the third one is not halogen;
R² is hydrogen, C₁-C₄-alkyl, C₂-C₄-alkenyl or C₂-C₄-alkynyl;
   wherein the aliphatic moieties of R² are unsubstituted or carry one, two, three or up to the maximum possible number of identical or different groups R^{2a}:
   - R^{2a}: is independently of one another selected from halogen, OH, CN, C₁-C₄-alkoxy, C₃-C₆-cycloalkyl, C₃-C₆-halogencycloalkyl and C₁-C₄-halogenalkoxy;
R³ is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein p is 0, 1 or 2, and
   wherein each of R³ is unsubstituted or further substituted by one, two, three or four R^{3a}:
   - R^{3a}: is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
R⁴, R^{4'}, and R^{4"} are independently of one another selected from hydrogen, halogen, C₁-C₆-alkyl, C₂-C₆-alkenyl, C₂-C₆-alkynyl, C₁-C₄-alkoxy, C₃-C₆-cycloalkyl, C₃-C₆-cycloalkenyl, C₃-C₆-cycloalkyl-C₁-C₄-alkyl, -N(R^{A})₂, C₃-C₆-halogencycloalkyl, aryl and aryloxy;
   wherein the aliphatic moieties of R⁴, R^{4'}, and R^{4"} are unsubstituted or further substituted by one, two, three or four of identical or different groups R^{4a}:
   - R^{4a}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is C₁-C₄-alkyl;
   wherein the cycloalkyl moieties of R⁴, R^{4'}, and R^{4"} are unsubstituted or carry one, two, three, four, five or up to the maximum number of identical or different groups R^{4b}:
   - R^{4b}: is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
   wherein the aryl and aryloxy moieties of R⁴, R^{4'}, and R^{4"} are unsubstituted or further substituted by one, two, three or four of identical or different groups R^{4c}:
   - R^{4c}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
   and wherein
   - R^{A}: is independently of one another selected from C₁-C₄-alkyl, C₁-C₄-halogenalkyl and -C(O)O-C₁-C₄-alkyl;
   or
R⁴ and R^{4'} together are =C(R^{4a})₂, and R" is as defined above;
R⁵ and R^{5'} are independently of one another selected from hydrogen or halogen;
m is 1 or 2;
X is O, S(O)n, wherein n is 0, 1 or 2, or NR^{N};
   - R^{N}: is selected from hydrogen, C₁-C₆-alkyl, C₂-C₆-alkenyl, C₂-C₆-alkynyl, C₁-C₆-alkoxy, -C(O)C₁-C₆-alkyl, C₃-C₆-cycloalkyl, C₃-C₆-cycloalkyl-C₁-C₄-alkyl, -S(O)₂- C₁-C₆-alkyl and -S(O)₂-aryl,
   wherein R^{N} is unsubstituted or further substituted by one, two, three or four of identical or different groups R^{Na}:
   - R^{Na}: is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and the N-oxides and the agriculturally acceptable salts thereof.

The compounds of formula (I) according to the present invention can be prepared by various routes known to the skilled person, for example as described in WO 2013/007767 or WO 2015/185708. There references describe substituted [1,2,4]triazole compounds, their fungicidal activity and preparation.

The compounds of formula (I) having the ether group -CH₂-O-CR¹R^{1'}R^{1"} can further be prepared, for example, as described below.

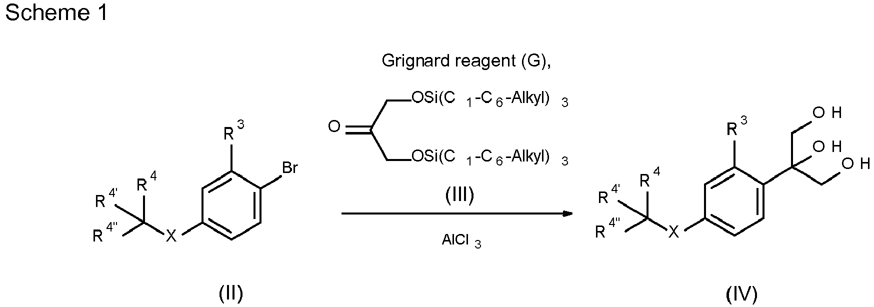

The compound (II) can be reacted with a Grignard reagent (G), such as iPrMgBr, followed by addition of a ketone (III), preferably under anhydrous conditions, to obtain compounds (IV), wherein R³, R⁴, R^{4'}, R^{4"} and X are as defined for compounds (I). A catalyst, such as CuCl₂, AlCl₃ or LiCl, can be optionally added. Before bringing the Grignard reagent (G) into the reaction, it can be contacted with LiCI thereby forming an addition product (G)·LiCl (e.g. iPrMgBr·LiCl). According to this alternative, (G)·LiCl is then used as a Grignard reagent. The use of LiCI together with Grignard reagents is generally known in the art, see for example Angew. Chem. Int. Ed. 2004, 43, 3333 and Angew. Chem. Int. Ed. 2006, 45, 159.

Compounds (II) are commercially available or can be prepared according to the methods known in the art.

Compounds (III) can be prepared by reacting dihydroxyacetone with a respective trialkylsilyl chloride (C₁-C₆-Alkyl)₃SiCl (V) as known to a skilled person. Trialkylsilylchlorides (V) are commetrially available or can be prepared according to methods known in the art.

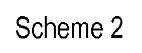

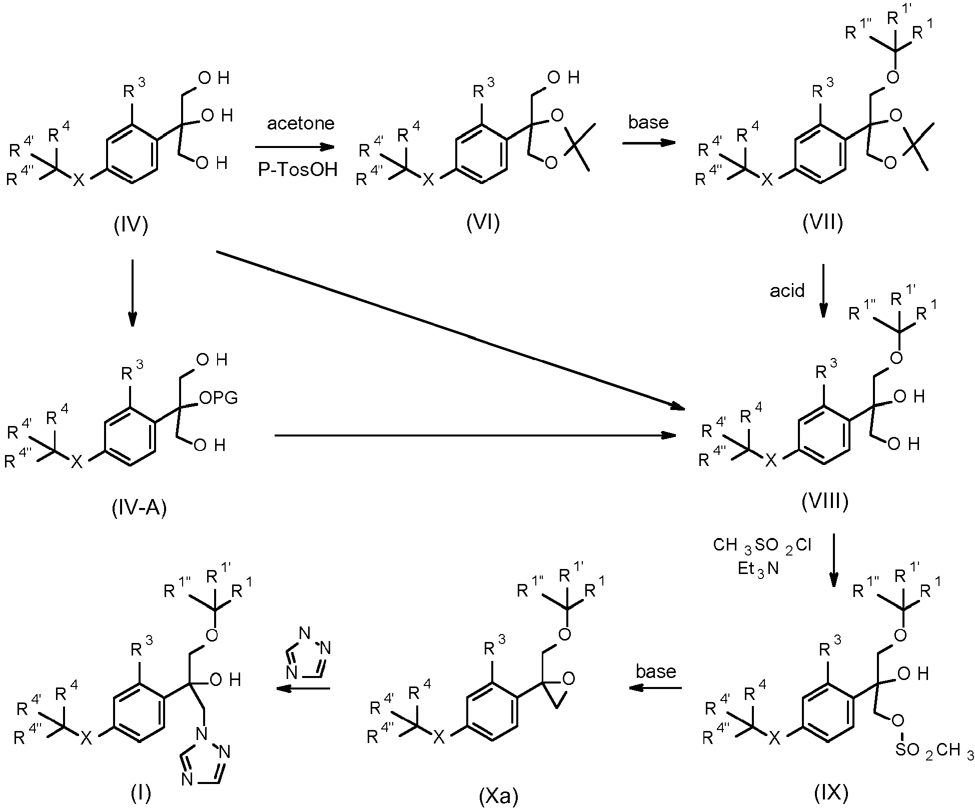

The compound (IV) can be converted into a cyclic dioxolane (VI) by reacting it with a respective ketone, e.g. acetone. The reaction can be accelerated by a strong acid, such as p-toluoenesulfonic acid. The compound (VI) is then converted to an ether (VII) followed by a ring opening reaction to give the compound (VIII). Both steps can be carried out according to methods known in the art. For example, the alcohol (VI) can be reacted with a respective halide Hal-C(R¹R^{1'}R^{1"}), wherein R¹, R^{1'} and R^{1"} are as defined for compounds (I), in the presence of a base. The ring opening can be carried out in acidic conditions, for ecample, in the presence of HCI or trifluoroacetic acid.

The compounds (VIII) can be also prepared by reacting compound (IV) or mono-protected compound (IV-A), wherein PG is a protecting group, with a suitable haloalkylation reagent. Examples of such reactions are known from CN 102701919 (reaction with tetrafluoroethylene), JP 2007332060 (reaction with tetrafluorodibromoethane), CN107235878 (reaction with a difluoromethylation agent). Protective groups such as ethers, esters, acetals, silyl ethers and many others are known in literature(Journal of Fluorine Chemistry, 136, 20-25, 2012; WO 2017/108723, WO 2016/123706, Angewandte Chemie, International Edition, 55(31), 9050-9054, 2016). Consecutively the protective group can be removed to get compounds (VIII).

Thereafter, the compound (VIII) can be reacted, for example, with methanesulfonyl chloride to give the monomesilate (IX) which in its turn is convented into the epoxide (X). Finaly, the reaction of the epoxide (X) with 1H-1,2,4-triazole gives the respective compound (I).

The compounds of formula (I) having the ether group -C(R⁵R^{5'})₂-O-CR¹R^{1'}R^{1"}, wherein at least one of R⁵ and R^{5'} is halogen, can further be prepared, for example, as depicted in Scheme 3.

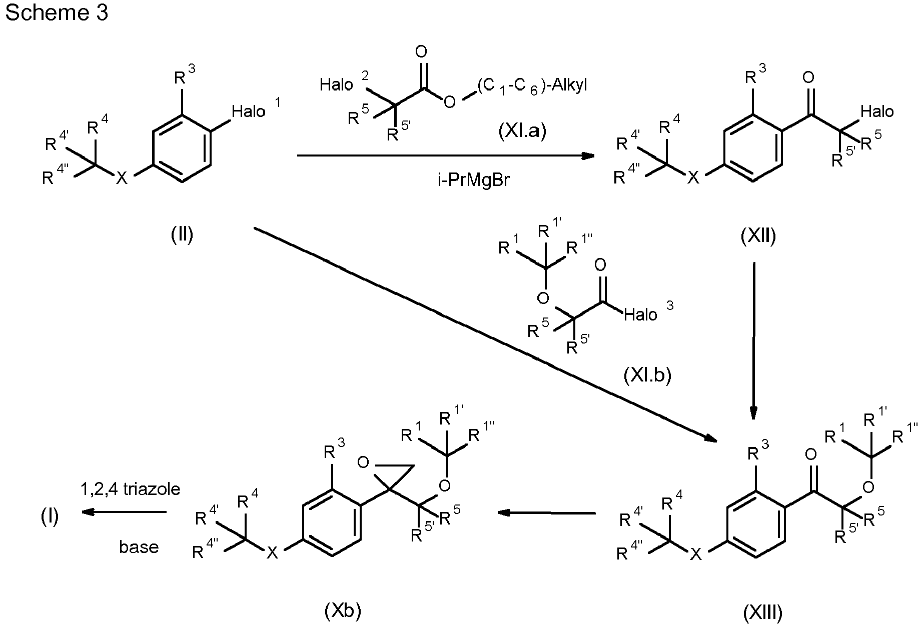

The compound (II), wherein R³, R⁴, R^{4'}, R^{4"} and X are as defined for compounds (I) and Halo¹ is a halogen selected from Br, Cl or I, can be reacted with a Grignard reagent (G), such as iPrMgBr, followed by addition of an ester (XI.a), wherein R⁵ and R^{5'} one or both are halogens as defined for compound (I) and Halo² is a halogen different from R⁵ and/or R⁵'. Halo² is preferably Br or Cl. The reaction is preferably carried out under anhydrous conditions and optionally in the presence of a catalyst, such as CuCl₂, AlCl₃ or LiCI, to obtain compounds (XII). Thereafter, the haloalkyl ketone (XII) is converted to the haloalkoxy ketone (XIII), e.g. using a respective alkoxide.

Alternatively compound (XIII) can be prepared by reacting compound (II) with compounds (XI.b), wherein R⁵ and R^{5'} one or both are halogens as defined for compound (I) and Halo³ is a halogen different from R⁵ and/or R^{5'}. Halo³ is preferably Br or Cl.

The epoxidation of compound (XIII) can be carried out using trimethylsulfonium methylsulfate. The subsequent reaction with 1,2,4 triazole and the base leads to the respective compounds (I). Theese two reactions are known from e.g. WO 2014/108286.

The N-oxides may be prepared from the compounds (I) according to conventional oxidation methods, e. g. by treating compounds (I) with an organic peracid such as metachloroper-benzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If individual compounds (I) cannot be obtained by the routes described above, they can be prepared by derivatization of other compounds (I).

If the synthesis yields mixtures of isomers, a separation is generally not necessarily required since in some cases the individual isomers can be interconverted during workup for use or during application (e. g. under the action of light, acids or bases). Such conversions may also take place after use, e. g. in the treatment of plants in the treated plant, or in the harmful fungus to be controlled.

Compounds (I) comprise chiral centers and they are generally obtained in the form of racemates. The R- and S-enantiomers of the compounds according to the invention can be separated and isolated in pure form with methods known by the skilled person, e.g. by using chiral HPLC. Both R- and S-enantiomers are fungicidally active.

The intermediate compounds (IV), (VI), (VII), (VIII), (IX) and (X) are new and are also subject matter of the present invention. A skilled person will readily understand that the preferences for the substituents, also in particular the ones given in the tables below for the respective substituents, given herein in connection with compounds (I) apply for the intermediates accordingly. Thereby, the substituents in each case have independently of each other or more preferably in combination the meanings as defined herein.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. A preferred embodiment of a C₁-C₆-alkyl is a C₂-C₄-alkyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-haloalkyl" or "C₁-C₆-halogenalkyl" refers to an alkyl group having 1 or 6 carbon atoms as defined above, wherein some or all of the hydrogen atoms in these groups may be replaced by halogen atoms as mentioned above. A preferred embodiment of a C₁-C₆-haloalkyl is a C₁-C₂-haloalkyl. Representative C₁-C₂-haloalkyl groups include chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein one or more of the hydrogen atoms in said alkyl group is replaced by an OH group. Representative C₁-C₆-hydroxyalkyl groups include hydroxymethyl, hydroxyethyl, hydroxypropyl and hydroxybutyl groups, and especially hydroxymethyl, 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

The term "C₂-C₆-alkenyl" refers to a straight-chain or branched unsaturated hydrocarbon radical having 2 to 6 carbon atoms and at least one double bond in any position. A preferred embodiment of a C₂-C₆-alkenyl is a C₂-C₄-alkenyl, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond. A preferred embodiment of a C₂-C₆-alkynyl is a C₂-C₄-alkynyl, such as ethynyl, prop-1-ynyl (-C≡C-CH₃), prop-2-ynyl (propargyl), but-1-ynyl, but-2-ynyl, but-3-ynyl, 1-methyl-prop-2-ynyl.

The term "C₂-C₄-haloalkenyl" or "C₂-C₄-halogenalkenyl" refers to an alkenyl group having 2 or 4 carbon atoms as defined above, wherein some or all of the hydrogen atoms in these groups may be replaced by halogen atoms as mentioned above. A preferred embodiment of a C₂-C₄-haloalkenyl is a C₂-C₃-haloalkenyl. Representative C₂-C₃-haloalkenyl groups include 1-F-ethenyl, 1-Cl-ethenyl, 2,2-di-F-ethenyl, 2,2-di-Cl-ethenyl, 3,3-di-F-prop-2-en-1-yl and 3,3-di-Cl-prop-2-en-1-yl, 2-Cl-allyl (-CH₂-CCl=CH₂), 2-Br-allyl (-CH₂-CBr=CH₂), 2-(CF₃)-allyl (-CH₂-C(CF₃)=CH₂), 3-Cl-allyl (-CH₂-CH=CClH), 3-Br-allyl (-CH₂-CH=CBrH), 3-(CF₃)-allyl (-CH₂-CH=C(CF₃)H).

The term "C₂-C₄-haloalkynyl" or "C₂-C₄-halogenalkynyl" refers to an alkynyl group having 2 or 4 carbon atoms as defined above, wherein some or all of the hydrogen atoms in these groups may be replaced by halogen atoms as mentioned above. A preferred embodiment of a C₂-C4-haloalkynyl is a C₂-C₃-haloalkynyl. Representative C₂-C₃-haloalkynyl groups include F-ethynyl, Cl-ethynyl, Br-ethynyl, Br-prop-2-ynyl (-CH₂-C≡C-Br) and Cl-prop-2-ynyl (-CH₂-C≡ C-CI).

The term "C₃-C₆-cycloalkyl" refers to monocyclic saturated hydrocarbon radicals having 3 to 6 carbon ring members, such as cyclopropyl, cyclobutyl, cyclopentyl, or cyclohexyl.

The term "C₃-C₆-cycloalkenyl" refers to monocyclic unsaturated, non-aromatic hydrocarbon radicals having 3 to 6 carbon ring members, such as cyclopropenyl, cyclobutenyl, cyclopentenyl, or cyclohexenyl.

The term "C₃-C₆-cycloalkyl-C₁-C₄-alkyl" refers to alkyl having 1 to 4 carbon atoms (as defined above), wherein one hydrogen atom of the alkyl radical is replaced by a cycloalkyl radical having 3 to 6 carbon atoms (as defined above).

The term "C₃-C₆-halogencycloalkyl" refers to monocyclic saturated hydrocarbon radicals having 3 to 6 carbon ring members as defined above, wherein some or all of the hydrogen atoms in these groups may be replaced by halogen atoms as mentioned above.

The term "C₃-C₆-cycloalkyloxy" refers to monocyclic saturated hydrocarbon radicals having 3 to 6 carbon ring members as defined above attached to a terminal oxygen atom, i.e., the moiety -O-C₃-C₆-cycloalkyl.

The term "C₁-C₆-alkoxy" refers to a straight-chain or branched alkyl group having 1 to 6 carbon atoms which is bonded via an oxygen at any position in the alkyl group. Examples are "C₁-C₄-alkoxy" groups, such as methoxy, ethoxy, n-propoxy, 1-methylethoxy, butoxy, 1-methy¬propoxy, 2-methylpropoxy or 1,1-dimethylethoxy.

The term "C₁-C₆-haloalkoxy" or "C₁-C₆-halogenalkoxy" refers to a C₁-C₆-alkoxy radical as defined above, wherein some or all of the hydrogen atoms in these groups may be replaced by halogen atoms as mentioned above. A preferred embodiment of a C₁-C₆-haloalkoxy is a C₁-C₄-haloalkoxy. Examples of C₁-C₄-haloalkoxy groups include substituents, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, OCF₂CHF₂, OCHF-CF₃, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C(O)O-(C₁-C₄)-alkyl" refers to an ester radical which is attached through the carbon atom of the group C(=O).

The term "aliphatic" or "aliphatic group" is to be understood to refer to a non-cyclic compound, substituent or residue composed of hydrogen and carbon atoms only, and it may be saturated or unsaturated, as well as linear or branched. An aliphatic compound, substituent or residue is non-aromatic and does not comprise any possibly given substitutions of the hydrogen atoms, however, it may be optionally substituted where indicated. Examples of an aliphatic compound, substituent or residue comprises alkyl, alkenyl, and alkynyl, all with a variable number of carbon atoms, but does not include hydrogen itself.

The term "cyclo-aliphatic" or "cyclo-aliphatic group" is to be understood to refer to a cyclic compound, substituent or residue composed of hydrogen and carbon atoms only, and it may be saturated or unsaturated. A cyclo-aliphatic compound, substituent or residue is non-aromatic and does not comprise any possibly given substitutions of the hydrogen atoms, however, it may be optionally substituted where indicated. Examples of a cyclo-aliphatic compound, substituent or residue comprises cycloalkyl, cycloalkenyl and cycloalkynyl, all with a variable number of carbon atoms, but does not include hydrogen itself.

The term "carbocycle" refers to a saturated or partially unsaturated 3-, 4- 5-, 6- or 7-membered carbocycle.

The term "saturated or partially unsaturated 3-, 4- 5-, 6- or 7-membered carbocycle" is to be understood as meaning both saturated or partially unsaturated carbocycles being composed of hydrogen and carbon atoms having 3, 4, 5, 6 or 7 ring members. Examples include cyclopropyl, cyclopropenyl, cyclobutyl, cyclobutenyl, cyclopentyl, cyclopentenyl, cyclopentadienyl, cyclohexyl, cyclohexenyl, cyclohexadienyl, cycloheptyl, cycloheptenyl, cycloheptadienyl, and the like. When substituted with one or more substituent(s), the any of the hydrogen atoms in the carbocycle may be replaced by said substituent(s), with the number of hydrogen atoms in the carbocycle being the maximum number of substituents.

The term "heterocycle" or "heterocyclyl" refers to a saturated or partially unsaturated 3-, 4-, 5-, 6-, or 7-membered heterocycle, wherein the ring member atoms of the heterocycle include besides carbon atoms 1, 2, 3 or 4 heteroatoms independently selected from the group of N, O and S, where S atoms as ring members may be present as S, SO or SO₂. It should be noted that the term heterocycle does not comprise aromatic residues.

The term "saturated or partially unsaturated 3-, 4-, 5-, 6-, or 7-membered heterocycle, wherein the ring member atoms of the heterocycle include besides carbon atoms 1, 2, 3 or 4 heteroatoms independently selected from the group of N, O and S", is to be understood as meaning both saturated and partially unsaturated heterocycles, for example:
a 3- or 4-membered saturated heterocycle which contains 1 or 2 heteroatoms from the group consisting of N, O and S as ring members, such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of N, O and S as ring members, such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or -4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "aryl" is to be understood to include mono-, bi- or tricyclic aromatic radicals having usually from 6 to 14, preferably 6, 10 or 14 carbon atoms. Exemplary aryl groups include phenyl, naphthyl, phenanthryl, anthracenyl, indenyl, azulenyl, biphenyl, biphenylenyl, and fluorenyl groups, more preferably phenyl, naphthyl, and biphenyl groups. Phenyl is preferred as aryl group.

The term "aryloxy" refers to an aryl radical as defined above attached to a terminal oxygen atom, i.e., the moiety -O-aryl.

If any of the variables is optionally substituted, it is understood that this applies to moieties containing carbon-hydrogen bonds, wherein the hydrogen atom is substituted by the corresponding substituent, however, not to moieties such as hydrogen, halogen, CN or the like. As an exemplary embodiment, if methyl is substituted by OH, a hydroxymethyl group is generated.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled. All different types of isomers are comprised by the compounds of formula (I), in particular enantiomers, diasteriomers or geometric isomers, and they all form part of the subject matter of the present invention.

Depending on the substitution pattern, the compounds of formula (I) and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds of formula (I) also apply to the intermediates.
- R¹, R^{1'} and/or R^{1"}: are independently of one another selected from hydrogen, halogen, C₁-C₄-alkyl, C₁-C₄-halogenalkyl, C₂-C₄-alkenyl, C₂-C₄-halogenalkenyl, C₂-C₄-alkynyl, or C₂-C₄-halogenalkynyl;
provided that if any two of R¹, R^{1'} and R^{1"} are hydrogens, the third one is not hydrogen or alkyl; and
provided that if any two of R¹, R^{1'} and R^{1"} are halogens, the third one is not halogen;
- R¹, R^{1'} and/or R^{1"}: preferably are independently of one another selected from hydrogen, halogen, C₁-C₄-halogenalkyl, C₂-C₄-halogenalkenyl, or C₂-C₄-halogenalkynyl,
provided that if any two of R¹, R^{1'} and R^{1"} are hydrogens, the third one is not hydrogen; and
provided that if any two of R¹, R^{1'} and R^{1"} are halogens, the third one is not halogen;
- R¹,: R^{1'} and/or R^{1"} more preferably are independently of one another selected from hydrogen, halogen, or C₁-C₂-halogenalkyl,
provided that if any two of R¹, R^{1'} and R^{1"} are hydrogens, the third one is not hydrogen; and
provided that if any two of R¹, R^{1'} and R^{1"} are halogens, the third one is not halogen;

According to one embodiment, two of R¹, R^{1'} and/or R^{1"} are hydrogens, and the tird one is halogen, such as F, Cl or Br; preferably F or Cl, most preferably F,

According to one embodiment, two of R¹, R^{1'} and/or R^{1"} are halogens, such as F, Cl or Br; preferably F or Cl, most preferably F, and the third one is hydrogen.

According to another embodiment, any one of R¹, R^{1'} and/or R^{1"} is C₁-C₄-alkyl, such as CH₃, C₂H₅, C₃H₇ (n-propyl), CH(CH₃)₂ (iso-propyl), CH₂CH(CH₃)₂ (iso-butly) or C(CH₃)₃ (tert-butyl); preferably C₁-C₃-alkyl, more preferably C₁-C₂-alkyl and two others of R¹, R^{1'} and R^{1"} are not hydrogens.

According to another embodiment, R¹, R^{1'} and/or R^{1"} is C₁-C₄-halogenalkyl, such as CF₃ or CHF₂, CF₂CH₃, CH₂CF₃, CHFCH₃ or CF₂CF₃; preferably C₁-C₃-halogenalkyl, more preferably C₁-C₂-halogenalkyl. Particularly preferred R¹ is CF₃ or CHF₂, expetially preferred is CHF₂.

According to another embodiment, R¹, R^{1'} and/or R^{1"} is C₂-C₄-alkenyl, such as CH=CH₂, CH₂CH=CH₂, CH=CHCH₃ or C(CH₃)=CH₂; preferably C₂-C₃-alkenyl, more preferably C₂-alkenyl.

According to another embodiment, R¹, R^{1'} and/or R^{1"} is C₂-C₄-halogenalkenyl, such as CH=CHF, CH=CF₂, C(F)=CH₂, C(F)=CHF, C(F)=CF₂, CH=CHCl, CH=CCl₂, C(Cl)=CH₂, C(Cl)=CHCl, C(Cl)=CCl₂, preferably C₂-C₃-halogenalkenyl, more preferably C₂-halogen-alkenyl.

According to another embodiment, R¹, R^{1'} and/or R^{1"} is from C₂-C₄-alkynyl, such as C≡CH, C≡CCH₃, CH₂-C≡C-H or CH₂-C≡C-CH₃; preferably C₂-C₃-alkynyl, more preferably C₂-alkenyl.

According to another embodiment, R¹, R^{1'} and/or R^{1"} is C₂-C₄-halogenalkynyl, such as C≡CCl, C≡CBr, C≡C-I, CH₂C≡CCl, CH₂C≡CBr preferably C₂-C₃-halogenalkynyl, more preferably C₂-halogenalkenyl.

The selection of each of the three variables R¹, R^{1'} and/or R^{1"} is made independent of one another, and R¹, R^{1'} and/or R^{1"} may be identical or different with respective provisios as indicated above.

Particularly preferred embodiments of R¹, R^{1'} and/or R^{1"} according to the invention are given in Table P1 below, wherein each line of lines P1-1 to P1-25 corresponds to one particular embodiment of the invention, wherein P1-1 to P1-25 are also in any combination with respective provisios a preferred embodiment of the present invention.

**Table P1:**

| **line** | R¹, R^{1'} and/or R^{1"} |
|---|---|
| P1-1 | H |
| P1-2 | F |
| P1-3 | Cl |
| P1-4 | Br |
| P1-5 | CF₃ |
| P1-6 | CHF₂ |
| P1-7 | CH₂F |
| P1-8 | CHCl₂ |
| P1-9 | CH₂Cl |
| P1-10 | CF₂CH₃ |
| P1-11 | CF₂CF₃ |
| P1-12 | CF₂CHF₂ |
| P1-13 | CH₂CF₃ |
| P1-14 | CH₂CHF₂ |
| P1-15 | CH=CHF |
| P1-16 | CH=CF₂ |
| P1-17 | C(F)=CH₂ |
| P1-18 | C(F)=CHF |
| P1-19 | C(F)=CF₂ |
| P1-20 | CH=CHCl |
| P1-21 | CH=CCl₂ |
| P1-22 | C(Cl)=CH₂ |
| P1-23 | C(Cl)=CHCl |
| P1-24 | C(Cl)=CCl₂ |
| P1-25 | C≡CCl |

In one embodiment of the present invention
R¹ is selected from hydrogen, C₁-C₄-alkyl, C₁-C₄-halogenalkyl, C₂-C₄-alkenyl, C₂-C₄-halogenalkenyl, C₂-C₄-alkynyl, C₂-C₄-halogenalkynyl; and
R^{1'} is selected from halogen, C₁-C₄-halogenalkyl, C₂-C₄-alkenyl, C₂-C₄-halogenalkenyl, C₂-C₄-alkynyl, C₂-C₄-halogenalkynyl; and
R^{1"} is selected from hydrogen, halogen, C₁-C₄-alkyl, C₁-C₄-halogenalkyl, C₂-C₄-alkenyl, C₂-C₄-halogenalkenyl, C₂-C₄-alkynyl, C₂-C₄-halogenalkynyl;

In another embodiment of the present invention
R¹ is selected from hydrogen, C₁-C₄-halogenalkyl, C₂-C₄-halogenalkenyl, C₂-C₄-halogenalkynyl; and
R^{1'} is selected from halogen, C₁-C₄-halogenalkyl, C₂-C₄-halogenalkenyl, C₂-C₄-halogenalkynyl; and
R^{1"} is selected from hydrogen, halogen, C₁-C₄-halogenalkyl, C₂-C₄-halogenalkenyl, C₂-C₄-halogenalkynyl;

In another embodiment of the present invention
R¹ is selected from hydrogen, or C₁-C₂-halogenalkyl; and
R^{1'} is selected from halogen, or C₁-C₂-halogenalkyl; and
R^{1"} is selected from hydrogen, halogen, or C₁-C₂-halogenalkyl;

In another embodiment of the present invention
R¹ is selected from hydrogen, or C₁-C₂-halogenalkyl; and
R^{1'} is selected from halogen, or C₁-C₂-halogenalkyl; and
R^{1"} is selected from hydrogen, or halogen;

In another embodiment of the present invention
R¹ is hydrogen, or C₁-C₂-halogenalkyl; and
R^{1'} is selected from halogen; and
R^{1"} is selected from halogen.

Particularly preferred combinations of R¹, R^{1'} and R^{1"}according to the invention are in Table P2 below, wherein each line of lines P2-1 to P2-26 corresponds to one particular embodiment of the invention.

**Table P1':**

| **line** | **R¹** | **R^{1'}** | **R^{1"}** |
|---|---|---|---|
| P-1 | H | F | H |
| P-2 | H | F | F |
| P-3 | H | CF₃ | H |
| P-4 | CF₃ | F | H |
| P-5 | CF₃ | F | F |
| P-6 | H | CHF₂ | H |
| P-7 | CHF₂ | F | H |
| P-8 | CHF₂ | F | F |
| P-9 | H | CH₂F | H |
| P-10 | CH₂F | F | H |
| P-11 | CH₂F | F | F |
| P-12 | H | CF₂CH₃ | H |
| P-13 | CF₂CH₃ | F | H |
| P-14 | CF₂CH₃ | F | F |
| P-15 | H | CF₂CF₃ | H |
| P-16 | CF₂CF₃ | F | H |
| P-17 | CF₂CF₃ | F | F |
| P-18 | H | CF₂CHF₂ | H |
| P-19 | CF₂CHF₂ | F | H |
| P-20 | CF₂CHF₂ | F | F |
| P-21 | H | CH₂CF₃ | H |
| P-22 | CH₂CF₃ | F | H |
| P-23 | CH₂CF₃ | F | F |
| P-24 | H | CH₂CHF₂ | H |
| P-25 | CH₂CHF₂ | F | H |
| P-26 | CH₂CHF₂ | F | F |

R² according to the invention is selected from hydrogen, C₁-C₄-alkyl, C₂-C₄-alkenyl, C₂-C₄-alkynyl; preferably from hydrogen or C₁-C₄-alkyl;
wherein the aliphatic moieties of R² are unsubstituted or carry one, two, three or up to the maximum possible number of identical or different groups R^{2a} which independently of one another are selected from halogen, OH, CN, C₁-C₄-alkoxy, C₃-C₆-cycloalkyl, C₃-C₆-halocycloalkyl and C₁-C₄-halogenalkoxy, preferably from halogen, OH, C₃-C₆-cycloalkyl, C₃-C₆-halocycloalkyl and C₁-C₄-halogenalkoxy.

According to one embodiment, R² is H. This is the preferred embodiment.

According to another embodiment, R² is selected from C₁-C₄-alkyl, C₂-C₄-alkenyl, C₂-C₄-alkynyl which are unsubstituted or carry one, two, three or up to the maximum possible number of identical or different groups R^{2a} as defined herein.

According to another embodiment, R² is C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R² is C₁-C₄-alkyl, that carry one, two or three or up to the maximum possible number of identical or different groups R^{2a}, as defined herein. According to a specific embodiment thereof, R² is C₁-C₄-halogenalkyl, more particularly C₁-C₂-halogenalkyl. According to a further specific embodiment thereof, R² is C₁-C₄-alkoxy-C₁-C₄-alkyl, such as CH₂OCH₃ or CH₂CH₂OCH₃. According to still a further specific embodiment thereof, R² is hydroxyl-C₁-C₄-alkyl, such as CH₂CH₂OH. Further specific embodiments thereof can be found in the below Table P2.

According to still another embodiment, R² is C₃-C₆-cycloalkyl-C₁-C₄-alkyl. A further embodiment relates to compounds, wherein R² is C₃-C₆-cycloalkyl-C₁-C₄-alkyl, more particularly C₃-C₆-cycloalkyl-C₁-C₂-alkyl, that is substituted by one, two or three or up to the maximum possible number of identical or different groups R^{2a}. According to a specific embodiment thereof, R² is C₃-C₆-halocycloalkyl-C₁-C₄-alkyl, more particularly C₃-C₆-halocycloalkyl-C₁-C₂-alkyl. Further specific embodiments thereof can be found in the below Table P2.

According to another embodiment, R² is C₂-C₄-alkenyl, such as CH₂CH=CH₂, CH₂C(CH₃)=CH₂ or CH₂CH=CHCH₃. A further embodiment relates to compounds, wherein R² is C₂-C₄-alkenyl that is substituted by one, two or three or up to the maximum possible number of identical or different groups R^{2a} as defined and preferably defined herein. According to a specific embodiment thereof, R² is C₂-C₄-halogenalkenyl, such as CH₂C(Cl)=CH₂ and CH₂C(H)=CHCl. According to a further specific embodiment thereof, R² is C₃-C₆-cycloalkyl-C₂-C₄-alkenyl or C₃-C₆-halocycloalkyl-C₂-C₄-alkenyl. Further specific embodiments thereof can be found in the below Table P2.

According to still another embodiment, R² is C₂-C₄-alkynyl, such as CH₂C≡CH or CH₂C≡CCH₃.

A further embodiment relates to compounds, wherein R² is C₂-C₄-alkynyl that is substituted by one, two or three or up to the maximum possible number of identical or different groups R^{2a}, as defined and preferably defined herein. According to a specific embodiment thereof, R² is C₂-C₄-halogenalkynyl. According to a further specific embodiment thereof, R² is C₃-C₆-cycloalkyl-C₂-C₄-alkynyl or C₃-C₆-halocycloalkyl-C₂-C₄-alkynyl. Further specific embodiments thereof can be found in the below Table P2.

Particularly preferred embodiments of R² according to the invention are in Table P2 below, wherein each line of lines P2-1 to P2-79 corresponds to one particular embodiment of the invention, wherein P2-1 to P2-79 are also in any combination a preferred embodiment of the present invention.

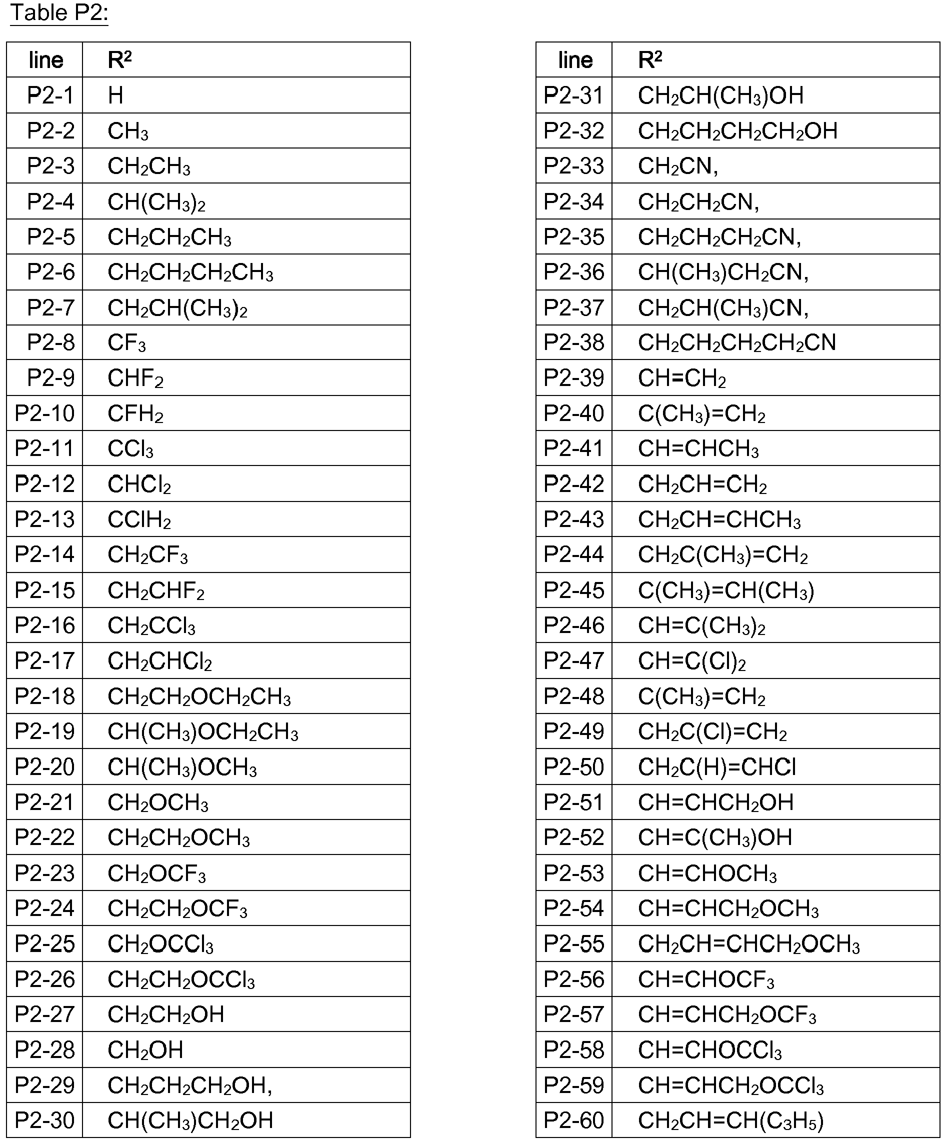

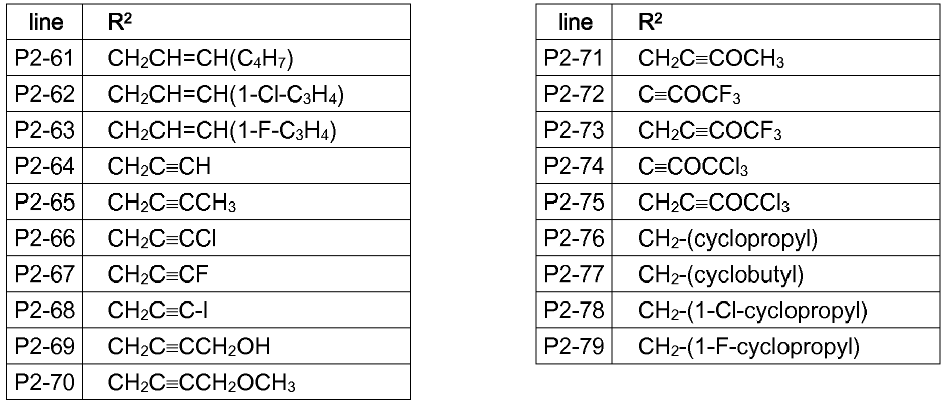

R³ according to the present invention is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl); preferably from halogen, C₁-C₄-alkyl, C₁-C₄-alkoxy and S(O)ₚ(C₁-C₄-alkyl), more preferably from halogen or C₁-C₄-alkyl;
wherein each of R³ is unsubstituted or further substituted by one, two, three or four R^{3a};
wherein R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy, and wherein p is 0, 1 or 2.

According one embodiment R³ is halogen, such as F, Cl or Br.

According another embodiment R³ is CN.

According to another embodiment R³ is C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R³ is C₁-C₄-alkyl, that carry one, two or three or up to the maximum possible number of identical or different groups R^{3a}, as defined herein. According to a specific embodiment thereof, R³ is C₁-C₄-halogenalkyl, preferably C₁-C₂-halogenalkyl, such as CF₃, CHF₂, CH₂F, CCl₃, CHCl₂ or CH₂Cl. Further specific embodiments thereof can be found in the below Table P3.

According to another embodiment R³ is C₁-C₄-alkoxy, preferably C₁-C₂-alkoxy, such as OCH₃, OC₂H₅, OCH(CH₃)₂. A further embodiment relates to compounds, wherein R³ is C₁-C₄-alkoxy, preferably C₁-C₂-alkoxy that carry one, two or three or up to the maximum possible number of identical or different groups R^{3a}, as defined herein. According to a specific embodiment thereof, R³ is C₁-C₄-halogenalkoxy, preferably C₁-C₂-halogenalkoxy, such as OCH₂F, OCHF₂, OCF₃, OCH₂Cl, OCHCl₂, OCCl₃. Further specific embodiments thereof can be found in the below Table P3.

According to another embodiment R³ is C₂-C₄-alkenyl, such as CH=CH₂, CH₂CH=CH₂, CH=CHCH₃ or C(CH₃)=CH₂. A further embodiment relates to compounds, wherein R³ is C₂-C₄-alkenyl that carry one, two or three or up to the maximum possible number of identical or different groups R^{3a}, as defined herein. According to a specific embodiment thereof, R³ is C₂-C₄-halogenalkenyl, such as CH=CHF, CH=CF₂, C(F)=CH₂, C(F)=CHF, C(F)=CF₂, CH=CHCl, CH=CCl₂, C(Cl)=CH₂, C(Cl)=CHCl, C(Cl)=CCl₂. Further specific embodiments thereof can be found in the below Table P3.

According to another embodiment R³ is C₂-C₄-alkynyl, such as C=CH. A further embodiment relates to compounds, wherein R³ is C₂-C₄-alkynyl that carry one, two or three or up to the maximum possible number of identical or different groups R^{3a}, as defined herein. According to a specific embodiment thereof, R³ is C₂-C₄-halogenalkynyl, such as C≡CCl, C≡CBr, C≡C-I. Further specific embodiments thereof can be found in the below Table P3.

According to another embodiment R³ is C₃-C₆-cycloalkyl, such as cyclopropyl, cyclobutyl, cyclopentyl, cyclohexyl. A further embodiment relates to compounds, wherein R³ is C₃-C₆-cycloalkyl that carry one, two or three or up to the maximum possible number of identical or different groups R^{3a}, as defined herein. Further specific embodiments thereof can be found in the below Table P3.

According to another embodiment R³ is S(O)ₚ(C₁-C₄-alkyl), more specifically S(C₁-C₄-alkyl), S(O)(C₁-C₄-alkyl) or S(O)₂(C₁-C₄-alkyl); preferably S(C₁-C₂-alkyl), S(O)(C₁-C₂-alkyl) and S(O)₂(C₁-C₂-alkyl). According to a particular embodiment thereof, R³ is selected from SCH₃, S(O)(CH₃) and S(O)₂(CH₃). Further specific embodiments thereof can be found in the below Table P3.

R^{3a} is selected from halogen, CN, OH, C₁-C₄-alkyl, C₁-C₄-halogenalkyl, C₃-C₆-cycloalkyl, C₃-C₈-halogencycloalkyl, C₁-C₄-alkoxy and C₁-C₄-halogenalkoxy, in particular selected from halogen, CN, C₁-C₂-alkyl, C₁-C₂-halogenalkyl, C₃-C₆-cycloalkyl, C₃-C₆-halogencycloalkyl, C₁-C₂-alkoxy and C₁-C₂-halogenalkoxy. Specifically, R^{3a} is independently selected from F, Cl, CN, OH, CH₃, halogenmethyl, cyclopropyl, halogencyclopropyl, OCH₃ and halogenmethoxy.

Particularly preferred embodiments of R³ according to the invention are in Table P3 below, wherein each line of lines P3-1 to P3-17 corresponds to one particular embodiment of the invention, wherein P3-1 to P3-17 are also in any combination with one another a preferred embodiment of the present invention. Thereby, for every R³ that is present in the inventive compounds, these specific embodiments and preferences apply independently of the meaning of any other R³ that may be present in the phenyl ring:

**Table P3:**

| **No.** | **R³** |
|---|---|
| P3-1 | H |
| P3-2 | Cl |
| P3-3 | F |
| P3-4 | Br |
| P3-5 | CN |
| P3-6 | CH₃ |
| P3-7 | CH₂CH₃ |
| P3-8 | CF₃ |
| P3-9 | CHF₂ |
| P3-10 | CH₂F |
| P3-11 | CCl₃ |
| P3-12 | CHCl₂ |
| P3-13 | CH₂Cl |
| P3-14 | OCH₃ |
| P3-15 | OCH₂CH₃ |
| P3-16 | OCF₃ |
| P3-17 | OCHF₂ |

R⁴, R^{4'}, and R^{4"} according to the present invention are independently of one another selected from hydrogen, halogen, C₁-C₆-alkyl, C₂-C₆-alkenyl, C₂-C₆-alkynyl, C₁-C₄-alkoxy, C₃-C₆-cycloalkyl, C₃-C₆-cycloalkenyl, C₃-C₆-cycloalkyl-C₁-C₄-alkyl, -N(R^{A})₂, C₃-C₆-halogencycloalkyl, aryl and aryloxy;
wherein the aliphatic moieties of R⁴, R^{4'}, and R^{4"} are unsubstituted or carry one, two, three or four of identical or different groups R^{4a}:
- R^{4a}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is C₁-C₄-alkyl;
wherein the cycloalkyl moieties of R⁴, R^{4'}, and R^{4"} are unsubstituted or carry one, two, three, four, five or up to the maximum number of identical or different groups R^{4b}:
- R^{4b}: is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
wherein the aryl and aryloxy moieties of R⁴, R^{4'}, and R^{4"} are unsubstituted or carry one, two, three or four of identical or different groups R^{4c}:
- R^{4c}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and wherein
- R^{A}: is independently of one another selected from C₁-C₄-alkyl, C₁-C₄-halogenalkyl and -C(O)O-C₁-C₄-alkyl;
or
R⁴ and R^{4'} together are =C(R^{4a})₂, and R" and R^{4a} are as defined above.

It should be noted that the selection of each of the three variables R⁴, R^{4'}, and R^{4"} is made independent of one another, and R⁴, R^{4'}, and R^{4"} may be identical or different.

According to one embodiment, R⁴, R^{4'}, and/or R^{4"} are hydrogen or halogen.

According to another embodiment, R⁴, R^{4'}, and/or R^{4"} are C₁-C₆-alkyl, such as CH₃, C₂H₅, C₃H₇, CH(CH₃)₂, CH₂CH(CH₃)₂ or C(CH₃)₃; preferably C₁-C₄-alkyl, more preferably C₁-C₃-alkyl. A further embodiment relates to compounds, wherein R⁴, R^{4'}, and/or R^{4"} are C₁-C₆-alkyl, preferably C₁-C₄-alkyl, more preferably C₁-C₃-alkyl, that carry one, two or three or up to the maximum possible number of identical or different groups R^{4a}, as defined herein. According to a specific embodiment thereof, R⁴, R^{4'}, and/or R^{4"} are C₁-C₆-halogenalkyl, preferably C₁-C₄-halogenalkyl, more preferably C₁-C₃-halogenalkyl, most preferably C₁-C₂-halogenalkyl such as CF₃, CF₂Br, CHF₂, CHFCI, CHFCF₃, CF₂CH₃, CF₂CHF₂, CH₂CF₃ or CF₂CF₃. According to a further specific embodiment thereof, R⁴, R^{4'}, and/or R^{4"} are C₁-C₄-alkoxy-C₁-C₆-alkyl, i.e., C₁-C₆-alkyl substituted by R^{4a} being C₁-C₄-alkoxy; preferably C₁-C₄-alkoxy-C₁-C₄-alkyl, such as CH₂-OCH₃. According to a further specific embodiment thereof, R⁴, R^{4'}, and/or R^{4"} are C₃-C₆-cycloalkyl-C₁-C₆-alkyl, i.e., C₁-C₆-alkyl substituted by R^{4a} being C₃-C₆-cycloalkyl; preferably C₃-C₆-cycloalkyl-C₁-C₄-alkyl, A further embodiment relates to compounds, wherein R⁴, R^{4'}, and/or R^{4"} are C₃-C₆-cycloalkyl-C₁-C₆-alkyl, preferably C₃-C₆-cycloalkyl-C₁-C₄-alkyl, that is substituted by one, two or three or up to the maximum possible number of identical or different groups R^{4a} in the alkyl moiety and/or substituted by one, two, three four or five or up to the maximum possible number of identical or different groups R^{4b} in the cycloalkyl moiety. R^{4a} and R^{4b} are in each case as defined herein. Further specific embodiments thereof can be found in the below Table P4.

According to another embodiment, one of R⁴, R^{4'}, and/or R^{4"} is methyl, ethyl or iso-propyl, preferably methyl. In another embodiment, one of R⁴, R^{4'}, and/or R^{4"} is methoxy or CF₃. In still another embodiment, two of R⁴, R^{4'}, and/or R^{4"} are methyl, and in another embodiment, all three residues R⁴, R^{4'}, and/or R^{4"} are methyl.

According to another embodiment, R⁴, R^{4'}, and/or R^{4"} is C₂-C₆-alkenyl, such as CH=CH₂, CH₂CH=CH₂, CH=CHCH₃ or C(CH₃)=CH₂, preferably C₂-C₄-alkenyl. A further embodiment relates to compounds, wherein R⁴, R^{4'}, and/or R^{4"} is C₂-C₆-alkenyl, preferably C₂-C₄-alkenyl, that carry one, two or three or up to the maximum possible number of identical or different groups R^{4a} as defined herein. According to a specific embodiment thereof, R⁴, R^{4'}, and/or R^{4"} is C₂-C₆-halogenalkenyl, preferably C₂-C₄-halogenalkenyl. Further specific embodiments thereof can be found in the below Table P4.

According to still another embodiment, R⁴, R^{4'}, and/or R^{4"} is C₂-C₆-alkynyl, such as C≡CH, C≡CCH₃, CH₂-C≡C-H or CH₂-C≡C-CH₃, preferably C₂-C₄-alkynyl. A further embodiment relates to compounds, wherein R⁴, R^{4'}, and/or R^{4"} is C₂-C₆-alkynyl, preferably C₂-C₄-alkynyl, that carry one, two or three or up to the maximum possible number of identical or different groups R^{4a}, as defined herein. According to a specific embodiment thereof, R⁴, R^{4'}, and/or R^{4"} is C₂-C₆-halogenalkynyl, preferably C₂-C₄-halogenalkynyl. According to a further specific embodiment thereof, R⁴, R^{4'}, and/or R^{4"} is C₃-C₆-cycloalkyl-C₂-C₆-alkynyl or C₃-C₆-halogencycloalkyl-C₂-C₆-alkynyl, preferably C₃-C₆-cycloalkyl-C₂-C₄-alkynyl or C₃-C₆-halogencycloalkyl-C₂-C₄-alkynyl. In particular, R⁴, R^{4'}, and/or R^{4"} is substituted C₂-C₄-alkynyl, such as C≡CCl, C≡CBr, C≡CSi(CH₃)₃. Further specific embodiments thereof can be found in the below Table P4.

According to still another embodiment, R⁴, R^{4'}, and/or R^{4"} is C₃-C₆-cycloalkyl, such as C₃H₅ (cyclopropyl), C₄H₇ (cyclobutyl), cyclopentyl or cyclohexyl. In a particular embodiment, one of R⁴, R^{4'}, and/or R^{4"} is cyclopropyl. A further embodiment relates to compounds, wherein R⁴, R^{4'}, and/or R^{4"} is C₃-C₆-cycloalkyl, such as C₃H₅ (cyclopropyl) or C₄H₇ (cyclobutyl), that is substituted by one, two, three four or five or up to the maximum possible number of identical or different groups R^{4b} as defined herein. According to a specific embodiment thereof, R⁴, R^{4'}, and/or R^{4 "} is C₃-C₆-halogencycloalkyl, such as halogencyclopropyl, in particular 1-F-cyclopropyl or 1-Cl-cyclopropyl. According to a further specific embodiment thereof, R⁴, R^{4'}, and/or R^{4"} is C₃-C₆-cycloalkyl-C₃-C₆-cycloalkyl, wherein each of said cycloalkyl-cycloalkyl moieties is unsubstituted or carries one, two or three R^{4b} as defined herein, such as 1-cyclopropyl-cyclopropyl or 2-cyclopropyl-cyclopropyl. Specific embodiments thereof can be found in the below Table P4.

In another embodiment, R⁴, R^{4'}, and/or R^{4"} is aryl, such as phenyl, naphthyl, and or a biphenyl group. In a particular embodiment, one or two of R⁴, R^{4'}, and/or R^{4"} is phenyl, preferably one of R⁴, R^{4'}, and/or R^{4"} is phenyl. A further embodiment relates to compounds, wherein R⁴, R^{4'}, and/or R^{4"} is aryl, such as phenyl, that is substituted by one, two, three four or five or up to the maximum possible number of identical or different groups R^{4c} as defined herein, such as phenyl substituted by F, Cl, CH₃, CF₃, CN, CO₂CH₃ or CHF₂. Specific embodiments thereof can be found in the below Table P4.

In another embodiment, R⁴, R^{4'}, and/or R^{4"} is aryloxy, such as phenoxy, or naphthoxy. In a particular embodiment, one or two of R⁴, R^{4'}, and/or R^{4"} is phenoxy, preferably one of R⁴, R^{4'}, and/or R^{4"} is phenoxy. A further embodiment relates to compounds, wherein R⁴, R^{4'}, and/or R^{4"} is aryloxy, such as phenoxy, that is substituted by one, two, three four or five or up to the maximum possible number of identical or different groups R^{4c} as defined herein, such as phenyl substituted by F, Cl, CH₃, CF₃, CN, CO₂CH₃ or CHF₂. Specific embodiments thereof can be found in the below Table P4.

In another embodiment, R⁴, R^{4'}, and/or R^{4"} is -N(R^{A})₂, wherein R^{A} is independently of one another selected from C₁-C₄-alkyl, C₁-C₄-halogenalkyl and -C(O)O-C₁-C₄-alkyl. In a specific embodiment, R^{A} is C₁-C₄-alkyl, such as methyl or ethyl. According to a preferred embodiment, all three substituents R^{A} are selected as methyl. Specific embodiments thereof can be found in the below Table P4.

Specifically, according to one preferred embodiment, R⁴, R^{4'}, and/or R^{4"} is independently selected from hydrogen, halogen, such as F, Cl or Br, C₁-C₄-alkyl, such as CH₃, C₂H₅, C₃H₇, CH(CH₃)₂, C(CH₃)₃, CH₂C(CH₃)₃ and CH₂CH(CH₃)₂, C₁-C₄-halogenalkyl, such as as CF₃, CF₂Br, CHF₂, CHFCl, CHFCF₃, CF₂CH₃, CF₂CHF₂, CH₂CF₃ or CF₂CF₃, C₁-C₄-halogenalkoxy, such as OCHF₂, OCF₂CHF₂, OCHCl-CF₃, OCF₂CHFCl and OCF₂CHFCF₃, C₂-C₄-alkenyl, such as CH=CH₂, -C(CH₃)=CH₂, -CH=C(CH₃)₂, -C(CH₃)=C(CH₃)₂, and -C(CH₃)=C(CH₃)H, C₂-C₃-halogenalkenyl, such as -CCl=CH₂, -CBr=CH₂, -C(CF₃)=CH₂, -C(H)=CCIH, -C(H)=CF₂, -C(H)=CCl₂, -C=CBrH and -C=C(CF₃)H, unsubstituted and substituted C₂-C₄-alkynyl, such as -C≡CH, -C≡CCH₃, -C≡CCl, -C≡CBr, -C≡CSi(CH₃)₃, and -C≡C(C₃H₅), and unsubstituted C₃-C₆-cycloalkyl, such as cyclopropyl, cyclobutyl and cyclopentyl, substituted C₃-C₆-cycloalkyl, such as 1-fluor-cyclopropyl and 1-chloro-cyclopropyl, unsubstituted C₃-C₆-cycloalkenyl, such as cyclopentenyl and cyclohexenyl, aryl, such as phenyl, or aryloxy, such as phenoxy.

In another embodiment, R⁴, R^{4'}, and/or R^{4"} is independently selected from hydrogen, F, Cl, Br, CH₃, C₂H₅, C₃H₇, CH(CH₃)₂, C(CH₃)₃, CH₂C(CH₃)₃, CH₂CH(CH₃)₂, CF₃, CF₂Br, CHF₂, CHFCl, CHFCF₃, CF₂CH₃, CF₂CHF₂, CH₂CF₃, CF₂CF₃ OCHF₂, OCF₂CHF₂, OCHCl-CF₃, OCF₂CHFCF₃, CH=CH₂, -C(CH₃)=CH₂, -CH=C(CH₃)₂, -C(CH₃)=C(CH₃)₂, -C(CH₃)=C(CH₃)H, -CCl=CH₂, -CBr=C H₂, -C(CF₃)=CH₂, -C=CClH, -C=CBrH, -C=C(CF₃)H, -C≡CH, -C≡CCH₃, -C≡CCl, -C≡CBr, -C≡CSi (CH₃)₃, -C≡C(C₃H₅), cyclopropyl, cyclobutyl, cyclopentyl, 1-fluor-cyclopropyl, 1-chloro-cyclopropyl, cyclopentenyl, cyclohexenyl, phenyl, and phenoxy.

In one embodiment, only one of R⁴, R^{4'}, and/or R^{4"} is hydrogen.

In another embodiment, at least one of R⁴, R^{4'}, and/or R^{4"} is methyl, preferably only one of R⁴, R^{4'}, and/or R^{4"} is methyl.

In still another embodiment, R⁴, R^{4'}, and/or R^{4"} are independently selected from hydrogen, halogen, C₁-C₄-alkyl, C₁-C₄-halogenalkyl, C₁-C₄-alkoxy, C₁-C₄-halogenalkoxy, C₂-C₄-alkenyl, C₂-C₃-halogenalkenyl, unsubstituted and substituted C₂-C₄-alkynyl, unsubstituted and substituted C₃-C₆-cycloalkyl, aryl and aryloxy, wherein the aliphatic moieties of R⁴, R^{4'}, and/or R^{4"} are unsubstituted or carry one, two, three or four R^{4a}, wherein the cycloalkyl moieties of R⁴, R^{4'}, and/or R^{4"} are unsubstituted or carry one, two, three or four R^{4b}, and wherein the aryloxy moieties of R⁴, R^{4'}, and/or R^{4"} are unsubstituted or carry one, two, three or four R^{4c}.

In still another embodiment, R⁴, R^{4'}, and/or R^{4"} are independently selected from hydrogen, F, Cl, C₁-C₄-alkyl, C₁-C₂-halogenalkyl, C₁-C₂-alkoxy, C₁-C₂-halogenalkoxy, cyclopropyl, phenyl and phenoxy, wherein the aliphatic moieties of R⁴, R^{4'}, and/or R^{4"} are unsubstituted or carry one, two, three or four R^{4a}, wherein the cycloalkyl moieties of R⁴, R^{4'}, and/or R^{4"} are unsubstituted or carry one, two, three or four R^{4b}, and wherein the aryloxy moieties of R⁴, R^{4'}, and/or R^{4"} are unsubstituted or carry one, two, three or four R^{4c}.

According to one specific embodiment, least one of R⁴, R^{4'}, and R^{4"} is not hydrogen.

According to another specific embodiment, all three R⁴, R^{4'}, and/or R^{4"} are halogens, preferably F.

According to another specific embodiment, two of R⁴, R^{4'}, and/or R^{4"} are halogens, preferably F and the third one is hydrogen.

According to another specific embodiment, two of R⁴, R^{4'}, and/or R^{4"} are hydrogens and the third one is halogen, preferably F.

R^{4a} are the possible substituents for the aliphatic moieties of R⁴, R^{4'}, and/or R^{4"}.

R^{4a} according to the invention is independently selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy.

According to one embodiment R^{4a} is independently selected from halogen, OH, CN, C₁-C₂-alkyl, C₁-C₂-alkoxy, C₃-C₆-cycloalkyl, C₃-C₆-halocycloalkyl and C₁-C₂-halogenalkoxy. Specifically, R^{4a} is independently selected from F, Cl, OH, CN, C₁-C₂-alkyl, C₁-C₂-alkoxy, cyclopropyl, 1-F-cyclopropyl, 1-Cl-cyclopropyl and C₁-C₂-halogenalkoxy.

R^{4b} are the possible substituents for the cycloalkyl moieties of R⁴, R^{4'}, and/or R^{4"}.

R^{4b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{4b} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{b} is independently selected from F, Cl, OH, CN, CH₃, OCH₃, cyclopropyl, 1-F-cyclopropyl, 1-Cl-cyclopropyl and halogenmethoxy.

R^{4c} are the possible substituents for the aryl, heteroaryl and aryloxy moieties of R⁴, R^{4'}, and/or R^{4"}.

R^{4c} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{4c} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{c} is independently selected from F, Cl, OH, CN, CH₃, OCH₃, cyclopropyl, 1-F-cyclopropyl, 1-Cl-cyclopropyl and halogenmethoxy.

In a preferred embodiment, R^{4a}, R^{4b}, and R^{4c} are independently selected from halogen, CN, and OH.

Particularly preferred embodiments of R⁴, R^{4'}, and/or R^{4"} according to the invention are in Table P4 below, wherein each line of lines P4-1 to P4-97 corresponds to one particular embodiment of the invention, wherein P4-1 to P4-97 are also in any combination a preferred embodiment of the present invention.

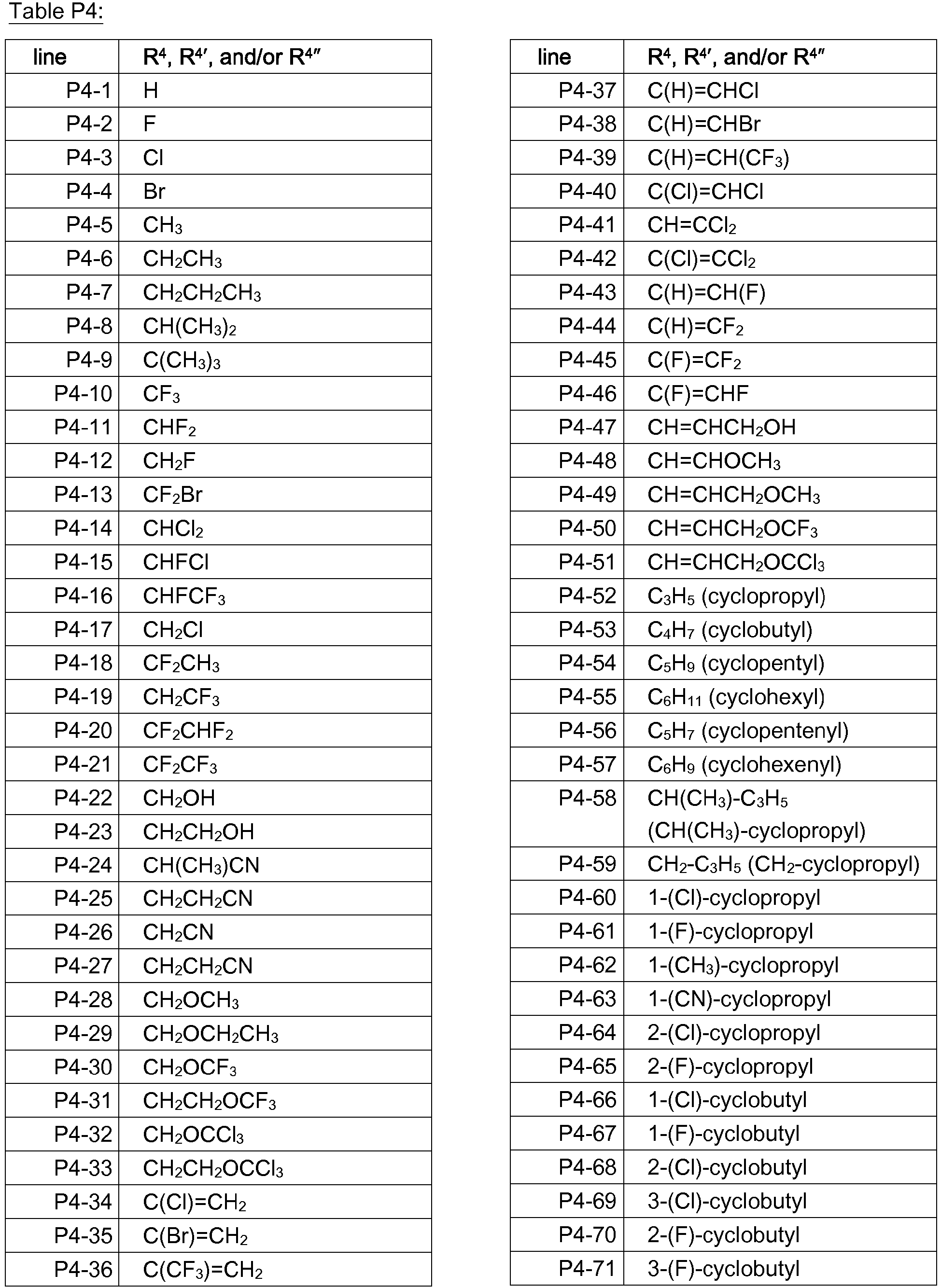

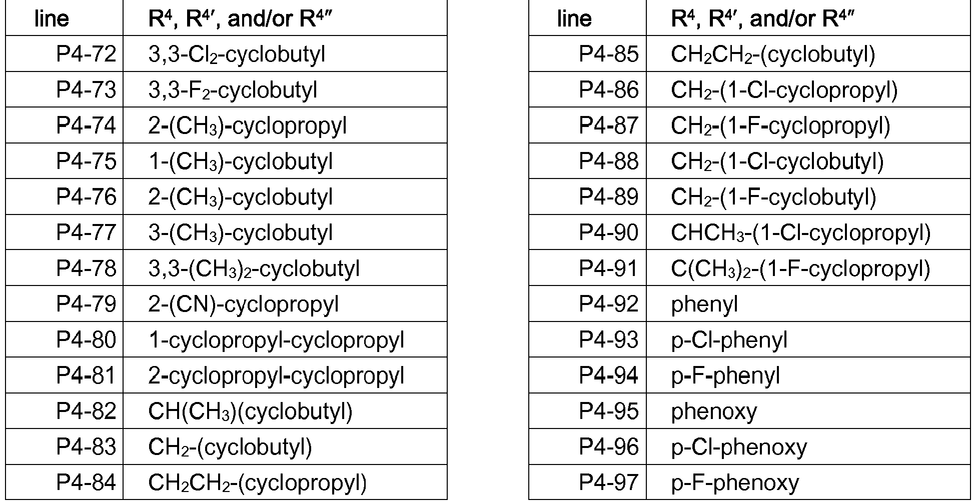

R⁵ and/or R^{5'} according to the present invention are independently selected from hydrogen or halogen, such as F, Cl or Br.

According to one embodiment, R⁵ and R^{5'} are hydrogens.

According to another embodiment, R⁵ is hydrogen and R^{5'} is halogen, preferably F or Cl. According to a specific embodiment thereof R^{5'} is Cl. According to a further specific embodiment thereof R^{5'} is F. This is the preferred embodiment.

According to another embodiment, R⁵ and R^{5'} are halogens, preferably F or Cl. According to a specific embodiment thereof R⁵ and R^{5'} are Cl. According to a further specific embodiment thereof R⁵ and R^{5'} are F. This is the preferred embodiment.

Specifically, CR⁵R^{5'} may be selected from CH₂, CF₂, CCl₂.

m according to the present invention is 1 or 2. According to one embodiment, m is 1. This is the preferred embodiment. According to another embodiment, m is 2.

X according to the present invention is O, S(O)ₙ, wherein n is 0,1 or 2, or NR^{N}; wherein R^{N} is selected from hydrogen, C₁-C₆-alkyl, C₂-C₆-alkenyl, C₂-C₆-alkynyl, C₁-C₆-alkoxy, -C(O)C₁-C₆-alkyl, C₃-C₆-cycloalkyl, C₃-C₆-cycloalkyl-C₁-C₄-alkyl, -S(O)₂- C₁-C₆-alkyl and -S(O)₂-aryl; wherein R^{N} is unsubstituted or further substituted by one, two, three or four of identical or different groups R^{Na}, which is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy.

According to one embodiment X is O.

According to another embodiment X is S(O)ₙ. According to one particular embodiment thereof X is S. According to another particular embodiment thereof X is S(O). According to another particular embodiment thereof X is S(O)₂.

According to one embodiment X is NR^{N}, for example NH, N-S(O)₂-CH₃ (N-(mesyl)) or N-S(O)₂-C₆H₄-CH₃ (N-(tosyl)).

In a preferred embodiment, X is O. Particularly preferred embodiments of the combination of R⁴, R^{4'}, and/or R^{4"} and X being O according to the invention are given in Table P5 below, wherein each line of lines P5-1 to P5-41 corresponds to one particular embodiment of the invention, wherein P5-1 to P5-41 are also in any combination a preferred embodiment of the present invention.

**Table P5:**

| **line** | **R^{4*}** | **R^{4'}** | **R^{4"}** |
|---|---|---|---|
| P5-1 | H | H | CH₃ |
| P5-2 | H | H | CH₂CH₃ |
| P5-3 | H | H | CH₂CH₂CH₃ |
| P5-4 | H | H | -CF₃ |
| P5-5 | H | H | -CH=CH₂ |
| P5-6 | H | H | -CH=CF₂ |
| P5-7 | H | H | -CF=CF₂ |
| P5-8 | H | H | -CF=CH₂ |
| P5-9 | H | H | -CCl=CH₂ |
| P5-10 | H | H | -CBr=CH₂ |
| P5-11 | H | H | -C(CF₃)=CH₂ |
| P5-12 | H | H | -CH=CClH |
| P5-13 | H | H | -CH=CBrH |
| P5-14 | H | H | -CH=C(CF₃)H |
| P5-15 | H | H | -C(CH₃)=CH₂ |
| P5-16 | H | H | -CH=C(CH₃)₂ |
| P5-17 | H | H | -C(CH₃)=C(CH₃)₂ |
| P5-18 | H | H | -C(CH₃)=C(CH₃)H |
| P5-19 | H | H | -C≡CH |
| P5-20 | H | H | -C≡CCH₃ |
| P5-21 | H | H | -C≡CCl |
| P5-22 | H | H | -C≡CBr |
| P5-23 | H | H | -C≡CSi(CH₃)₃ |
| P5-24 | H | H | -C≡C(C₃H₅) |
| P5-25 | H | H | C₃H₅(cyclopropyl) |
| P5-26 | H | H | C₄H₇(cyclobutyl) |
| P5-27 | H | H | C₅H₉(cyclopentyl) |
| P5-28 | H | H | C₅H₇ (cyclopentenyl) |
| P5-29 | H | H | C₆H₉ (cyclohexenyl) |
| P5-30 | H | H | Si(CH₃)₃ |
| P5-31 | H | H | CH₂-Si(CH₃)₃ |
| P5-32 | F | F | F |
| P5-33 | F | F | CHF₂ |
| P5-34 | F | F | CHFCl |
| P5-35 | F | F | CHFCF₃ |
| P5-36 | F | F | CF₂Br |
| P5-37 | F | F | CF=CF₂ |
| P5-38 | F | F | CH=CH₂ |
| P5-39 | F | F | H |
| P5-40 | CH₃ | CH₃ | H |
| P5-41 | CH₃ | CH₂CH₃ | H |
| P5-42 | Cl | CF₃ | H |
| P5-43 | Cl | F | CHF₂ |
| P5-44 | =CF₂ | | H |
| P5-45 | =CF₂ | | F |
| P5-46 | =CFCl | | F |
| P5-47 | =CHCl | | Cl |
| P5-48 | C₃H₅(cyclopropyl) | | H |
| P5-49 | C₄H₇(cyclobutyl) | | H |
| P5-50 | C₅H₉(cyclopentyl) | | H |

| | | | |
|---|---|---|---|
| *: If only one entry is given for both R⁴ and R^{4'}, the two substituents R⁴ and R^{4'} together form the given residue, together with the carbon atom to which R⁴ and R^{4'} are connected. | | | |

In another preferred embodiment, X is S. Particularly preferred embodiments of the combination of R⁴, R^{4'}, and/or R^{4"} and X being S according to the invention are if all three residues R⁴, R^{4'}, and/or R^{4"} are F, or if R⁴ and R^{4'} are both F and R^{4"} is H.

One embodiment relates to compounds of formula (I), wherein X is O, R⁵ and R^{5'} are H and m is 1 (compounds I.A). Another embodiment to compounds of formula (I), wherein X is O, R⁵ and R^{5'} are F and m is 1 (compounds I.B):

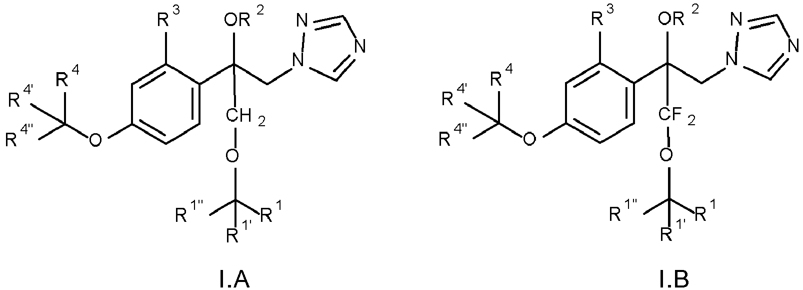

Another embodiment relates to compounds of formula (I), wherein X is S, R⁵ and R^{5'} are H and m is 1 (compounds I.C). Another embodiment to compounds of formula (I), wherein X is S and m is 1 or 2 (compounds I.D):

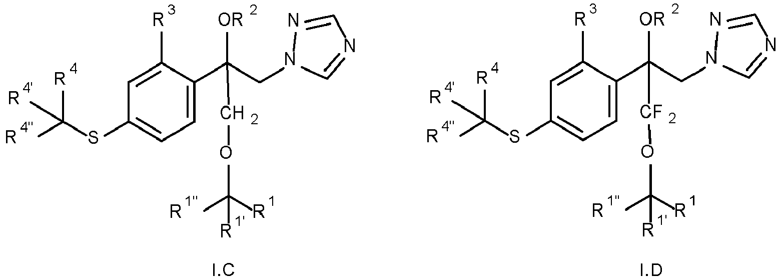

A further specific embodiment relates to compounds of formula (I), wherein X is S (compounds I.C and 1.D), wherein all three residues R⁴, R^{4'} and R^{4"} are F; or wherein R⁴ and R^{4'} are both F and R^{4"} is H.

Another embodiment relates to compounds of formula (I), wherein X is O, R⁴ and R^{4'} are both hydrogen, and R^{4'} is optionally substituted phenyl, wherein the phenyl may be unsubstituted (both R^{C1} and R^{C2} are hydrogen), or carry one (i.e., one of R^{C1} or R^{C2} is hydrogen) or two substituents R^{C1} and R^{C2}, m is 1 and R⁵ and R^{5'} are H (compounds I.E) or R⁵ and R^{5'} are F (compounds 1.F). It is understood that in compounds I.E and 1.F, if R^{C1/2} is selected as R^{C1}, then R^{C2/1} is selected as R^{C2}, and - *vice versa -* if R^{C2/1} is selected as R^{C2}, then R^{C2/1} is selected as R^{C1}:

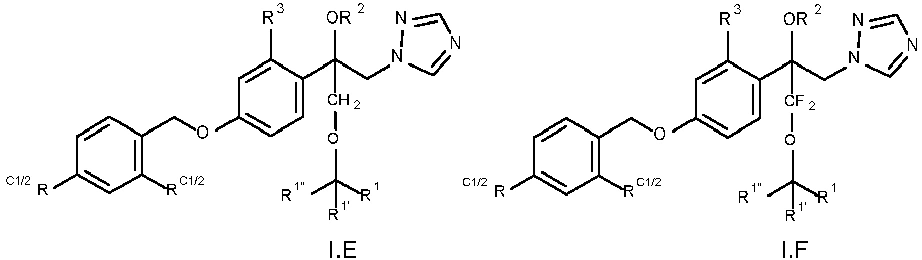

Particular preference is given to compounds of formula (I), in particular of formula (1.A), formula (I.B), formula (I.C) and formula (I.D), having R¹, R^{1'}, R^{1"}, R⁴, R^{4'} and R^{4"} as defined in the Table D1, wherein each line of lines D1-1 to D1-220 corresponds to one particular embodiment of the invention.

**Table D1:**

| **line** | R¹ | R^{1'} | R^{1"} | R⁴ | R^{4'} | R^{4"} |
|---|---|---|---|---|---|---|
| D1-1 | H | -CF₃ | H | H | H | -CF₃ |
| D1-2 | H | -CF₃ | H | H | H | -CH=CH₂ |
| D1-3 | H | -CF₃ | H | H | H | -CH=CF₂ |
| D1-4 | H | -CF₃ | H | H | H | -CF=CF₂ |
| D1-5 | H | -CF₃ | H | H | H | -CF=CH₂ |
| D1-6 | H | -CF₃ | H | H | H | -CCl=CH₂ |
| D1-7 | H | -CF₃ | H | H | H | -CBr=CH₂ |
| D1-8 | H | -CF₃ | H | H | H | -C(CF₃)=CH₂ |
| D1-9 | H | -CF₃ | H | H | H | -CH=CClH |
| D1-10 | H | -CF₃ | H | H | H | -CH=CBrH |
| D1-11 | H | -CF₃ | H | H | H | -CH=C(CF₃)H |
| D1-12 | H | -CF₃ | H | H | H | -C(CH₃)=CH₂ |
| D1-13 | H | -CF₃ | H | H | H | -CH=C(CH₃)₂ |
| D1-14 | H | -CF₃ | H | H | H | -C(CH₃)=C(CH₃)₂ |
| D1-15 | H | -CF₃ | H | H | H | -C(CH₃)=C(CH₃)H |
| D1-16 | H | -CF₃ | H | H | H | -C≡CH |
| D1-17 | H | -CF₃ | H | H | H | -C≡CCH₃ |
| D1-18 | H | -CF₃ | H | H | H | -C≡CCl |
| D1-19 | H | -CF₃ | H | H | H | -C≡CBr |
| D1-20 | H | -CF₃ | H | H | H | -C≡CSi(CH₃)₃ |
| D1-21 | H | -CF₃ | H | H | H | -C≡C(C₃H₅) |
| D1-22 | H | -CF₃ | H | H | H | C₃H₅(cyclopropyl) |
| D1-23 | H | -CF₃ | H | H | H | C₄H₇(cyclobutyl) |
| D1-24 | H | -CF₃ | H | H | H | C₅H₉(cyclopentyl) |
| D1-25 | H | -CF₃ | H | H | H | C₅H₇ (cyclopentenyl) |
| D1-26 | H | -CF₃ | H | H | H | C₆H₉ (cyclohexenyl) |
| D1-27 | H | -CF₃ | H | H | H | SCH₃ |
| D1-28 | H | -CF₃ | H | H | H | S(O)CH₃ |
| D1-29 | H | -CF₃ | H | H | H | Si(CH₃)₃ |
| D1-30 | H | -CF₃ | H | H | H | CH₂-Si(CH₃)₃ |
| D1-31 | H | -CF₃ | H | F | F | F |
| D1-32 | H | -CF₃ | H | F | F | CHF₂ |
| D1-33 | H | -CF₃ | H | F | F | CHFCl |
| D1-34 | H | -CF₃ | H | F | F | CHFCF₃ |
| D1-35 | H | -CF₃ | H | F | F | CF₂Br |
| D1-36 | H | -CF₃ | H | F | F | CF=CF₂ |
| D1-37 | H | -CF₃ | H | F | F | CH=CH₂ |
| D1-38 | H | -CF₃ | H | F | F | H |
| D1-39 | H | -CF₃ | H | Cl | CF₃ | H |
| D1-40 | H | -CF₃ | H | Cl | F | CHF₂ |
| D1-41 | H | -CF₃ | H | =CF₂ | | H |
| D1-42 | H | -CF₃ | H | =CF₂ | | F |
| D1-43 | H | -CF₃ | H | =CFCl | | F |
| D1-44 | H | -CF₃ | H | =CHCl | | Cl |
| D1-45 | H | F | F | H | H | -CF₃ |
| D1-46 | H | F | F | H | H | -CH=CH₂ |
| D1-47 | H | F | F | H | H | -CH=CF₂ |
| D1-48 | H | F | F | H | H | -CF=CF₂ |
| D1-49 | H | F | F | H | H | -CF=CH₂ |
| D1-50 | H | F | F | H | H | -CCl=CH₂ |
| D1-51 | H | F | F | H | H | -CBr=CH₂ |
| D1-52 | H | F | F | H | H | -C(CF₃)=CH₂ |
| D1-53 | H | F | F | H | H | -CH=CClH |
| D1-54 | H | F | F | H | H | -CH=CBrH |
| D1-55 | H | F | F | H | H | -CH=C(CF₃)H |
| D1-56 | H | F | F | H | H | -C(CH₃)=CH₂ |
| D1-57 | H | F | F | H | H | -CH=C(CH₃)₂ |
| D1-58 | H | F | F | H | H | -C(CH₃)=C(CH₃)₂ |
| D1-59 | H | F | F | H | H | -C(CH₃)=C(CH₃)H |
| D1-60 | H | F | F | H | H | -C≡C H |
| D1-61 | H | F | F | H | H | -C≡CCH₃ |
| D1-62 | H | F | F | H | H | -C≡CCl |
| D1-63 | H | F | F | H | H | -C≡CBr |
| D1-64 | H | F | F | H | H | -C≡CSi(CH₃)₃ |
| D1-65 | H | F | F | H | H | -C≡C(C₃H₅) |
| D1-66 | H | F | F | H | H | C₃H₅(cyclopropyl) |
| D1-67 | H | F | F | H | H | C₄H₇(cyclobutyl) |
| D1-68 | H | F | F | H | H | C₅H₉(cyclopentyl) |
| D1-69 | H | F | F | H | H | C₅H₇ (cyclopentenyl) |
| D1-70 | H | F | F | H | H | C₆H₉ (cyclohexenyl) |
| D1-71 | H | F | F | H | H | SCH₃ |
| D1-72 | H | F | F | H | H | S(O)CH₃ |
| D1-73 | H | F | F | H | H | Si(CH₃)₃ |
| D1-74 | H | F | F | H | H | CH₂-Si(CH₃)₃ |
| D1-75 | H | F | F | F | F | F |
| D1-76 | H | F | F | F | F | CHF₂ |
| D1-77 | H | F | F | F | F | CHFCl |
| D1-78 | H | F | F | F | F | CHFCF₃ |
| D1-79 | H | F | F | F | F | CF₂Br |
| D1-80 | H | F | F | F | F | CF=CF₂ |
| D1-81 | H | F | F | F | F | CH=CH₂ |
| D1-82 | H | F | F | F | F | H |
| D1-83 | H | F | F | Cl | CF₃ | H |
| D1-84 | H | F | F | Cl | F | CHF₂ |
| D1-85 | H | F | F | =CF₂ | | H |
| D1-86 | H | F | F | =CF₂ | | F |
| D1-87 | H | F | F | =CFCl | | F |
| D1-88 | H | F | F | =CHCl | | Cl |
| D1-89 | CHF₂ | H | H | H | H | -CF₃ |
| D1-90 | CHF₂ | H | H | H | H | -CH=CH₂ |
| D1-91 | CHF₂ | H | H | H | H | -CH=CF₂ |
| D1-92 | CHF₂ | H | H | H | H | -CF=CF₂ |
| D1-93 | CHF₂ | H | H | H | H | -CF=CH₂ |
| D1-94 | CHF₂ | H | H | H | H | -CCl=CH₂ |
| D1-95 | CHF₂ | H | H | H | H | -CBr=CH₂ |
| D1-96 | CHF₂ | H | H | H | H | -C(CF₃)=CH₂ |
| D1-97 | CHF₂ | H | H | H | H | -CH=CClH |
| D1-98 | CHF₂ | H | H | H | H | -CH=CBrH |
| D1-99 | CHF₂ | H | H | H | H | -CH=C(CF₃)H |
| D1-100 | CHF₂ | H | H | H | H | -C(CH₃)=CH₂ |
| D1-101 | CHF₂ | H | H | H | H | -CH=C(CH₃)₂ |
| D1-102 | CHF₂ | H | H | H | H | -C(CH₃)=C(CH₃)₂ |
| D1-103 | CHF₂ | H | H | H | H | -C(CH₃)=C(CH₃)H |
| D1-104 | CHF₂ | H | H | H | H | -C≡CH |
| D1-105 | CHF₂ | H | H | H | H | -C≡CCH₃ |
| D1-106 | CHF₂ | H | H | H | H | -C≡CCl |
| D1-107 | CHF₂ | H | H | H | H | -C≡CBr |
| D1-108 | CHF₂ | H | H | H | H | -C≡CSi(CH₃)₃ |
| D1-109 | CHF₂ | H | H | H | H | -C≡C(C₃H₅) |
| D1-110 | CHF₂ | H | H | H | H | C₃H₅(cyclopropyl) |
| D1-111 | CHF₂ | H | H | H | H | C₄H₇(cyclobutyl) |
| D1-112 | CHF₂ | H | H | H | H | C₅H₉(cyclopentyl) |
| D1-113 | CHF₂ | H | H | H | H | C₅H₇ (cyclopentenyl) |
| D1-114 | CHF₂ | H | H | H | H | C₆H₉ (cyclohexenyl) |
| D1-115 | CHF₂ | H | H | H | H | SCH₃ |
| D1-116 | CHF₂ | H | H | H | H | S(O)CH₃ |
| D1-117 | CHF₂ | H | H | H | H | Si(CH₃)₃ |
| D1-118 | CHF₂ | H | H | H | H | CH₂-Si(CH₃)₃ |
| D1-119 | CHF₂ | H | H | F | F | F |
| D1-120 | CHF₂ | H | H | F | F | CHF₂ |
| D1-121 | CHF₂ | H | H | F | F | CHFCl |
| D1-122 | CHF₂ | H | H | F | F | CHFCF₃ |
| D1-123 | CHF₂ | H | H | F | F | CF₂Br |
| D1-124 | CHF₂ | H | H | F | F | CF=CF₂ |
| D1-125 | CHF₂ | H | H | F | F | CH=CH₂ |
| D1-126 | CHF₂ | H | H | F | F | H |
| D1-127 | CHF₂ | H | H | Cl | CF₃ | H |
| D1-128 | CHF₂ | H | H | Cl | F | CHF₂ |
| D1-129 | CHF₂ | H | H | =CF₂ | | H |
| D1-130 | CHF₂ | H | H | =CF₂ | | F |
| D1-131 | CHF₂ | H | H | =CFCl | | F |
| D1-132 | CHF₂ | H | H | =CHCl | | Cl |
| D1-133 | CHF₂ | F | F | H | H | -CF₃ |
| D1-134 | CHF₂ | F | F | H | H | -CH=CH₂ |
| D1-135 | CHF₂ | F | F | H | H | -CH=CF₂ |
| D1-136 | CHF₂ | F | F | H | H | -CF=CF₂ |
| D1-137 | CHF₂ | F | F | H | H | -CF=CH₂ |
| D1-138 | CHF₂ | F | F | H | H | -CCl=CH₂ |
| D1-139 | CHF₂ | F | F | H | H | -CBr=CH₂ |
| D1-140 | CHF₂ | F | F | H | H | -C(CF₃)=CH₂ |
| D1-141 | CHF₂ | F | F | H | H | -CH=CClH |
| D1-142 | CHF₂ | F | F | H | H | -CH=CBrH |
| D1-143 | CHF₂ | F | F | H | H | -CH=C(CF₃)H |
| D1-144 | CHF₂ | F | F | H | H | -C(CH₃)=CH₂ |
| D1-145 | CHF₂ | F | F | H | H | -CH=C(CH₃)₂ |
| D1-146 | CHF₂ | F | F | H | H | -C(CH₃)=C(CH₃)₂ |
| D1-147 | CHF₂ | F | F | H | H | -C(CH₃)=C(CH₃)H |
| D1-148 | CHF₂ | F | F | H | H | -C≡CH |
| D1-149 | CHF₂ | F | F | H | H | -C≡CCH₃ |
| D1-150 | CHF₂ | F | F | H | H | -C≡CCl |
| D1-151 | CHF₂ | F | F | H | H | -C≡CBr |
| D1-152 | CHF₂ | F | F | H | H | -C≡CSi(CH₃)₃ |
| D1-153 | CHF₂ | F | F | H | H | -C≡C(C₃H₅) |
| D1-154 | CHF₂ | F | F | H | H | C₃H₅(cyclopropyl) |
| D1-155 | CHF₂ | F | F | H | H | C₄H₇(cyclobutyl) |
| D1-156 | CHF₂ | F | F | H | H | C₅H₉(cyclopentyl) |
| D1-157 | CHF₂ | F | F | H | H | C₅H₇ (cyclopentenyl) |
| D1-158 | CHF₂ | F | F | H | H | C₆H₉ (cyclohexenyl) |
| D1-159 | CHF₂ | F | F | H | H | SCH₃ |
| D1-160 | CHF₂ | F | F | H | H | S(O)CH₃ |
| D1-161 | CHF₂ | F | F | H | H | Si(CH₃)₃ |
| D1-162 | CHF₂ | F | F | H | H | CH₂-Si(CH₃)₃ |
| D1-163 | CHF₂ | F | F | F | F | F |
| D1-164 | CHF₂ | F | F | F | F | CHF₂ |
| D1-165 | CHF₂ | F | F | F | F | CHFCl |
| D1-166 | CHF₂ | F | F | F | F | CHFCF₃ |
| D1-167 | CHF₂ | F | F | F | F | CF₂Br |
| D1-168 | CHF₂ | F | F | F | F | CF=CF₂ |
| D1-169 | CHF₂ | F | F | F | F | CH=CH₂ |
| D1-170 | CHF₂ | F | F | F | F | H |
| D1-171 | CHF₂ | F | F | Cl | CF₃ | H |
| D1-172 | CHF₂ | F | F | Cl | F | CHF₂ |
| D1-173 | CHF₂ | F | F | =CF₂ | | H |
| D1-174 | CHF₂ | F | F | =CF₂ | | F |
| D1-175 | CHF₂ | F | F | =CFCl | | F |
| D1-176 | CHF₂ | F | F | =CHCl | | Cl |
| D1-177 | CH₂F | F | F | H | H | -CF₃ |
| D1-178 | CH₂F | F | F | H | H | -CH=CH₂ |
| D1-179 | CH₂F | F | F | H | H | -CH=CF₂ |
| D1-180 | CH₂F | F | F | H | H | -CF=CF₂ |
| D1-181 | CH₂F | F | F | H | H | -CF=CH₂ |
| D1-182 | CH₂F | F | F | H | H | -CCl=CH₂ |
| D1-183 | CH₂F | F | F | H | H | -CBr=CH₂ |
| D1-184 | CH₂F | F | F | H | H | -C(CF₃)=CH₂ |
| D1-185 | CH₂F | F | F | H | H | -CH=CClH |
| D1-186 | CH₂F | F | F | H | H | -CH=CBrH |
| D1-187 | CH₂F | F | F | H | H | -CH=C(CF₃)H |
| D1-188 | CH₂F | F | F | H | H | -C(CH₃)=CH₂ |
| D1-189 | CH₂F | F | F | H | H | -CH=C(CH₃)₂ |
| D1-190 | CH₂F | F | F | H | H | -C(CH₃)=C(CH₃)₂ |
| D1-191 | CH₂F | F | F | H | H | -C(CH₃)=C(CH₃)H |
| D1-192 | CH₂F | F | F | H | H | -C≡C H |
| D1-193 | CH₂F | F | F | H | H | -C≡CCH₃ |
| D1-194 | CH₂F | F | F | H | H | -C≡CCl |
| D1-195 | CH₂F | F | F | H | H | -C≡CBr |
| D1-196 | CH₂F | F | F | H | H | -C≡CSi(CH₃)₃ |
| D1-197 | CH₂F | F | F | H | H | -C≡C(C₃H₅) |
| D1-198 | CH₂F | F | F | H | H | C₃H₅(cyclopropyl) |
| D1-199 | CH₂F | F | F | H | H | C₄H₇(cyclobutyl) |
| D1-200 | CH₂F | F | F | H | H | C₅H₉(cyclopentyl) |
| D1-201 | CH₂F | F | F | H | H | C₅H₇ (cyclopentenyl) |
| D1-202 | CH₂F | F | F | H | H | C₆H₉ (cyclohexenyl) |
| D1-203 | CH₂F | F | F | H | H | SCH₃ |
| D1-204 | CH₂F | F | F | H | H | S(O)CH₃ |
| D1-205 | CH₂F | F | F | H | H | Si(CH₃)₃ |
| D1-206 | CH₂F | F | F | H | H | CH₂-Si(CH₃)₃ |
| D1-207 | CH₂F | F | F | F | F | F |
| D1-208 | CH₂F | F | F | F | F | CHF₂ |
| D1-209 | CH₂F | F | F | F | F | CHFCl |
| D1-210 | CH₂F | F | F | F | F | CHFCF₃ |
| D1-211 | CH₂F | F | F | F | F | CF₂Br |
| D1-212 | CH₂F | F | F | F | F | CF=CF₂ |
| D1-213 | CH₂F | F | F | F | F | CH=CH₂ |
| D1-214 | CH₂F | F | F | F | F | H |
| D1-215 | CH₂F | F | F | Cl | CF₃ | H |
| D1-216 | CH₂F | F | F | Cl | F | CHF₂ |
| D1-217 | CH₂F | F | F | =CF₂ | | H |
| D1-218 | CH₂F | F | F | =CF₂ | | F |
| D1-219 | CH₂F | F | F | =CFCl | | F |
| D1-220 | CH₂F | F | F | =CHCl | | Cl |

Particular preference is given to compounds of formula (I), in particular of formula (1.A), formula (I.B), formula (I.C) and formula (I.D), having R² and R³ as defined in the Table D2, wherein each line of lines D2-1 to D2-35 corresponds to one particular embodiment of the invention.

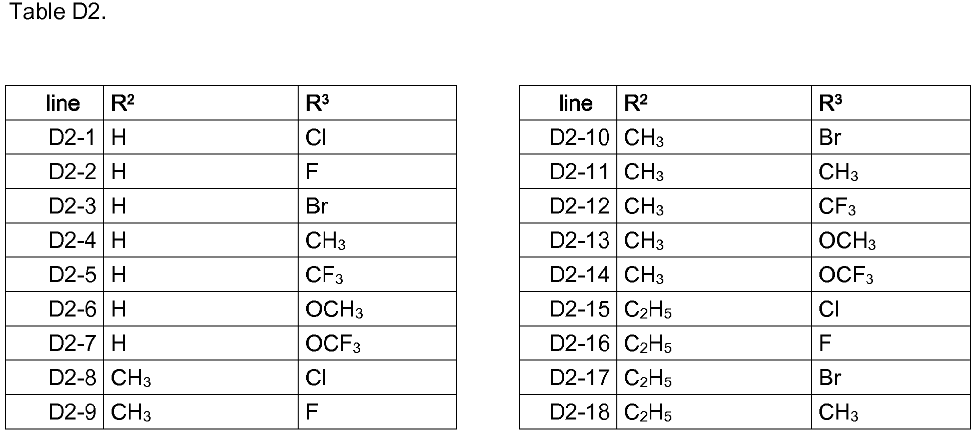

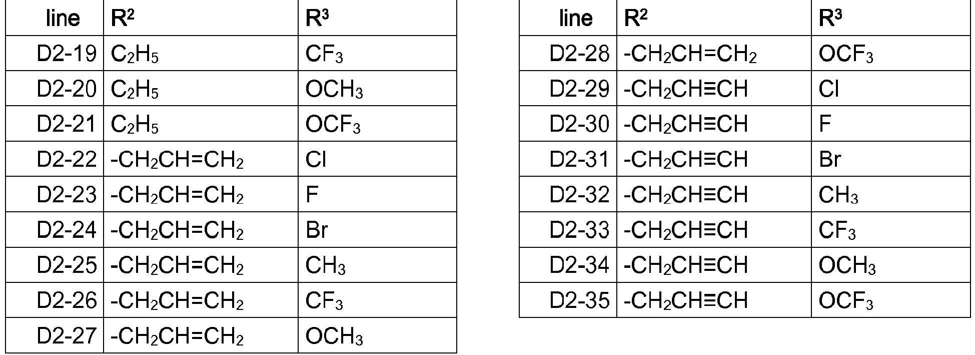

Particular preference is given to compounds of formula (I), in particular of formula (I.E) or of formula (I.F), having R³, R^{C1} and R^{C2} as defined in the Table D3, wherein each line of lines D3-1 to D3-70 corresponds to one particular embodiment of the invention.

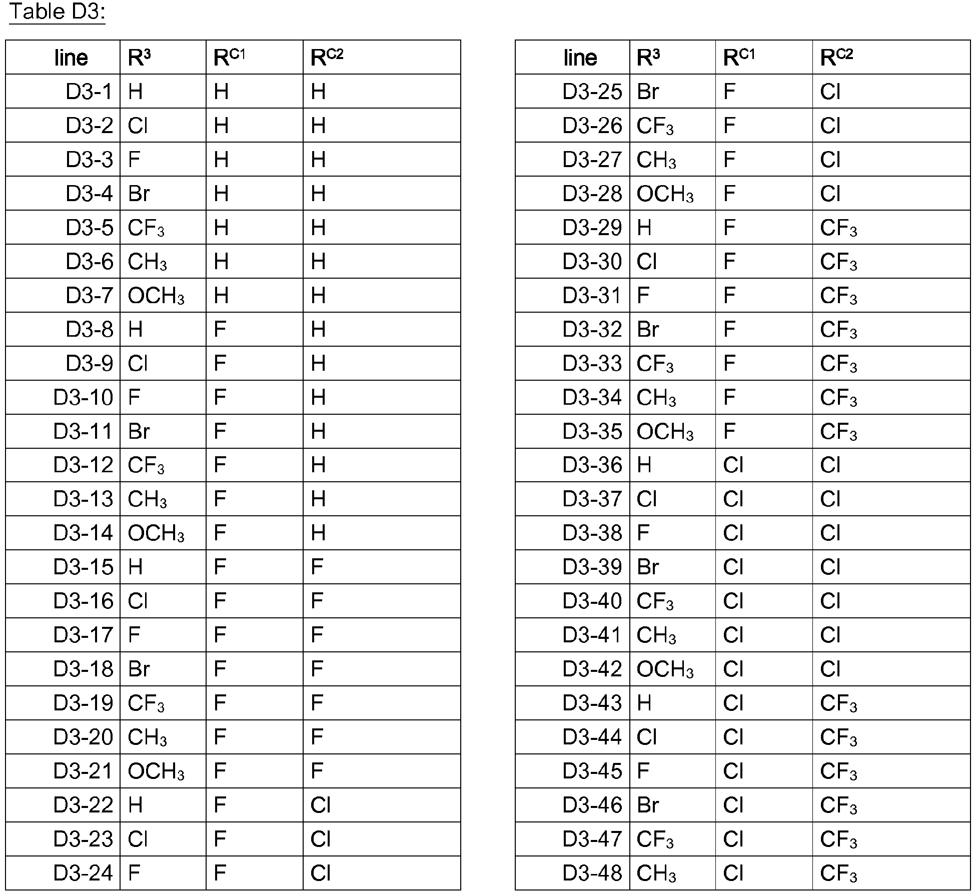

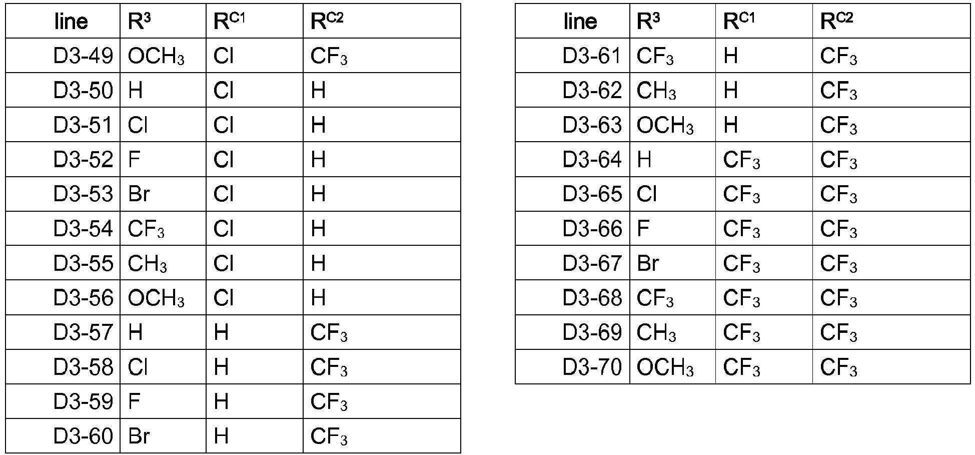

In particular, preference is given to the compounds of the formula Formula (I.A) that are compiled in the Tables 1.1.A -35.1.A. Each of the groups mentioned for a substituent in the tables is furthermore per se, independently of the combination in which it is mentioned, a particularly preferred aspect of the substituent in question.

### Table 1.1.A

Compounds of the formula (I.A) in which the combination of R² and R³ corresponds to line D2-1 of Table D2 and the combination of R¹, R^{1'}, R^{1"}, R⁴, R^{4'} and R^{4"} for each individual compound corresponds in each case to one line of Table D1 (compounds I.A.D2-1.D1-1 to I.A.D2-1.D1-220).

### Table 2.1.A

Compounds of the formula (I.A) in which the combination of R² and R³ corresponds to line D2-2 of Table D2 and the combination of R¹, R^{1'}, R^{1"}, R⁴, R^{4'} and R^{4"} for each individual compound corresponds in each case to one line of Table D1 (compounds I.A.D2-2.D1-1 to I.A.D2-2.D1-220).

### Table 3.1.A

Compounds of the formula (I.A) in which the combination of R² and R³ corresponds to line D2-3 of Table D2 and the combination of R¹, R^{1'}, R^{1"}, R⁴, R^{4'} and R^{4"} for each individual compound corresponds in each case to one line of Table D1 (compounds I.A.D2-3.D1-1 to I.A.D2-3.D1-220).

### Table 4.1.A

Compounds of the formula (I.A) in which the combination of R² and R³ corresponds to line D2-4 of Table D2 and the combination of R¹, R^{1'}, R^{1"}, R⁴, R^{4'} and R^{4"} for each individual compound corresponds in each case to one line of Table D1 (compounds I.A.D2-4.D1-1 to I.A.D2-4.D1-220).

### Table 5.1.A

Compounds of the formula (I.A) in which the combination of R² and R³ corresponds to line D2-5 of Table D2 and the combination of R¹, R^{1'}, R^{1"}, R⁴, R^{4'} and R^{4"} for each individual compound corresponds in each case to one line of Table D1 (compounds I.A.D2-5.D1-1 to I.A.D2-5.D1-220).

### Table 6.1.A

Compounds of the formula (I.A) in which the combination of R² and R³ corresponds to line D2-6 of Table D2 and the combination of R¹, R^{1'}, R^{1"}, R⁴, R^{4'} and R^{4"} for each individual compound corresponds in each case to one line of Table D1 (compounds I.A.D2-6.D1-1 to I.A.D2-6.D1-220).

### Table 7.1.A

Compounds of the formula (I.A) in which the combination of R² and R³ corresponds to line D2-7 of Table D2 and the combination of R¹, R^{1'}, R^{1"}, R⁴, R^{4'} and R^{4"} for each individual compound corresponds in each case to one line of Table D1 (compounds I.A.D2-7.D1-1 to I.A.D2-7.D1-220).

### Table 8.1.A

Compounds of the formula (I.A) in which the combination of R² and R³ corresponds to line D2-8 of Table D2 and the combination of R¹, R^{1'}, R^{1"}, R⁴, R^{4'} and R^{4"} for each individual compound corresponds in each case to one line of Table D1 (compounds I.A.D2-8.D1-1 to I.A.D2-8.D1-220).

### Table 9.1.A

Compounds of the formula (I.A) in which the combination of R² and R³ corresponds to line D2-9 of Table D2 and the combination of R¹, R^{1'}, R^{1"}, R⁴, R^{4'} and R^{4"} for each individual compound corresponds in each case to one line of Table D1 (compounds I.A.D2-9.D1-1 to I.A.D2-9.D1-220).

### Table 10.1.A

Compounds of the formula (I.A) in which the combination of R² and R³ corresponds to line D2-10 of Table D2 and the combination of R¹, R^{1'}, R^{1"}, R⁴, R^{4'} and R^{4"} for each individual compound corresponds in each case to one line of Table D1 (compounds I.A.D2-10.D1-1 to I.A.D2-10.D1-220).

### Table 11.1.A

Compounds of the formula (I.A) in which the combination of R² and R³ corresponds to line D2-11 of Table D2 and the combination of R¹, R^{1'}, R^{1"}, R⁴, R^{4'} and R^{4"} for each individual compound corresponds in each case to one line of Table D1 (compounds I.A.D2-11.D1-1 to I.A.D2-11.D1-220).

### Table 12.1.A

Compounds of the formula (I.A) in which the combination of R² and R³ corresponds to line D2-12 of Table D2 and the combination of R¹, R^{1'}, R^{1"}, R⁴, R^{4'} and R^{4"} for each individual compound corresponds in each case to one line of Table D1 (compounds I.A.D2-12.D1-1 to I.A.D2-12.D1-220).

### Table 13.1.A

Compounds of the formula (I.A) in which the combination of R² and R³ corresponds to line D2-13 of Table D2 and the combination of R¹, R^{1'}, R^{1"}, R⁴, R^{4'} and R^{4"} for each individual compound corresponds in each case to one line of Table D1 (compounds I.A.D2-13.D1-1 to I.A.D2-13.D1-220).

### Table 14.1.A

Compounds of the formula (I.A) in which the combination of R² and R³ corresponds to line D2-14 of Table D2 and the combination of R¹, R^{1'}, R^{1"}, R⁴, R^{4'} and R^{4"} for each individual compound corresponds in each case to one line of Table D1 (compounds I.A.D2-14.D1-1 to I.A.D2-14.D1-220).

### Table 15.1.A

Compounds of the formula (I.A) in which the combination of R² and R³ corresponds to line D2-15 of Table D2 and the combination of R¹, R^{1'}, R^{1"}, R⁴, R^{4'} and R^{4"} for each individual compound corresponds in each case to one line of Table D1 (compounds I.A.D2-15.D1-1 to I.A.D2-15.D1-220).

### Table 16.1.A

Compounds of the formula (I.A) in which the combination of R² and R³ corresponds to line D2-16 of Table D2 and the combination of R¹, R^{1'}, R^{1"}, R⁴, R^{4'} and R^{4"} for each individual compound corresponds in each case to one line of Table D1 (compounds I.A.D2-16.D1-1 to I.A.D2-16.D1-220).

### Table 17.1.A

Compounds of the formula (I.A) in which the combination of R² and R³ corresponds to line D2-17 of Table D2 and the combination of R¹, R^{1'}, R^{1"}, R⁴, R^{4'} and R^{4"} for each individual compound corresponds in each case to one line of Table D1 (compounds I.A.D2-17.D1-1 to I.A.D2-17.D1-220).

### Table 18.1.A

Compounds of the formula (I.A) in which the combination of R² and R³ corresponds to line D2-188 of Table D2 and the combination of R¹, R^{1'}, R^{1"}, R⁴, R^{4'} and R^{4"} for each individual compound corresponds in each case to one line of Table D1 (compounds I.A.D2-18.D1-1 to I.A.D2-18.D1-220).

### Table 19.1.A

Compounds of the formula (I.A) in which the combination of R² and R³ corresponds to line D2-19 of Table D2 and the combination of R¹, R^{1'}, R^{1"}, R⁴, R^{4'} and R^{4"} for each individual compound corresponds in each case to one line of Table D1 (compounds I.A.D2-19.D1-1 to I.A.D2-19.D1-220).

### Table 20.1.A

Compounds of the formula (I.A) in which the combination of R² and R³ corresponds to line D2-20 of Table D2 and the combination of R¹, R^{1'}, R^{1"}, R⁴, R^{4'} and R^{4"} for each individual compound corresponds in each case to one line of Table D1 (compounds I.A.D2-20.D1-1 to I.A.D2-20.D1-220).

### Table 21.1.A

Compounds of the formula (I.A) in which the combination of R² and R³ corresponds to line D2-21 of Table D2 and the combination of R¹, R^{1'}, R^{1"}, R⁴, R^{4'} and R^{4"} for each individual compound corresponds in each case to one line of Table D1 (compounds I.A.D2-21.D1-1 to I.A.D2-21.D1-220).

### Table 22.1.A

Compounds of the formula (I.A) in which the combination of R² and R³ corresponds to line D2-22 of Table D2 and the combination of R¹, R^{1'}, R^{1"}, R⁴, R^{4'} and R^{4"} for each individual compound corresponds in each case to one line of Table D1 (compounds I.A.D2-22.D1-1 to I.A.D2-22.D1-220).

### Table 23.1.A

Compounds of the formula (I.A) in which the combination of R² and R³ corresponds to line D2-23 of Table D2 and the combination of R¹, R^{1'}, R^{1"}, R⁴, R^{4'} and R^{4"} for each individual compound corresponds in each case to one line of Table D1 (compounds I.A.D2-23.D1-1 to I.A.D2-23.D1-220).

### Table 24.1.A

Compounds of the formula (I.A) in which the combination of R² and R³ corresponds to line D2-24 of Table D2 and the combination of R¹, R^{1'}, R^{1"}, R⁴, R^{4'} and R^{4"} for each individual compound corresponds in each case to one line of Table D1 (compounds I.A.D2-24.D1-1 to I.A.D2-24.D1-220).

### Table 25.1.A

Compounds of the formula (I.A) in which the combination of R² and R³ corresponds to line D2-25 of Table D2 and the combination of R¹, R^{1'}, R^{1"}, R⁴, R^{4'} and R^{4"} for each individual compound corresponds in each case to one line of Table D1 (compounds I.A.D2-25.D1-1 to I.A.D2-25.D1-220).

### Table 26.1.A

Compounds of the formula (I.A) in which the combination of R² and R³ corresponds to line D2-26 of Table D2 and the combination of R¹, R^{1'}, R^{1"}, R⁴, R^{4'} and R^{4"} for each individual compound corresponds in each case to one line of Table D1 (compounds I.A.D2-26.D1-1 to I.A.D2-26.D1-220).

### Table 27.1.A

Compounds of the formula (I.A) in which the combination of R² and R³ corresponds to line D2-27 of Table D2 and the combination of R¹, R^{1'}, R^{1"}, R⁴, R^{4'} and R^{4"} for each individual compound corresponds in each case to one line of Table D1 (compounds I.A.D2-27.D1-1 to I.A.D2-27.D1-220).

### Table 28.1.A

Compounds of the formula (I.A) in which the combination of R² and R³ corresponds to line D2-28 of Table D2 and the combination of R¹, R^{1'}, R^{1"}, R⁴, R^{4'} and R^{4"} for each individual compound corresponds in each case to one line of Table D1 (compounds I.A.D2-28.D1-1 to I.A.D2-28.D1-220).

### Table 29.1.A

Compounds of the formula (I.A) in which the combination of R² and R³ corresponds to line D2-29 of Table D2 and the combination of R¹, R^{1'}, R^{1"}, R⁴, R^{4'} and R^{4"} for each individual compound corresponds in each case to one line of Table D1 (compounds I.A.D2-29.D1-1 to I.A.D2-29.D1-220).

### Table 30.1.A

Compounds of the formula (I.A) in which the combination of R² and R³ corresponds to line D2-30 of Table D2 and the combination of R¹, R^{1'}, R^{1"}, R⁴, R^{4'} and R^{4"} for each individual compound corresponds in each case to one line of Table D1 (compounds I.A.D2-30.D1-1 to I.A.D2-30.D1-220).

### Table 31.1.A

Compounds of the formula (I.A) in which the combination of R² and R³ corresponds to line D2-31 of Table D2 and the combination of R¹, R^{1'}, R^{1"}, R⁴, R^{4'} and R^{4"} for each individual compound corresponds in each case to one line of Table D1 (compounds I.A.D2-31.D1-1 to I.A.D2-31.D1-220).

### Table 32.1.A

Compounds of the formula (I.A) in which the combination of R² and R³ corresponds to line D2-32 of Table D2 and the combination of R¹, R^{1'}, R^{1"}, R⁴, R^{4'} and R^{4"} for each individual compound corresponds in each case to one line of Table D1 (compounds I.A.D2-32.D1-1 to I.A.D2-32.D1-220).

### Table 33.1.A

Compounds of the formula (I.A) in which the combination of R² and R³ corresponds to line D2-33 of Table D2 and the combination of R¹, R^{1'}, R^{1"}, R⁴, R^{4'} and R^{4"} for each individual compound corresponds in each case to one line of Table D1 (compounds I.A.D2-33.D1-1 to I.A.D2-33.D1-220).

### Table 34.1.A

Compounds of the formula (I.A) in which the combination of R² and R³ corresponds to line D2-34 of Table D2 and the combination of R¹, R^{1'}, R^{1"}, R⁴, R^{4'} and R^{4"} for each individual compound corresponds in each case to one line of Table D1 (compounds I.A.D2-34.D1-1 to I.A.D2-34.D1-220).

### Table 35.1.A

Compounds of the formula (I.A) in which the combination of R² and R³ corresponds to line D2-35 of Table D2 and the combination of R¹, R^{1'}, R^{1"}, R⁴, R^{4'} and R^{4"} for each individual compound corresponds in each case to one line of Table D1 (compounds I.A.D2-35.D1-1 to I.A.D2-35.D1-220).

Further, preference is given to the compounds of the formula Formula (I.B) that are compiled in the Tables 1.1.B -35.1.B. Each of the groups mentioned for a substituent in the tables is furthermore per se, independently of the combination in which it is mentioned, a particularly preferred aspect of the substituent in question.

### Table 1.1.B

Compounds of the formula (I.B) in which the combination of R² and R³ corresponds to line D2-1 of Table D2 and the combination of R¹, R^{1'}, R^{1"}, R⁴, R^{4'} and R^{4"} for each individual compound corresponds in each case to one line of Table D1 (compounds I.B.D2-1.D1-1 to I.B.D2-1.D1-220).

### Table 2.1.B

Compounds of the formula (I.B) in which the combination of R² and R³ corresponds to line D2-2 of Table D2 and the combination of R¹, R^{1'}, R^{1"}, R⁴, R^{4'} and R^{4"} for each individual compound corresponds in each case to one line of Table D1 (compounds I.B.D2-2.D1-1 to I.B.D2-2.D1-220).

### Table 3.1.B

Compounds of the formula (I.B) in which the combination of R² and R³ corresponds to line D2-3 of Table D2 and the combination of R¹, R^{1'}, R^{1"}, R⁴, R^{4'} and R^{4"} for each individual compound corresponds in each case to one line of Table D1 (compounds I.B.D2-3.D1-1 to I.B.D2-3.D1-220).

### Table 4.1.B

Compounds of the formula (I.B) in which the combination of R² and R³ corresponds to line D2-4 of Table D2 and the combination of R¹, R^{1'}, R^{1"}, R⁴, R^{4'} and R^{4"} for each individual compound corresponds in each case to one line of Table D1 (compounds I.B.D2-4.D1-1 to I.B.D2-4.D1-220).

### Table 5.1.B

Compounds of the formula (I.B) in which the combination of R² and R³ corresponds to line D2-5 of Table D2 and the combination of R¹, R^{1'}, R^{1"}, R⁴, R^{4'} and R^{4"} for each individual compound corresponds in each case to one line of Table D1 (compounds I.B.D2-5.D1-1 to I.B.D2-5.D1-220).

### Table 6.1.B

Compounds of the formula (I.B) in which the combination of R² and R³ corresponds to line D2-6 of Table D2 and the combination of R¹, R^{1'}, R^{1"}, R⁴, R^{4'} and R^{4"} for each individual compound corresponds in each case to one line of Table D1 (compounds I.B.D2-6.D1-1 to I.B.D2-6.D1-220).

### Table 7.1.B

Compounds of the formula (I.B) in which the combination of R² and R³ corresponds to line D2-7 of Table D2 and the combination of R¹, R^{1'}, R^{1"}, R⁴, R^{4'} and R^{4"} for each individual compound corresponds in each case to one line of Table D1 (compounds I.B.D2-7.D1-1 to I.B.D2-7.D1-220).

### Table 8.1.B

Compounds of the formula (I.B) in which the combination of R² and R³ corresponds to line D2-8 of Table D2 and the combination of R¹, R^{1'}, R^{1"}, R⁴, R^{4'} and R^{4"} for each individual compound corresponds in each case to one line of Table D1 (compounds I.B.D2-8.D1-1 to I.B.D2-8.D1-220).

### Table 9.1.B

Compounds of the formula (I.B) in which the combination of R² and R³ corresponds to line D2-9 of Table D2 and the combination of R¹, R^{1'}, R^{1"}, R⁴, R^{4'} and R^{4"} for each individual compound corresponds in each case to one line of Table D1 (compounds I.B.D2-9.D1-1 to I.B.D2-9.D1-220).

### Table 10.1.B

Compounds of the formula (I.B) in which the combination of R² and R³ corresponds to line D2-10 of Table D2 and the combination of R¹, R^{1'}, R^{1"}, R⁴, R^{4'} and R^{4"} for each individual compound corresponds in each case to one line of Table D1 (compounds I.B.D2-10.D1-1 to I.B.D2-10.D1-220).

### Table 11.1.B

Compounds of the formula (I.B) in which the combination of R² and R³ corresponds to line D2-11 of Table D2 and the combination of R¹, R^{1'}, R^{1"}, R⁴, R^{4'} and R^{4"} for each individual compound corresponds in each case to one line of Table D1 (compounds I.B.D2-11.D1-1 to I.B.D2-11.D1-220).

### Table 12.1.B

Compounds of the formula (I.B) in which the combination of R² and R³ corresponds to line D2-12 of Table D2 and the combination of R¹, R^{1'}, R^{1"}, R⁴, R^{4'} and R^{4"} for each individual compound corresponds in each case to one line of Table D1 (compounds I.B.D2-12.D1-1 to I.B.D2-12.D1-220).

### Table 13.1.B

Compounds of the formula (I.B) in which the combination of R² and R³ corresponds to line D2-13 of Table D2 and the combination of R¹, R^{1'}, R^{1"}, R⁴, R^{4'} and R^{4"} for each individual compound corresponds in each case to one line of Table D1 (compounds I.B.D2-13.D1-1 to I.B.D2-13.D1-220).

### Table 14.1.B

Compounds of the formula (I.B) in which the combination of R² and R³ corresponds to line D2-14 of Table D2 and the combination of R¹, R^{1'}, R^{1"}, R⁴, R^{4'} and R^{4"} for each individual compound corresponds in each case to one line of Table D1 (compounds I.B.D2-14.D1-1 to I.B.D2-14.D1-220).

### Table 15.1.B

Compounds of the formula (I.B) in which the combination of R² and R³ corresponds to line D2-15 of Table D2 and the combination of R¹, R^{1'}, R^{1"}, R⁴, R^{4'} and R^{4"} for each individual compound corresponds in each case to one line of Table D1 (compounds I.B.D2-15.D1-1 to I.B.D2-15.D1-220).

### Table 16.1.B

Compounds of the formula (I.B) in which the combination of R² and R³ corresponds to line D2-16 of Table D2 and the combination of R¹, R^{1'}, R^{1"}, R⁴, R^{4'} and R^{4"} for each individual compound corresponds in each case to one line of Table D1 (compounds I.B.D2-16.D1-1 to I.B.D2-16.D1-220).

### Table 17.1.B

Compounds of the formula (I.B) in which the combination of R² and R³ corresponds to line D2-17 of Table D2 and the combination of R¹, R^{1'}, R^{1"}, R⁴, R^{4'} and R^{4"} for each individual compound corresponds in each case to one line of Table D1 (compounds I.B.D2-17.D1-1 to I.B.D2-17.D1-220).

### Table 18.1.B

Compounds of the formula (I.B) in which the combination of R² and R³ corresponds to line D2-188 of Table D2 and the combination of R¹, R^{1'}, R^{1"}, R⁴, R^{4'} and R^{4"} for each individual compound corresponds in each case to one line of Table D1 (compounds I.B.D2-18.D1-1 to I.B.D2-18.D1-220).

### Table 19.1.B

Compounds of the formula (I.B) in which the combination of R² and R³ corresponds to line D2-19 of Table D2 and the combination of R¹, R^{1'}, R^{1"}, R⁴, R^{4'} and R^{4"} for each individual compound corresponds in each case to one line of Table D1 (compounds I.B.D2-19.D1-1 to I.B.D2-19.D1-220).

### Table 20.1.B

Compounds of the formula (I.B) in which the combination of R² and R³ corresponds to line D2-20 of Table D2 and the combination of R¹, R^{1'}, R^{1"}, R⁴, R^{4'} and R^{4"} for each individual compound corresponds in each case to one line of Table D1 (compounds I.B.D2-20.D1-1 to I.B.D2-20.D1-220).

### Table 21.1.B

Compounds of the formula (I.B) in which the combination of R² and R³ corresponds to line D2-21 of Table D2 and the combination of R¹, R^{1'}, R^{1"}, R⁴, R^{4'} and R^{4"} for each individual compound corresponds in each case to one line of Table D1 (compounds I.B.D2-21.D1-1 to I.B.D2-21.D1-220).

### Table 22.1.B

Compounds of the formula (I.B) in which the combination of R² and R³ corresponds to line D2-22 of Table D2 and the combination of R¹, R^{1'}, R^{1"}, R⁴, R^{4'} and R^{4"} for each individual compound corresponds in each case to one line of Table D1 (compounds I.B.D2-22.D1-1 to I.B.D2-22.D1-220).

### Table 23.1.B

Compounds of the formula (I.B) in which the combination of R² and R³ corresponds to line D2-23 of Table D2 and the combination of R¹, R^{1'}, R^{1"}, R⁴, R^{4'} and R^{4"} for each individual compound corresponds in each case to one line of Table D1 (compounds I.B.D2-23.D1-1 to I.B.D2-23.D1-220).

### Table 24.1.B

Compounds of the formula (I.B) in which the combination of R² and R³ corresponds to line D2-24 of Table D2 and the combination of R¹, R^{1'}, R^{1"}, R⁴, R^{4'} and R^{4"} for each individual compound corresponds in each case to one line of Table D1 (compounds I.B.D2-24.D1-1 to I.B.D2-24.D1-220).

### Table 25.1.B

Compounds of the formula (I.B) in which the combination of R² and R³ corresponds to line D2-25 of Table D2 and the combination of R¹, R^{1'}, R^{1"}, R⁴, R^{4'} and R^{4"} for each individual compound corresponds in each case to one line of Table D1 (compounds I.B.D2-25.D1-1 to I.B.D2-25.D1-220).

### Table 26.1.B

Compounds of the formula (I.B) in which the combination of R² and R³ corresponds to line D2-26 of Table D2 and the combination of R¹, R^{1'}, R^{1"}, R⁴, R^{4'} and R^{4"} for each individual compound corresponds in each case to one line of Table D1 (compounds I.B.D2-26.D1-1 to I.B.D2-26.D1-220).

### Table 27.1.B

Compounds of the formula (I.B) in which the combination of R² and R³ corresponds to line D2-27 of Table D2 and the combination of R¹, R^{1'}, R^{1"}, R⁴, R^{4'} and R^{4"} for each individual compound corresponds in each case to one line of Table D1 (compounds I.B.D2-27.D1-1 to I.B.D2-27.D1-220).

### Table 28.1.B

Compounds of the formula (I.B) in which the combination of R² and R³ corresponds to line D2-28 of Table D2 and the combination of R¹, R^{1'}, R^{1"}, R⁴, R^{4'} and R^{4"} for each individual compound corresponds in each case to one line of Table D1 (compounds I.B.D2-28.D1-1 to I.B.D2-28.D1-220).

### Table 29.1.B

Compounds of the formula (I.B) in which the combination of R² and R³ corresponds to line D2-29 of Table D2 and the combination of R¹, R^{1'}, R^{1"}, R⁴, R^{4'} and R^{4"} for each individual compound corresponds in each case to one line of Table D1 (compounds I.B.D2-29.D1-1 to I.B.D2-29.D1-220).

### Table 30.1.B

Compounds of the formula (I.B) in which the combination of R² and R³ corresponds to line D2-30 of Table D2 and the combination of R¹, R^{1'}, R^{1"}, R⁴, R^{4'} and R^{4"} for each individual compound corresponds in each case to one line of Table D1 (compounds I.B.D2-30.D1-1 to I.B.D2-30.D1-220).

### Table 31.1.B

Compounds of the formula (I.B) in which the combination of R² and R³ corresponds to line D2-31 of Table D2 and the combination of R¹, R^{1'}, R^{1"}, R⁴, R^{4'} and R^{4"} for each individual compound corresponds in each case to one line of Table D1 (compounds I.B.D2-31.D1-1 to I.B.D2-31.D1-220).

### Table 32.1.B

Compounds of the formula (I.B) in which the combination of R² and R³ corresponds to line D2-32 of Table D2 and the combination of R¹, R^{1'}, R^{1"}, R⁴, R^{4'} and R^{4"} for each individual compound corresponds in each case to one line of Table D1 (compounds I.B.D2-32.D1-1 to I.B.D2-32.D1-220).

### Table 33.1.B

Compounds of the formula (I.B) in which the combination of R² and R³ corresponds to line D2-33 of Table D2 and the combination of R¹, R^{1'}, R^{1"}, R⁴, R^{4'} and R^{4"} for each individual compound corresponds in each case to one line of Table D1 (compounds I.B.D2-33.D1-1 to I.B.D2-33.D1-220).

### Table 34.1.B

Compounds of the formula (I.B) in which the combination of R² and R³ corresponds to line D2-34 of Table D2 and the combination of R¹, R^{1'}, R^{1"}, R⁴, R^{4'} and R^{4"} for each individual compound corresponds in each case to one line of Table D1 (compounds I.B.D2-34.D1-1 to I.B.D2-34.D1-220).

### Table 35.1.B

Compounds of the formula (I.B) in which the combination of R² and R³ corresponds to line D2-35 of Table D2 and the combination of R¹, R^{1'}, R^{1"}, R⁴, R^{4'} and R^{4"} for each individual compound corresponds in each case to one line of Table D1 (compounds I.B.D2-35.D1-1 to I.B.D2-35.D1-220).

The compounds (I) and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). Some are systemically effective and they can be used in crop protection as foliar fungicides, fungicides for seed dressing, and soil fungicides. Moreover, they are suitable for controlling harmful fungi, which inter alia occur in wood or roots of plants.

The compounds (I) and the compositions according to the invention are particularly important in the control of a multitude of phytopathogenic fungi on various cultivated plants, such as cereals, e. g. wheat, rye, barley, triticale, oats, or rice; beet, e. g. sugar beet or fodder beet; fruits, e. g. pomes (apples, pears, etc.), stone fruits (plums, peaches, almonds, cherries, etc.), or soft fruits, which are also called berries (strawberries, raspberries, blackberries, gooseberries, etc.); leguminous plants, e. g. lentils, peas, alfalfa, or soybeans; oil plants, e. g. oilseed rape, mustard, olives, sunflowers, coconut, cocoa beans, castor oil plants, oil palms, ground nuts, or soybeans; cucurbits, e. g. squashes, cucumber, or melons; fiber plants, e. g. cotton, flax, hemp, or jute; citrus fruits, e. g. oranges, lemons, grapefruits, or mandarins; vegetables, e. g. spinach, lettuce, asparagus, cabbages, carrots, onions, tomatoes, potatoes, cucurbits, or paprika; lauraceous plants, e. g. avocados, cinnamon, or camphor; energy and raw material plants, e. g. corn, soybean, oilseed rape, sugar cane, or oil palm; corn; tobacco; nuts; coffee; tea; bananas; vines (table grapes and grape juice grape vines); hop; turf; sweet leaf (also called Stevia); natural rubber plants; or ornamental and forestry plants, e. g. flowers, shrubs, broad-leaved trees, or evergreens (conifers, eucalypts, etc.); and on the plant propagation material, such as seeds; and on the crop material of these plants.

Preferably, compounds I and compositions thereof, respectively are used for controlling a multitude of fungi on field crops, such as potatoes, sugar beets, tobacco, wheat, rye, barley, oats, rice, corn, cotton, soybeans, oilseed rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant, such as seeds; and vegetative plant materials, such as cuttings and tubers (e. g. potatoes), which can be used for the multiplication of the plant. This includes seeds, roots, fruits, tubers, bulbs, rhizomes, shoots, sprouts and other parts of plants; including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by mutagenesis or genetic engineering to provide a new trait to a plant or to modify an already present trait. Mutagenesis includes techniques of random mutagenesis using X-rays or mutagenic chemicals, but also techniques of targeted mutagenesis, to create mutations at a specific locus of a plant genome. Targeted mutagenesis techniques frequently use oligonucleotides or proteins like CRISPR/Cas, zinc-finger nucleases, TALENs or meganucleases to achieve the targeting effect. Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve a trait. These integrated genes are also referred to as transgenes in the art, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by mutagenesis and breeding comprise plant varieties commercially available under the name Clearfield®.

Herbicide tolerance has been created via the use of transgenes to glyphosate, glufosinate, 2,4-D, dicamba, oxynil herbicides, like bromoxynil and ioxynil, sulfonylurea herbicides, ALS inhibitors and 4-hydroxyphenylpyruvate dioxygenase (HPPD) inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621, goxv247; for tolerance to glufosinate: pat and bar, for tolerance to 2,4-D: aad-1, aad-12; for tolerance to dicamba: dmo; for tolerance to oxynil herbicies: bxn; for tolerance to sulfonylurea herbicides: zm-hra, csr1-2, gm-hra, S4-HrA; for tolerance to ALS inhibitors: csr1-2; and for tolerance to HPPD inhibitors: hppdPF, W336, avhppd-03.

Transgenic corn events comprising herbicide tolerance genes include, but are not limited to, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275. Transgenic soybean events comprising herbicide tolerance genes include, but are not limited to, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127. Transgenic cotton events comprising herbicide tolerance genes include, but are not limited to, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40. Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants: Transgenes which have most frequently been used are toxin genes of *Bacillus* spp. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin, such as genes coding for protease inhibitors, like CpTI and pinII, have been transferred to other plants. A further approach uses transgenes such as dvsnf7 to produce double-stranded RNA in plants.

Transgenic corn events comprising genes for insecticidal proteins or double stranded RNA include, but are not limited to, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098. Transgenic soybean events comprising genes for insecticidal proteins include, but are not limited to, MON87701, MON87751 and DAS-81419. Transgenic cotton events comprising genes for insecticidal proteins include, but are not limited to, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by using the transgene athb17, being present for example in corn event MON87403, or by using the transgene bbx32, being present for example in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, such as drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process resulting in a cultivated plant with stacked traits. Preferred combinations of traits are combinations of herbicide tolerance traits to different groups of herbicides, combinations of insect tolerance to different kind of insects, in particular tolerance to lepidopteran and coleopteran insects, combinations of herbicide tolerance with one or several types of insect resistance, combinations of herbicide tolerance with increased yield as well as combinations of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase). Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527; for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571; for potato events E12, F10, J3, J55, V11, X17, Y9 in WO14/178910, WO14/178913, WO14/178941, WO14/179276, WO16/183445, WO17/062831, WO17/062825; for rice events LLRICE06, LLRICE601, LLRICE62 in WO00/026345, WO00/026356, WO00/026345; and for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compounds I and compositions according to the invention, respectively, on cultivated plants may result in effects which are specific to a cultivated plant comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

The compounds I and compositions thereof, respectively, are particularly suitable for controlling the following plant diseases:
*Albugo* spp. (white rust) on ornamentals, vegetables (e. g. *A. candida*) and sunflowers (e.g. *A. tragopogonis*); *Alternaria* spp. (Alternaria leaf spot) on vegetables (e.g. *A. daucior A. porri*), oilseed rape (*A. brassicicola* or *brassicae*), sugar beets (*A. tenuis*), fruits (e.g. *A. grandis*), rice, soybeans, potatoes and tomatoes (e.g. *A. solani, A. grandis* or *A. alternata*), tomatoes (e.g. *A. solanior A. alternata*) and wheat (e.g. A. *triticina*); *Aphanomyces* spp. on sugar beets and vegetables; *Ascochyta* spp. on cereals and vegetables, e. g. *A. tritici*(*anthracnose*) on wheat and *A. hordei* on barley; *Aureobasidium zeae* (syn. *Kapatiella* zeae) on corn; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages); *B. squamosa* or *B. alliion* onion family), oilseed rape, ornamentals (e.g. *B eliptica*), vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi*(*Dutch* elm disease) on elms; *Cercospora* spp. (Cercospora leaf spots) on corn (e. g. Gray leaf spot: *C. zeae-maydis*), rice, sugar beets (e. g. *C. beticola*), sugar cane, vegetables, coffee, soybeans (e. g. *C. sojina* or *C. kikuchit*) and rice; *Cladobotryum* (*syn. Dactylium*) spp. (e.g. *C. mycophilum*
(formerly *Dactylium dendroides,* teleomorph: *Nectria albertinii, Nectria rosella* syn. *Hypomyces rosellus)* on mushrooms; *Cladosporium* spp. on tomatoes (e. g. *C. fulvum* leaf mold) and cereals, e. g. *C. herbarum* (black ear) on wheat; *Clavicepspurpurea* (ergot) on cereals; *Cochliobolus* (anamorph: *Helminthosporium* of *Bipolaris*) spp. (leaf spots) on corn (C. *carbonum*), cereals (e. g. *C. sativus,* anamorph: *B. sorokiniana*) and rice (e. g. *C. miyabeanus,* anamorph: *H. oryzae*); *Colletotrichum* (teleomorph: *Glomerella*) spp. (anthracnose) on cotton (e. g. *C. gossypii*), corn (e. g. *C. graminicola:* Anthracnose stalk rot), soft fruits, potatoes (e. g. *C. coccodes*: black dot), beans (e. g. *C. lindemuthianum*), soybeans (e. g. *C. truncatumor C. gloeosporioides*), vegetables (e.g. *C. lagenariumor C*. *capsici*), fruits (e.g. *C. acutatum*), coffee (e.g. *C. coffeanumor C. kahawae*) and *C. gloeosporioides* on various crops; *Corticium* spp., e. g. *C. sasakii*(sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia*) *necatrix*(*root* and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D. tritici-repentis*: tan spot), rice and turf; Esca (dieback, apoplexy) on vines, caused by *Formitiporia* (syn. *Phellinus*) *punctata, F. mediterranea, Phaeomoniella chlamydospora* (formerly *Phaeoacremonium chlamydosporum*), *Phaeoacremonium aleophilum* and/or *Botryosphaeria obtusa*; *Elsinoe* spp. on pome fruits (*E. pyri*), soft fruits (*E. veneta:* anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, oilseed rape (e. g. *E. cruciferarum*)*; Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f*.* sp. *glycines* now syn. *F. virguliforme*) and *F. tucumaniae* and *F. brasiliense* each causing sudden death syndrome on soybeans, and *F. verticil-lioides* on corn; *Gaeumannomyces graminis* (take-all) on cereals (e. g. wheat or barley) and corn; *Gibberella* spp. on cereals (e. g. *G. zeae)* and rice (e. g. *G. fujikuroi*: Bakanae disease); *Glomerella cingulata* on vines, pome fruits and other plants and *G. gossypii* on cotton; Grain-staining complex on rice; *Guignardia bidwellii*(black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals, potatoes and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (syn. *Monilia* spp.: bloom and twig blight, brown rot) on stone fruits and other rosaceous plants; *Mycosphaerella* spp. on cereals, bananas, soft fruits and ground nuts, such as e. g. *M. graminicola* (anamorph: *Zymoseptoria tritici* formerly *Septoria tritici:* Septoria blotch) on wheat or *M. fijiensis* (syn. *Pseudocercospora fijiensis.* black Sigatoka disease) and *M. musicola* on bananas, *M. arachidicola* (syn. *M. arachidis* or *Cercospora arachidis*), *M. berkeleyi* on peanuts, *M. pisi* on peas and *M. brassiciola* on brassicas; *Peronospora* spp. (downy mildew) on cabbage (e. g. *P. brassicae*), oilseed rape (e. g. *P. parasitica*)*,* onions (e. g. *P. destructor*)*,* tobacco (*P. tabacina*) and soybeans (e. g. *P. manshurica*)*; Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (syn. *Leptosphaeria biglobosa* and *L. maculans*: root and stem rot) on oilseed rape and cabbage, *P. betae* (root rot, leaf spot and damping-off) on sugar beets and *P. zeae-maydis* (syn. *Phyllostica zeae*) on corn; *Phomopsis* spp. on sunflowers, vines (e. g. *P. viticola*: can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*)*,* soybeans (e. g. *P. megasperma,* syn. *P. sojae*), potatoes and tomatoes (e. g. *P. infestans*: late blight) and broad-leaved trees (e. g. *P. ramorum*: sudden oak death); *Plasmodiophora brassicae* (club root) on cabbage,oilseed rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits (e. g. *P. leucotricha* on apples) and curcurbits (*P. xanthii*); *Polymyxa* spp., e. g. on cereals, such as barley and wheat (*P. graminis*) and sugar beets (*P. betae*) and thereby transmitted viral diseases; *Pseudocercosporella herpotrichoides* (syn. *Oculimacula yallundae, O. acuformis*: eye-spot, teleomorph: *Tapesia yallundae*) on cereals, e. g. wheat or barley; *Pseudoperonospora* (downy mildew) on various plants, e. g. *P. cubensis* on cucurbits or *P. humilion* hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii*(orange rust) on sugar cane and *P. asparagi*on asparagus; *Pyrenopeziza spp.,* e.g. *P. brassicae* on oilseed rape; *Pyrenophora* (anamorph: *Drechslera) tritici-repentis* (tan spot) on wheat or *P. teres* (net blotch) on barley; *Pyricularia* spp., e. g. *P. oryzae* (teleomorph: *Magnaporthe grisea*: rice blast) on rice and *P. grisea* on turf and cereals; *Pythium* spp. (damping-off) on turf, rice, corn, wheat, cotton, oilseed rape, sunflowers, soybeans, sugar beets, vegetables and various other plants (e. g. *P. ultimum* or *P. aphanidermatum*) and *P. oligandrum* on mushrooms; *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, oilseed rape, potatoes, sugar beets, vegetables and various other plants, e. g. *R. solani*(root and stem rot) on soybeans, *R. solani*(sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* and *R. commune* (scald) on barley, rye and triticale; *Sarocladium oryzae* and *S. attenuatum* (sheath rot) on rice; *Sclerotinia* spp. (stem rot or white mold) on vegetables (*S. minor* and *S. sclerotiorum*) and field crops, such as oilseed rape, sunflowers (e. g. *S. sclerotiorum)* and soybeans, *S. rolfsii* (syn. *Athelia rolfsii*) on soybeans, peanut, vegetables, corn, cereals and ornamentals; *Septoria* spp. on various plants, e. g. *S. glycines* (brown spot) on soybeans, *S. tritici* (syn. *Zymoseptoria tritici,* Septoria blotch) on wheat and *S.* (syn. *Stagonospora*) *nodorum* (Stagonospora blotch) on cereals; *Uncinula* (syn. *Erysiphe*) *necator* (powdery mildew, anamorph: *Oidium tuckeri*) on vines; *Setosphaeria* spp. (leaf blight) on corn (e. g. *S. turcicum,* syn. *Helminthosporium turcicum*) and turf; *Sphacelotheca* spp. (smut) on corn, (e. g. *S. reiliana,* syn. *Ustilago reiliana:* head smut), sorghum und sugar cane; *Sphaerotheca fuliginea* (syn. *Podosphaera xanthii*: powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeosphaeria*] *nodorum,* syn. *Septoria nodorum*) on wheat; *Synchytrium endobioticum* on potatoes (potato wart disease); *Taphrina* spp., e. g. *T. deformans* (leaf curl disease) on peaches and *T. pruni*(plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn*. T. caries,* wheat bunt) and *T. controversa* (dwarf bunt) on wheat; *Trichoderma harzianum on mushrooms*; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoii*)*,* sugar beets (e. g. *U. betae* or *U. beticola*) and on pulses (e.g. *U. vignae, U. pisi, U. viciae-fabae* and *U. fabae*)*; Ustilago* spp. (loose smut) on cereals (e. g. *U. nuda and U. avaenae),* corn (e. g. *U. maydis*: corn smut) and sugar cane; *Venturia* spp. (scab) on apples (e. g. *V*. *inaequalis*) and pears; and *Verticillium* spp. (wilt) on various plants, such as fruits and ornamentals, vines, soft fruits, vegetables and field crops, e. g. *V. longisporum* on oilseed rape, *V. dahliae* on strawberries, oilseed rape, potatoes and tomatoes, and *V*. *fungicola* on mushrooms; *Zymoseptoria tritici* on cereals.

The compounds I and compositions thereof, respectively, are also suitable for controlling harmful microorganisms in the protection of stored products or harvest, and in the protection of materials.

The term "stored products or harvest" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. Preferably, "stored products" is understood to denote natural substances of plant origin and their processed forms, more preferably fruits and their processed forms, such as pomes, stone fruits, soft fruits and citrus fruits and their processed forms. The compounds I and compositions thereof according the present invention can prevent disadvantageous effects such as decay, discoloration or mold.

The term "protection of materials" is to be understood to denote the protection of technical and non-living materials, such as adhesives, glues, wood, paper, paperboard, textiles, leather, paint dispersions, plastics, cooling lubricants, fiber, or fabrics; against the infestation and destruction by harmful microorganisms, such as fungi and bacteria. As to the protection of materials, particular attention is paid to the following harmful fungi: Ascomycetes, such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes, such as *Coniophora* spp., *Coriolus* spp., *Gloeophyllum* spp*., Lentinus* spp*., P*/*eurotus* spp*., Poria* spp., *Serpula* spp*.* and *Tyromyces* spp.; Deuteromycetes, such as *Aspergillus* spp., *Cladosporium* spp., *Penicillium* spp., *Trichoderma* spp., *Alternaria* spp., *Paecilomyces* spp.; and Zygomycetes, such as *Mucor*spp*..* In the protection of stored products and harvest in addition the following yeast fungi are worthy of note: *Candida* spp. and *Saccharomyces cerevisae.*

The compounds I and compositions thereof, respectively, may be used for improving the health of a plant. The invention also relates to a method for improving plant health by treating a plant, its propagation material, and/or the locus where the plant is growing or is to grow with an effective amount of compounds I and compositions thereof, respectively.

The term "plant health" is to be understood to denote a condition of the plant and/or its products which is determined by several indicators alone or in combination with each other, such as yield (e. g. increased biomass and/or increased content of valuable ingredients), plant vigor (e. g. improved plant growth and/or greener leaves ("greening effect")), quality (e. g. improved content or composition of certain ingredients), and tolerance to abiotic and/or biotic stress. The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "fungicidally effective amount" denotes an amount of the composition or of the compounds I, which is sufficient for controlling harmful fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, and alkylated naphthalenes; alcohols, e. g. ethanol, propanol, butanol, benzyl alcohol, cyclohexanol, glycols; DMSO; ketones, e. g. cyclohexanone; esters, e. g. lactates, carbonates, fatty acid esters, gamma-butyrolactone; fatty acids; phosphonates; amines; amides, e. g. *N-*methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and of alkyl naphthalenes, sulfosuccinates, or sulfosuccinamates. Examples of sulfates are sulfates of fatty acids, of oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, *N-*substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of *N-*substituted fatty acid amides are fatty acid glucamides or fatty acid alkanolamides. Examples of esters are fatty acid esters, glycerol esters, or monoglycerides. Examples of sugar-based surfactants are sorbitans, ethoxylated sorbitans, sucrose and glucose esters, or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide, and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives, such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.

### ii) Dispersible concentrates (DC)

5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.

### iii) Emulsifiable concentrates (EC)

15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.

### iv) Emulsions (EW, EO, ES)

5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition, up to 40 wt% binder (e. g. polyvinyl alcohol) is added.

### vi) Water-dispersible granules and water-soluble granules (WG, SG)

50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.

### viii) Gel (GW, GF)

In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.

### ix) Microemulsion (ME)

5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.

### x) Microcapsules (CS)

An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.

### xi) Dustable powders (DP, DS)

1-10 wt% of a compound I are ground finely and mixed intimately with solid carrier (e. g. finely divided kaolin) ad 100 wt%.

### xii) Granules (GR, FG)

0.5-30 wt% of a compound I is ground finely and associated with solid carrier (e. g. silicate) ad 100 wt%. Granulation is achieved by extrusion, spray-drying or fluidized bed.

### xiii) Ultra-low volume liquids (UL)

1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95 %, preferably between 0.1 and 90%, more preferably between 1 and 70 %, and in particular between 10 and 60 %, by weight of active substance. The active substances are employed in a purity of from 90 % to 100 %, preferably from 95-% to 100 % (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60 % by weight, preferably from 0.1 to 40 %, in the ready-to-use preparations. Application can be carried out before or during sowing. Methods for applying compound I and compositions thereof, respectively, onto plant propagation material, especially seeds, include dressing, coating, pelleting, dusting, soaking, as well as in-furrow application methods. Preferably, compound I or the compositions thereof, respectively, are applied on to the plant propagation material by a method such that germination is not induced, e. g. by seed dressing, pelleting, coating, and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials, such as seeds, e. g. by dusting, coating, or drenching, amounts of active substance of from 0.1 to 1000 g, preferably from 1 to 1000 g, more preferably from 1 to 100 g and most preferably from 5 to 100 g, per 100 kg of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix, or, if appropriate, not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial, or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on microorganisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

When living microorganisms, such as microbial pesticides from groups L1), L3) and L5), form part of such kit, it must be taken care that choice and amounts of the components (e. g. chemical pesticides) and of the further auxiliaries should not influence the viability of the microbial pesticides in the composition mixed by the user. Especially for bactericides and solvents, compatibility with the respective microbial pesticide has to be taken into account.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-*N-*methyl-acetamide (A.1.18), pyribencarb (A.1.19), triclopyricarb/chlorodincarb (A.1.20), famoxadone (A.1.21), fenamidone (A.1.21), methyl-*N-*[2-[(1,4-dimethyl-5-phenyl-pyrazol-3-yl)oxylmethyl]phenyl]-*N*-methoxy-carbamate (A.1.22), metyltetrapole (A.1.25), (*Z*,2*E*)-5-[1-(2,4-dichlorophenyl)pyrazol-3-yl]-oxy-2-methoxyimino-*N*,3-dimethyl-pent-3-en-amide (A.1.34), (*Z*,2*E*)-5-[l-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*-3-ethyl-1,1-dimethyl-indan-4-yl]pyridine-3-carboxamide (A.3.35), 2-(difluoromethyl)-*N*(1,1-dimethyl-3-propyl-indan-4-yl)pyridine-3-carboxamide (A.3.36), 2-(difluoromethyl)-*N*[(3*R*-1,1-dimethyl-3-propyl-indan-4-yl]pyridine-3-carboxamide (A.3.37), 2-(difluoromethyl)-*N*(3-isobutyl-1,1-dimethyl-indan-4-yl)pyridine-3-carboxamide (A.3.38), 2-(difluoromethyl)-*N*[(3*R*-3-isobutyl-1,1-dimethyl-indan-4-yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), ipfentrifluconazole (B.1.37), mefentrifluconazole (B.1.38), 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N-*ethyl-2-[(3-ethynyl-8-methyl-6-quinolyl)oxy]butanamide (D.1.8), -*N-*ethyl-2-[(3-ethynyl-8-methyl-6-quinolyl)oxy]-2-methyl sulfanyl-acetamide (D.1.9), 2-[(3-ethynyl-8-methyl-6-quinolyl)oxy]-*N*(2-fluoroethyl)butanamide (D.1.10), 2-[(3-ethynyl-8-methyl-6-quinolyl)oxy]-*N-*(2-fluoroethyl)-2-methoxy-acetamide (D.1.11), 2-[(3-ethynyl-8-methyl-6-quinolyl)oxy]-*N-*propyl-butanamide (D.1.12), 2-[(3-ethynyl-8-methyl-6-quinolyl)oxy]-2-methoxy-*N-*propyl-acetamide (D.1.13), 2-[(3-ethynyl-8-methyl-6-quinolyl)oxy]-2-methylsulfanyl-*N-*propyl-acetamide (D.1.14), 2-[(3-ethynyl-8-methyl-6-quinolyl)oxy]-*N*(2-fluoroethyl)-2-methylsulfanyl-acetamide (D.1.15), 4-(2-bromo-4-fluorophenyl)-*N*(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N-*tetralin-1-yl-pyridine-2-carboxamide (G.5.6), 4-[1-[2-[5-cyclopropyl-3-(difluoromethyl)pyrazol-1-yl]acetyl]-4-piperidyl]-*N-*tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N-*tetralin-1-yl-pyridine-2-carboxamide (G.5.9), 4-[1-[2-[3,5-bis(trifluoromethyl)pyrazol-1-yl]acetyl]-4-piperidyl]-*N-*tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N-*ethyl-*N-*methyl formamidine (K.1.28), *N*'-[4-[[3-[(4-chlorophenyl)methyl]-1,2,4-thiadiazol-5-yl]-oxy]-2,5-dimethyl-phenyl]-*N*-ethyl-*N-*methyl-formamidine (K.1.29), *N*'-(5-bromo-6-indan-2-yloxy-2-methyl-3-pyridyl)-*N-*ethyl-*N-*methyl-formamidine (K.1.30), *N*'-[5-bromo-6-[1-(3,5-difluorophenyl)ethoxy]-2-methyl-3-pyridyl]-*N-*ethyl-*N-*methyl-formamidine (K.1.31), *N-*[5-bromo-6-(4-isopropylcyclohexoxy)-2-methyl-3-pyridyl]-*N*-ethyl-*N-*methyl-formamidine (K.1.32), *N'-*[5-bromo-2-methyl-6-(1-phenylethoxy)-3-pyridyl]-*N-*ethyl-*N-*methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N-*ethyl-*N*-methyl formamidine (K.1.35), 2-(4-chloro-phenyl)-*N-*[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1-(4,6-dimethoxy-pyrimidin-2-yl)-2-methyl-1*H-*benzoimidazole (K.1.39), ethyl (2)-3-amino-2-cyano-3-phenyl-prop-2-enoate (K.1.40), picarbutrazox (K.1.41), pentyl *N*-[6-[[(2)-[(1-methyltetrazol-5-yl)-phenyl-methylene]amino]oxymethyl]-2-pyridyl]carbamate (K.1.42), but-3-ynyl *N-*[6-[[(2)-[(1-methyltetrazol-5-yl)-phenyl-methylene]amino]oxymethyl]-2-pyridyl]carbamate (K.1.43), ipflufenoquin (K.1.44), quinofumelin (K.1.47), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,1-dimethyl-4-phenoxy-phenyl)-*N-*ethyl-*N-*methyl-formamidine (K.1.53), pyrifenamine (K.1.54);
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as *B. velezensis*)*, B. megaterium, B. mojavensis, B. mycoides, B. pumilus, B. simplex, B. solisalsi, B. subtilis, B. subtilis* var. *amyloliquefaciens, B. velezensis, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum*), *Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: harpin protein, *Reynoutria sachalinensis* extract;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae*, *B. t.* ssp. *kurstaki*, *B. t.* ssp. *tenebrionis*, *Beauveria bassiana*, *B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpazea* nucleopolyhedrovirus (HzNPV), *Helicoverpa zea*single capsid nucleopolyhedrovirus (HzSNPV), *Heterorhabditis bacteriophora, Isaria fumosorosea, Lecanicilliumlongisporum, L. muscarium, Metarhizium anisopliae, M. anisopliae* var. *anisopliae*, *M. anisopliae* var. *acridum*, *Nomuraea rileyi*, *Paecilomyces fumosoroseus*, *P. lilacinus, Paenibacillus popilliae, Pasteuria* spp., *P. nishizawae, P. penetrans*, *P. ramosa*, *P. thornea*, *P. usgae*, *Pseudomonas fluorescens*, *Spodoptera littoral is*nucleopolyhedrovirus (SpliNPV), *Steinernema carpocapsae*, *S. feltiae, S. kraussei*, *Streptomyces galbus*, *S. microflavus*,
   L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl 1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E,Z,Z*)-3,8,11-tetradecatrienyl acetate, (*Z*,*E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes*, Neem oil, Quillay extract;
   L5) Microbial pesticides with plant stress reducing, plant growth regulator, plant growth promoting and/or yield enhancing activity: *Azospirillum amazonense*, *A. brasilense*, *A. lipoferum*, *A. irakense*, *A. halopraeferens, Bradyrhizobium* spp., *B. elkanii*, *B. japonicum, B. liaoningense*, *B. lupini, Delftia acidovorans*, *Glomus intraradices, Mesorhizobium* spp., *Rhizobium leguminosarum* bv. *phaseoli, R. I*. bv. *trifolii, R. I.* bv. *viciae, R. tropici*, *Sinorhizobium mellloti*;
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat, mepiquat chloride, naphthaleneacetic acid, *N*-6-benzyladenine, paclobutrazol, prohexadione, prohexadione-calcium, prohydrojasmon, thidiazuron, triapenthenol, tributyl phosphorotrithioate, 2,3,5-tri-iodobenzoic acid, trinexapac-ethyl, uniconazole;
N) Herbicides from classes N.1 to N.15
   N.1 Lipid biosynthesis inhibitors: alloxydim, alloxydim-sodium, butroxydim, clethodim, clodinafop, clodinafop-propargyl, cycloxydim, cyhalofop, cyhalofop-butyl, diclofop, diclofopmethyl, fenoxaprop, fenoxaprop-ethyl, fenoxaprop-P, fenoxaprop-P-ethyl, fluazifop, fluazifopbutyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-chloro-4-cyclo¬propyl-2'-fluoro[1,1'-biphenyl]-3-yl)-5-hydroxy-2,2,6,6-tetramethyl-2*H-*pyran-3(6*H*)-one (1312337-72-6); 4-(2',4'-dichloro-4-cyclopropyl[1,1'-biphenyl]-3-yl)-5-hydroxy-2,2,6,6-tetramethyl-2*H*-pyran-3(6*H*)-one (1312337-45-3); 4-(4'-chloro-4-ethyl-2'-fluoro[1,1'-biphenyl]-3-yl)-5-hydroxy-2,2,6,6-tetramethyl-2*H*-pyran-3(6*H*)-one (1033757-93-5); 4-(2',4'-dichloro-4-ethyl[1,1'-biphenyl]-3-yl)-2,2,6,6-tetramethyl-2*H*-pyran-3,5(4*H*,6*H)*-dione (1312340-84-3); 5-(acetyloxy)-4-(4'-chloro-4-cyclopropyl-2'-fluoro[1,1'-biphenyl]-3-yl)-3,6-dihydro-2,2,6,6-tetramethyl-2*H*-pyran-3-one (1312337-48-6); 5-(acetyloxy)-4-(2',4'-dichloro-4-cyclopropyl-[1,1'-biphenyl]-3-yl)-3,6-dihydro-2,2,6,6-tetramethyl-2*H-*pyran-3-one; 5-(acetyloxy)-4-(4'-chloro-4-ethyl-2'-fluoro[1,1'-biphenyl]-3-yl)-3,6-dihydro-2,2,6,6-tetramethyl-2*H-*pyran-3-one (1312340-82-1); 5-(acetyloxy)-4-(2',4'-dichloro-4-ethyl[1,1'-biphenyl]-3-yl)-3,6-dihydro-2,2,6,6-tetramethyl-2*H*-pyran-3-one (1033760-55-2); 4-(4'-chloro-4-cyclopropyl-2'-fluoro[1,1'-biphenyl]-3-yl)-5,6-dihydro-2,2,6,6-tetramethyl-5-oxo-2*H-*pyran-3-yl carbonic acid methyl ester (1312337-51-1); 4-(2',4'-dichloro-4-cyclopropyl-[1,1'-biphenyl]-3-yl)-5,6-dihydro-2,2,6,6-tetramethyl-5-oxo-2*H-*pyran-3-yl carbonic acid methyl ester; 4-(4'-chloro-4-ethyl-2'-fluoro[1,1'-biphenyl]-3-yl)-5,6-dihydro-2,2,6,6-tetramethyl-5-oxo-2*H-*pyran-3-yl carbonic acid methyl ester (1312340-83-2); 4-(2',4'-dichloro-4-ethyl¬[1,1'-biphenyl]-3-yl)-5,6-dihydro-2,2,6,6-tetramethyl-5-oxo-2*H-*pyran-3-yl carbonic acid methyl ester (1033760-58-5); benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate, vernolate;
   N.2 ALS inhibitors: amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuronmethyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl, tritosulfuron, imazamethabenz, imazamethabenz-methyl, imazamox, imazapic, imazapyr, imazaquin, imazethapyr; cloransulam, cloransulam-methyl, diclosulam, flumetsulam, florasulam, metosulam, penoxsulam, pyrimisulfan, pyroxsulam; bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methyl¬ethyl ester (420138-41-6), 4-[[[2-[(4,6-dimethoxy-2-pyrimidinyl)oxy]phenyl]¬methyl]amino]-benzoic acid propyl ester (420138-40-5), *N*-(4-bromophenyl)-2-[(4,6-dimethoxy-2-pyrimidinyl)oxy]benzenemethanamine (420138-01-8); flucarbazone, flucarbazone-sodium, propoxycarbazone, propoxycarbazone-sodium, thiencarbazone, thiencarbazone-methyl; triafamone;
   N.3 Photosynthesis inhibitors: amicarbazone; chlorotriazine; ametryn, atrazine, chloridazone, cyanazine, desmetryn, dimethametryn,hexazinone, metribuzin, prometon, prometryn, propazine, simazine, simetryn, terbumeton, terbuthylazin, terbutryn, trietazin; chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron, thiadiazuron, desmedipham, karbutilat, phenmedipham, phenmediphamethyl, bromofenoxim, bromoxynil and its salts and esters, ioxynil and its salts and esters, bromacil, lenacil, terbacil, bentazon, bentazon-sodium, pyridate, pyridafol, pentanochlor, propanil; diquat, diquat-dibromide, paraquat, paraquat-dichloride, paraquat-dimetilsulfate;
   N.4 protoporphyrinogen-IX oxidase inhibitors: acifluorfen, acifluorfen-sodium, azafenidin, bencarbazone, benzfendizone, bifenox, butafenacil, carfentrazone, carfentrazone-ethyl, chlormethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (353292-31-6), *N-*ethyl-3-(2 ,6-d ichloro-4-trifluoromethylphenoxy)-5-methyl-1-*H-*pyrazole-1-carboxamide (452098-92-9), *N*-tetrahydrofurfuryl-3-(2,6-dichloro-4-trifluoromethylphenoxy)-5-methyl-1*H-*pyrazole-1-carboxamide (915396-43-9), *N-*ethyl-3-(2-chloro-6-fluoro-4-trifluoromethyl¬phenoxy)-5-methyl-1*H-*pyrazole-1-carboxamide (452099-05-7), *N-*tetrahydro¬furfuryl-3-(2-chloro-6-fluoro-4-trifluoro¬methylphenoxy)-5-methyl-1*H-*pyrazole-1-carboxamide (452100-03-7), 3-[7-fluoro-3-oxo-4-(prop-2-ynyl)-3,4-dihydro-2*H-*benzo[1,4]oxazin-6-yl]-1,5-dimethyl-6-thioxo-[1,3,5]triazinan-2,4-dione (451484-50-7), 2-(2,2,7-trifluoro-3-oxo-4-prop-2-ynyl-3,4-dihydro-2*H*-benzo[1,4]oxazin-6-yl)-4,5,6,7-tetrahydro-isoindole-1,3-dione (1300118-96-0), 1-methyl-6-trifluoro¬methyl-3-(2,2,7-tri-fluoro-3-oxo-4-prop-2-ynyl-3,4-dihydro-2*H*-benzo[1,4]oxazin-6-yl)-1*H*-pyrimidine-2,4-dione (1304113-05-0), methyl (*E*)-4-[2-chloro-5-[4-chloro-5-(difluoromethoxy)-1*H-*methyl-pyrazol-3-yl]-4-fluoro-phenoxy]-3-methoxy-but-2-enoate (948893-00-3), 3-[7-chloro-5-fluoro-2-(trifluoromethyl)-1*H-*benzimidazol-4-yl]-1-methyl-6-(trifluoromethyl)-1*H-*pyrimidine-2,4-dione (212754-02-4);
   N.5 Bleacher herbicides: beflubutamid, diflufenican, fluridone, flurochloridone, flurtamone, norflurazon, picolinafen, 4-(3-trifluoromethyhphenoxy)-2-(4-trifluoromethylphenyl)pyrimidine (180608-33-7); benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone; aclonifen, amitrole, flumeturon;
   N.6 EPSP synthase inhibitors: glyphosate, glyphosate-isopropylammonium, glyposate-potassium, glyphosate-trimesium (sulfosate);
   N.7 Glutamine synthase inhibitors: bilanaphos (bialaphos), bilanaphos-sodium, glufosinate, glufosinate-P, glufosinate-ammonium;
   N.8 DHP synthase inhibitors: asulam;
   N.9 Mitosis inhibitors: benfluralin, butralin, dinitramine, ethalfluralin, fluchloralin, oryzalin, pendimethalin, prodiamine, trifluralin; amiprophos, amiprophos-methyl, butamiphos; chlorthal, chlorthal-dimethyl, dithiopyr, thiazopyr, propyzamide, tebutam; carbetamide, chlorpropham, flamprop, flamprop-isopropyl, flamprop-methyl, flamprop-M-isopropyl, flamprop-M-methyl, propham;
   N.10 VLCFA inhibitors: acetochlor, alachlor, butachlor, dimethachlor, dimethenamid, dimethenamid-P, metazachlor, metolachlor, metolachlor-S, pethoxamid, pretilachlor, propachlor, propisochlor, thenylchlor, flufenacet, mefenacet, diphenamid, naproanilide, napropamide, napropamide-M, fentrazamide, anilofos, cafenstrole, fenoxasulfone, ipfencarbazone, piperophos, pyroxasulfone, isoxazoline compounds of the formulae II.1, II.2, II.3, II.4, II.5, II.6, II.7, II.8 and II.9 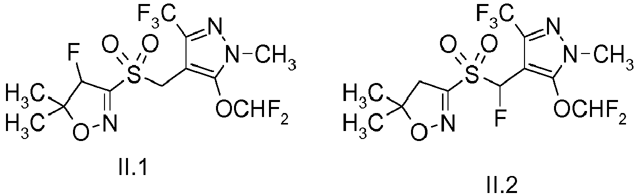 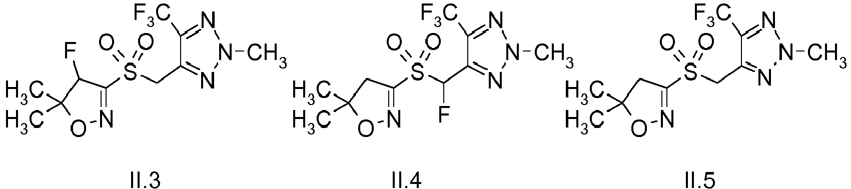 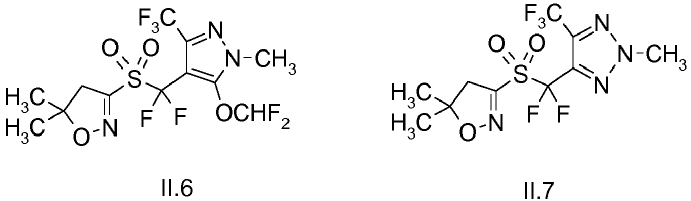 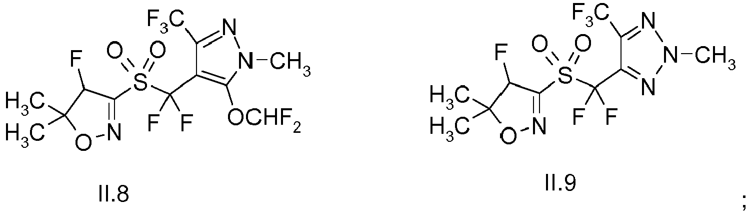
   N.11 Cellulose biosynthesis inhibitors: chlorthiamid, dichlobenil, flupoxam, indaziflam, isoxaben, triaziflam, 1-cyclohexyl-5-pentafluorphenyloxy-14-[1,2,4,6]thiatriazin-3-ylamine (175899-01-1);
   N.12 Decoupler herbicides: dinoseb, dinoterb, DNOC and its salts;
   N.13 Auxinic herbicides: 2,4-D and its salts and esters, clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters (943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 4-amino-3-chloro-6-(4-chloro-2-fluoro-3-methoxyphenyl)-5-fluoropyridine-2-carboxylic acid, benzyl 4-amino-3-chloro-6-(4-chloro-2-fluoro-3-methoxyphenyl)-5-fluoropyridine-2-carboxylate (1390661-72-9);
   N.14 Auxin transport inhibitors: diflufenzopyr, diflufenzopyr-sodium, naptalam, naptalam-sodium;
   N.15 Other herbicides: bromobutide, chlorflurenol, chlorflurenol-methyl, cinmethylin, cumyluron, cyclopyrimorate (499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (403640-27-7), methyl azide, methyl bromide, methyldymron, methyl iodide, MSMA, oleic acid, oxaziclomefone, pelargonic acid, pyributicarb, quinoclamine, tridiphane;
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; DDT, methoxychlor;
   O.4 Nicotinic acetylcholine receptor agonists (nAChR): acetamiprid, clothianidin, cycloxaprid, dinotefuran, imidacloprid, nitenpyram, thiacloprid, thiamethoxam; 4,5-dihydro-*N*-nitro-1-(2-oxiranylmethyl)-1*H-*imidazol-2-amine, (2*E*)-1-[(6-chloropyridin-3-yl)methyl]-*N*'-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor, flupyradifurone, triflumezopyrim;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen;
   O.8 miscellaneous non-specific (multi-site) inhibitors: methyl bromide and other alkyl halides; chloropicrin, sulfuryl fluoride, borax, tartar emetic;
   O.9 Chordotonal organ TRPV channel modulators: pymetrozine, pyrifluquinazon; flonicamid; O.10 Mite growth inhibitors: clofentezine, hexythiazox, diflovidazin; etoxazole;
   O.11 Microbial disruptors of insect midgut membranes: *Bacillus thuringiensis, Bacillus sphaericus* and the insecticdal proteins they produce: *Bacillus thuringiensis* subsp. *israelensis, Bacillus sphaericus, Bacillus thuringiensis* subsp. *aizawai, Bacillus thuringiensis* subsp. *kurstaki, Bacillus thuringiensis* subsp. *tenebrionis,* the Bt crop proteins: Cry1Ab, Cry1Ac, Cry1Fa, Cry2Ab, mCry3A, Cry3Ab, Cry3Bb, Cry34/35Ab1;
   0.12 Inhibitors of mitochondrial ATP synthase: diafenthiuron; azocyclotin, cyhexatin, fenbutatin oxide, propargite, tetradifon;
   0.13 Uncouplers of oxidative phosphorylation via disruption of the proton gradient: chlorfenapyr, DNOC, sulfluramid;
   0.14 Nicotinic acetylcholine receptor (nAChR) channel blockers: bensultap, cartap hydrochloride, thiocyclam, thiosultap sodium;
   0.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
   0.16 Inhibitors of the chitin biosynthesis type 1: buprofezin;
   0.17 Moulting disruptors: cyromazine;
   0.18 Ecdyson receptor agonists: methoxyfenozide, tebufenozide, halofenozide, fufenozide, chromafenozide;
   0.19 Octopamin receptor agonists: amitraz;
   O.20 Mitochondrial complex III electron transport inhibitors: hydramethylnon, acequinocyl, fluacrypyrim, bifenazate;
   0.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cyanophenyl)-1-[3-(trifluoromethyl)phenyl]ethylidene]-*N*[4-(difluoromethoxy)phenyl]-hydrazinecar-boxamide, *N*-(3-chloro-2-methyl phenyl)-2-[(4-chlorophenyl)-[4-[methyl(methylsulfonyl)-amino]phenyl]methylene]-hydrazinecarboxamide;
   O.23 Inhibitors of the of acetyl CoA carboxylase: spirodiclofen, spiromesifen, spirotetramat, spiropidion;
   O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen;
   O.26 Ryanodine receptor-modulators: flubendiamide, chlorantraniliprole, cyantraniliprole, cyclaniliprole, tetraniliprole; (*R*)-3-chloro-*N*¹-2-methyl-4-[1,2,2,2 -tetrafluoro-1-(trifluoromethyl)-ethyl]phenyl}-*N*²-(1-methyl-2-methylsulfonylethyl)phthalamide, (*S*)-3-chloro-*N*¹-{2-methyl-4-[1,2,2,2-tetrafluoro-1-(trifluoromethyl)ethyl]phenyl}-*N*²-(1-methyl-2-methylsulfonylethyl)-phthalamide, methyl-2-[3,5-dibromo-2-({[3-bromo-1-(3-chloropyridin-2-yl)-1*H*-pyrazol-5-yl]-carbonyl}amino)benzoyl]-1,2-dimethylhydrazinecarboxylate; *N-*[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; *N*-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; *N*-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; *N-*[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; *N-*[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; *N*-[2-(5-amino-1,3,4-thiadiazol-2-yl)-4-chloro-6-methylphenyl]-3-bromo-1-(3-chloro-2-pyridinyl)-1*H-*pyrazole-5-carboxamide; 3-chloro-1-(3-chloro-2-pyridinyl)-*N*-[2,4-dichloro-6-[[(1-cyano-1-methylethyl)amino]carbonyl]phenyl]-1*H-*pyrazole-5-carboxamide; tetrachlorantraniliprole; *N-*[4-chloro-2-[[(1,1-dimethylethyl)amino]carbonyl]-6-methylphenyl]-1-(3-chloro-2-pyridinyl)-3-(fluoromethoxy)-1*H*-pyrazole-5-carboxamide; cyhalodiamide;
   O.27: Chordotonal organ Modulators - undefined target site: flonicamid;
   O.28. insecticidal active compounds of unknown or uncertain mode of action: afidopyropen, afoxolaner, azadirachtin, amidoflumet, benzoximate, broflanilide, bromopropylate, chinomethionat, cryolite, dicloromezotiaz, dicofol, flufenerim, flometoquin, fluensulfone, fluhexafon, fluopyram, fluralaner, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1*H*-1,2,4-triazole-5-amine, *Bacillus firmus* I-1582; flupyrimin; fluazaindolizine; 4-[5-(3,5-dichlorophenyl)-5-(trifluoromethyl)-4*H*-isoxazol-3-yl]-2-methyl-*N*-(1-oxothietan-3-yl)benzamide; fluxametamide; 5-[3-[2,6-dichloro-4-(3,3-dichloroallyloxy)phenoxy]propoxy]-1*H-*pyrazole; 4-cyano-*N-*[2-cyano-5-[[2,6-dibromo-4-[1,2,2,3,3,3-hexafluoro-1-(trifluoromethyl)propyl]phenyl]carbamoyl]phenyl]-2-methyl-benzamide; 4-cyano-3-[(4-cyano-2-methyl-benzoyl)amino]-*N-*[2,6-dichloro-4-[1,2,2,3,3,3-hexafluoro-1-(trifluoromethyl)propyl]phenyl]-2-fluoro-benzamide; *N-*[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; *N-*[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; *N-*[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-*N-*[2-cyano-5-[[2,6-dichloro-4-[1,2,2,3,3,3-hexafluoro-1-(trifluoromethyl)propyl]phenyl]carbamoyl]phenyl]-2-methyl-benzamide; 4-cyano-*N-*[2-cyano-5-[[2,6-dichloro-4-[1,2,2,2-(trifiuoromethyl)ethyl]phenyl]carbamoyl]phenyl]-2-methyl-benzamide; *N-*[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; *N*-methylsulfonyl-6-[2-(3-pyridyl)thiazol-5-yl]pyridine-2-carboxamide; *N-*methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-isopropyl-*N*,5-dimethyl-*N*-pyridazin-4-yl-pyrazole-4-carboxamide; 1-(1,2-dimethylpropyl)-*N-*ethyl-5-methyl-*N-*pyridazin-4-yl-pyrazole-4-carboxamide; *N*,5-dimethyl-*N*-pyridazin-4-yl-1-(2,2,2-trifluoro-1-methyl-ethyl)pyrazole-4-carboxamide; 1-[1-(1-cyanocyclopropyl)ethyl]-*N-*ethyl-5-methyl-*N-*pyridazin-4-yl-pyrazole-4-carboxamide; *N-*ethyl-1-(2-fluoro-1-methyl-propyl)-5-meth-yl-*N-*pyridazin-4-yl-pyrazole-4-carboxamide; 1-(1,2-dimethylpropyl)-N,5-dimethyl-*N-*pyridazin-4-yl-pyrazole-4-carboxamide; 1-[1-(1-cyanocyclopropyl)ethyl]-*N*,5-dimethyl-*N*-pyridazin-4-yl-pyrazole-4-carboxamide; *N*-methyl-1-(2-fluoro-1-methyl-propyl]-5-methyl-*N-*pyridazin-4-yl-pyrazole-4-carboxamide; 1-(4,4-difluorocyclohexyl)-*N*-ethyl-5-methyl-*N*-pyridazin-4-yl-pyrazole-4-carboxamide; 1-(4,4-difluorocyclohexyl)-*N*,5-dimethyl-*N*-pyridazin-4-yl-pyrazole-4-carboxamide, *N*-(-methylethyl)-2-(3-pyridinyl)-2*H-*indazole-4-carboxamide; *N-*cyclopropyl-2-(3-pyridinyl)-2*H*-indazole-4-carboxamide; *N-*cyclohexyl-2-(3-pyridinyl)-2*H-*indazole-4-carboxamide; 2-(3-pyridinyl)-*N*-(2,2,2-trifluoroethyl)-2*H*-indazole-4-carboxamide;2-(3-pyridinyl)-*N*-[(tetrahydro-2-furanyl)methyl]-2*H*-indazole-5-carboxamide; methyl 2-[[2-(3-pyridinyl)-2*H*-indazol-5-yl]carbonyl]hydrazinecarboxylate; *N-*[(2,2-difluorocyclopropyl)methyl]-2-(3-pyridinyl)-2*H-*indazole-5-carboxamide; *N-*(2,2-difluoropropyl)-2-(3-pyridinyl)-2*H*-indazole-5-carboxamide; 2-(3-pyridinyl)-*N*(2-pyrimidinylmethyl)-2*H-*indazole-5-carboxamide; *N*[(5-methyl-2-pyrazinyl)methyl]-2-(3-pyridinyl)-2*H*-indazole-5-carboxamide, tyclopyrazoflor; sarolaner, lotilaner, *N*[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; M.UN.22a 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine, 4-[5-(3,5-dichlorophenyl)-5-(trifluoromethyl)-4*H*-isoxazol-3-yl]-*N*-[(4*R*-2-ethyl-3-oxo-isoxazolidin-4-yl]-2-methyl-benzamide, 4-[5-(3,5-dichloro-4-fluoro-phenyl)-5-(trifluoromethyl)-4*H*-isoxazol-3-yl]-*N-*[(4*R*)-2-ethyl-3-oxo-isoxazolidin-4-yl]-2-methyl-benzamide; *N-*[4-chloro-3-(cyclopropylcarbamoyl)phenyl]-2-methyl-5-(1,1,2,2,2-pentafluoroethyl)-4-(trifluoromethyl)pyrazole-3-carboxamide, *N*[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; chloro-*N-*(1-cyanocyclopropyl)-5-[1-[2-methyl-5-(1,1,2,2,2-pentafluoroethyl)-4-(trifluoromethyl)pyrazol-3-yl]pyrazol-4-yl]benzamide, oxazosulfyl, [(2*S*,3*R*,4*R*,5*S*,6*S*)-3,5-dimethoxy-6-methyl-4-propoxy-tetrahydropyran-2-yl]-*N*-[4-[1-[4-(trifluoromethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl] N-[4-[1-[4-(trifluoromethoxy)phenyl]-1,2,4-triazol-3-yl]phenyl]carbamate, [(2*S*,3*R*,4*R*,5*S*,6*S*)-3,5-dimethoxy-6-methyl-4-propoxy-tetrahydropyran-2-yl]-*N-*[4-[1-[4-(1,1,2,2,2-pentafluoroethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl]-*N*[4-[1-[4-(1,1,2,2,2-pentafluoroethoxy)phenyl]-1,2,4-triazol-3-yl]phenyl]carbamate, (2*Z*)̅-3-(2-isopropylphenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one.

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441). Some compounds are identified by their CAS Registry Number which is separated by hyphens into three parts, the first consisting from two up to seven digits, the second consisting of two digits, and the third consisting of a single digit.

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day. In case of a mixture comprising a pesticide II selected from group L), it is preferred that the pesticide II is applied as last treatment.

According to the invention, the solid material (dry matter) of the biopesticides (with the exception of oils such as Neem oil) are considered as active components (e. g. to be obtained after drying or evaporation of the extraction or suspension medium in case of liquid formulations of the microbial pesticides).

In accordance with the present invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 x 10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as *Steinernema feltiae.*

In the binary mixtures and compositions according to the invention the weight ratio of the component 1) and the component 2) generally depends from the properties of the active components used, usually it is in the range of from 1:10,000 to 10,000:1, often it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to further embodiments of the mixtures and compositions, the weight ratio of the component 1) and the component 2) usually is in the range of from 20,000:1 to 1:10, often in the range of from 10,000:1 to 1:1, regularly in the range of from 5,000:1 to 5:1, preferably in the range of from 5,000:1 to 10:1, more preferably in the range of from 2,000:1 to 30:1, even more preferably in the range of from 2,000:1 to 100:1 and in particular in the range of from 1,000:1 to 100:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

According to further embodiments of the mixtures and compositions, the weight ratio of the component 1) and the component 2) usually is in the range of from 10:1 to 1:20,000, often in the range of from 1:1 to 1:10,000, regularly in the range of from 1:5 to 1:5,000, preferably in the range of from 1:10 to 1:5,000, more preferably in the range of from 1:30 to 1:2,000, even more preferably in the range of from 1:100 to 1:2,000 to and in particular in the range of from 1:100 to 1:1,000.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

When mixtures comprising microbial pesticides are employed in crop protection, the application rates preferably range from about 1 x 10⁶ to 5 x 10¹⁶ (or more) CFU/ha, preferably from about 1 x 10⁸ to about 1 x 10¹³ CFU/ha, and even more preferably from about 1 x 10⁹ to 5 x 10¹⁵ CFU/ha and particularly preferred even more preferably from 1 x 10¹² to 5 x 10¹⁴ CFU/ha. In the case of (entomopathogenic) nematodes as microbial pesticides (e. g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10⁹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹² CFU per 100 kg of seed.

Preference is also given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3) and (A.2.4); particularly selected from (A.2.3) and (A.2.4).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration nhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.34), (B.1.37), (B.1.38), (B.1.43) and (B.1.46); particularly selected from (B.1.5), (B.1.8), (B.1.10), (B.1.17), (B.1.22), (B.1.23), (B.1.25), (B.1.33), (B.1.34), (B.1.37), (B.138), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6),(C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (1.2.2) and (1.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44) and (K.1.47); particularly selected from (K.1.41), (K.1.44) and (K.1.47).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.
1) Many of these biopesticides have been deposited under deposition numbers mentioned herein (the prefices such as ATCC or DSM refer to the acronym of the respective culture collection, for details see e. g. here: http://www. wfcc.info/ccinfo/collection/by_acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect® from bio-ferm GmbH, Austria), *Azospirillum brasilense* Sp245 originally isolated in wheat reagion of South Brazil (Passo Fundo) at least prior to 1980 (BR 11005; e. g. GELFIX® Gramíneas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maíz® from Simbiose-Agro, Brazil; Plant Soil 331, 413-425, 2010), *Bacillus amyloliquefaciens* strain AP-188 (NRRL B-50615 and B-50331; US 8,445,255); *B. amyloliquefaciens* ssp*. plantarum* strains formerly also sometimes referred to as *B. subtilis,* recently together with *B. methylotrophicus*, and *B. velezensis* classified as *B. velezensis* (Int. J. Syst. Evol. Microbiol. 66, 1212-1217, 2016): *B. a.* ssp. *plantarum* or *B. velezensis* D747 isolated from air in Kikugawa-shi, Japan (US 20130236522 A1; FERM BP-8234; e. g. Double Nickel™ 55 WDG from Certis LLC, USA), *B. a.* ssp. *plantarum* or *B. velezensis* FZB24 isolated from soil in Brandenburg, Germany (also called SB3615; DSM 96-2; J. Plant Dis. Prot. 105, 181-197, 1998; e. g. Taegro® from Novozyme Biologicals, Inc., USA), *B. a.* ssp. *plantarum* or *B. velezensis* FZB42 isolated from soil in Brandenburg, Germany (DSM 23117; J. Plant Dis. Prot. 105, 181-197, 1998; e. g. RhizoVital® 42 from AbiTEP GmbH, Germany), *B. a.* ssp. *plantarum* or *B. velezensis* MBI600 isolated from faba bean in Sutton Bonington, Nottinghamshire, U.K. at least before 1988 (also called 1430; NRRL B-50595; US 2012/0149571 A1; e. g. Integral® from BASF Corp., USA), *B. a.* ssp. *plantarum* or *B. velezensis* QST-713 isolated from peach orchard in 1995 in California, U.S.A. (NRRL B-21661; e. g. Serenade® MAX from Bayer Crop Science LP, USA), *B. a.* ssp. *plantarum* or *B. velezensis* TJ1000 isolated in 1992 in South Dakoda, U.S.A. (also called 1BE; ATCC BAA-390; CA 2471555 A1; e. g. QuickRoots™ from TJ Technologies, Watertown, SD, USA); *B. firmus* CUCM I-1582, a variant of parental strain EIP-N1 (CNCM I-1556) isolated from soil of central plain area of Israel (WO 2009/126473, US 6,406,690; e. g. Votivo® from Bayer CropScience LP, USA), *B. pumilus* GHA 180 isolated from apple tree rhizosphere in Mexico (IDAC 260707-01; e. g. PRO-MIX® BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata® or Ballad® Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis ssp. aizawai* ABTS*-*1857 isolated from soil taken from a lawn in Ephraim, Wisconsin, U.S.A., in 1987 (also called ABG-6346; ATCC SD-1372; e. g. XenTari® from BioFa AG, Münsingen, Germany), *B. t.* ssp. *kurstaki* ABTS*-*351 identical to HD-1 isolated in 1967 from diseased Pink Bollworm black larvae in Brownsville, Texas, U.S.A. (ATCC SD-1275; e. g. Dipel® DF from Valent BioSciences, IL, USA), *B. t.* ssp. *kurstaki*SB4 isolated from *E. saccharina* larval cadavers (NRRL B-50753; e. g. Beta Pro® from BASF Agricultural Specialities (Pty) Ltd., South Africa), *B. t.* ssp. *tenebrionis* NB-176-1*,* a mutant of strain NB-125, a wild type strain isolated in 1982 from a dead pupa of the beetle Tenebrio molitor (DSM 5480; EP 585 215 B1; e. g. Novodor® from Valent BioSciences, Switzerland), *Beauveria bassiana* GHA (ATCC 74250; e. g. BotaniGard® 22WGP from Laverlam Int. Corp., USA), *B. bassiana* JW-1 (ATCC 74040; e. g. Naturalis® from CBC (Europe) S.r.l., Italy), *B. bassiana* PPRI 5339 isolated from the larva of the tortoise beetle *Conchyloctenia punctata* (NRRL 50757; e. g. BroadBand® from BASF Agricultural Specialities (Pty) Ltd., South Africa), *Bradyrhizobium elkanii* strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo®, Histick®, Hicoat® Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); *B. japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B. japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape
   (WO 1996/021358; DSM 9660; e. g. Contans® WG, Intercept® WG from Bayer CropScience AG, Germany), harpin (alpha-beta) protein (Science 257, 85-88, 1992; e. g. Messenger™ or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex® from Adermatt Biocontrol, Switzerland; Diplomata® from Koppert, Brazil; Vivus® Max from AgBiTech Pty Ltd., Queensland, Australia), *Helicoverpa zea* single capsid nucleopolyhedrovirus (HzSNPV) (e. g. Gemstar® from Certis LLC, USA), *Helicoverpa zea* nucleopolyhedrovirus ABA-NPV-U (e. g. Heligen® from AgBiTech Pty Ltd., Queensland, Australia), *Heterorhabditis bacteriophora* (e. g. Nemasys® G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97™ or PreFeRal® from Certis LLC, USA), *Metarhizium anisopliae* var. *anisopliae* F52 also called 275 or V275 isolated from codling moth in Austria (DSM 3884, ATCC 90448; e. g. Met52® Novozymes Biologicals BioAg Group, Canada), *Metschnikowia fructicola* 277 isolated from grapes in the central part of Israel (US 6,994,849; NRRL Y-30752; e. g. formerly Shemer® from Agrogreen, Israel), *Paecilomyces ilacinus* 251 isolated from infected nematode eggs in the Philippines (AGAL 89/030550; WO1991/02051; Crop Protection 27, 352-361, 2008; e. g. BioAct®from Bayer CropScience AG, Germany and MeloCon® from Certis, USA), *Paenibacillus alvei*NAS6G6 isolated from the rhizosphere of grasses in South Africa at least before 2008
   (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P. epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva™ PN from Syngenta Crop Protection, LLC, USA), *Penicillium bilaiae* (also called *P. bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), *Reynoutria sachalinensis* extract (EP 0307510 B1; e. g. Regalia® SC from Marrone Biolnnovations, Davis, CA, USA or Milsana® from BioFa AG, Germany), *Steinernema carpocapsae* (e. g. Millenium® from BASF Agricultural Specialities Limited, UK), *S. feltiae* (e. g. Nemashield® from BioWorks, Inc., USA; Nemasys® from BASF Agricultural Specialities Limited, UK), *Streptomyces microffavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus® from BASF Agricultural Specialities (Pty) Ltd., South Africa), *T. harzianum* T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA).

According to one embodiment of the inventive mixtures, the at least one pesticide II is selected from the groups L1) to L5):
L1) Microbial pesticides with fungicidal, bactericidal, viricidal and/or plant defense activator activity: *Aureobasidium pullulans* DSM 14940 and DSM 14941 (L1.1), *Bacillus amyloliquefaciens*AP*-188* (L.1.2), *B. amyloliquefaciens* ssp. *plantarum* D747 (L.1.3), *B. amyloliquefaciens* ssp. *plantarum* FZB24 (L.1.4), *B. amyloliquefaciens* ssp. *plantarum* FZB42 (L.1.5), *B. amyloliquefaciens* ssp. *plantarum* MBI600 (L.1.6), *B. amyloliquefaciens* ssp. *plantarum* QST-713 (L.1.7), *B. amyloliquefaciens* ssp. *plantarum* TJ1000 (L.1.8), *B. pumilus* GB34 (L.1.9), *B. pumilus* GHA 180 (L.1.10), *B. pumilus* INR-7 (L.1.11), *B. pumilus* KFP9F (L.1.12), *B. pumilus* QST 2808 (L.1.13), *B. simplex* ABU 288 (L.1.14), *B. subtilis* FB17 (L.1.15), *Coniothyrium minitans* CON/M/91-08 (L.1.16), *Metschnikowia fructicola* NRRL Y-30752 (L.1.17), *Paenibacillus alvei* NAS6G6 (L.1.18), *P. epiphyticus*Lu17015 (L.1.25), *P. polymyxa* ssp. *plantarum* Lu16774 (L.1.26), *P. p.* ssp. *plantarum* strain Lu17007 (L.1.27), *Penicillium bilaiae*ATCC 22348 (L.1.19), *P. bilaiae*ATCC 20851 (L.1.20), *Penicillium bilaiae*ATCC 18309 (L.1.21), *Streptomyces microflavus* NRRL B-50550 (L.1.22), *Trichoderma asperelloides* JM41R (L.1.23), *T. harzianum* T-22 (L.1.24);
L2) Biochemical pesticides with fungicidal, bactericidal, viricidal and/or plant defense activator activity: harpin protein (L.2.1), *Reynoutria sachalinensis* extract (L.2.2);
L3) Microbial pesticides with insecticidal, acaricidal, molluscidal and/or nematicidal activity: *Bacillus firmus* I-1582 (L.3.1); *B. thuringiensis* ssp. *aizawai* ABTS-1857 (L.3.2), *B. t.* ssp. *kurstaki* ABTS*-*351 (L.3.3), *B. t.* ssp. *kurstaki* SB4 (L.3.4), *B. t.* ssp. *tenebrionis* NB-176-1 (L.3.5), *Beauveria bassiana* GHA (L.3.6), *B. bassiana* JW-1 (L.3.7), *B. bassiana* PPRI 5339 (L.3.8), *Burkholderia* sp. A396 (L.3.9), *Helicoverpa armigera* nucleopolyhedrovirus (HearNPV) (L.3.10), *Helicoverpazea* nucleopolyhedrovirus (HzNPV) ABA-NPV-U (L.3.11), *Helicoverpa zea* single capsid nucleopolyhedrovirus (HzSNPV) (L.3.12), *Heterohabditis bacteriophora* (L.3.13), *Isaria fumosorosea* Apopka-97 (L.3.14), *Metarhizium anisopliae* var. *anisopliae* F52 (L.3.15), *Paecilomyces lilacinus* 251 (L.3.16), *Pasteuria nishizawae* Pn1 (L.3.17), *Steinernema carpocapsae* (L.3.18), *S. feltiae* (L.3.19);
L4) Biochemical pesticides with insecticidal, acaricidal, molluscidal, pheromone and/or nematicidal activity: cis-jasmone (L.4.1), methyl jasmonate (L.4.2), Quillay extract (L.4.3);
L5) Microbial pesticides with plant stress reducing, plant growth regulator, plant growth promoting and/or yield enhancing activity: *Azospirillum brasilense* Ab-V5 and Ab-V6 (L.5.1), *A. brasilense* Sp245 (L.5.2), *Bradyrhizobium elkanii* SEMI A 587 (L.5.3), *B. elkanii* SEMIA 5019 (L.5.4), *B. japonicum* 532c (L.5.5), *B. japonicum* E-109 (L.5.6), *B. japonicum* SEMIA 5079 (L.5.7), *B. japonicum* SEMIA 5080 (L.5.8).

The present invention furthermore relates to agrochemical compositions comprising a mixture of at least one compound I (component 1) and at least one biopesticide selected from the group L) (component 2), in particular at least one biopesticide selected from the groups L1) and L2), as described above, and if desired at least one suitable auxiliary.

The present invention furthermore relates to agrochemical compositions comprising a mixture of of at least one compound I (component 1) and at least one biopesticide selected from the group L) (component 2), in particular at least one biopesticide selected from the groups L3) and L4), as described above, and if desired at least one suitable auxiliary.

Preference is also given to mixtures comprising as pesticide II (component 2) a biopesticide selected from the groups L1), L3) and L5), preferably selected from strains denoted above as (L.1.2), (L.1.3), (L.1.4), (L.1.5), (L.1.6), (L.1.7), (L.1.8), (L.1.10), (L.1.11), (L.1.12), (L.1.13), (L.1.14), (L.1.15), (L.1.17), (L.1.18), (L.1.19), (L.1.20), (L.1.21), (L.1.25), (L.1.26), (L.1.27), (L.3.1); (L.3.9), (L.3.16), (L.3.17), (L.5.1), (L.5.2), (L.5.3), (L.5.4), (L.5.5), (L.5.6), (L.5.7), (L.5.8); (L.4.2), and (L.4.1); even more preferably selected from (L.1.2), (L.1.6), (L.1.7), (L.1.8), (L.1.11), (L.1.12), (L.1.13), (L.1.14), (L.1.15), (L.1.18), (L.1.19), (L.1.20), (L.1.21), (L.3.1); (L.3.9), (L.3.16), (L.3.17), (L.5.1), (L.5.2), (L.5.5), (L.5.6); (L.4.2), and (L.4.1). These mixtures are particularly suitable for treatment of propagation materials, i. e. seed treatment purposes and likewise for soil treatment. These seed treatment mixtures are particularly suitable for crops such as cereals, corn and leguminous plants such as soybean.

Preference is also given to mixtures comprising as pesticide II (component 2) a biopesticide selected from the groups L1), L3) and L5), preferably selected from strains denoted above as (L1.1), (L.1.2), (L.1.3), (L.1.6), (L.1.7), (L.1.9), (L.1.11), (L.1.12), (L.1.13), (L.1.14), (L.1.15), (L.1.17), (L.1.18), (L.1.22), (L.1.23), (L.1.24), (L.1.25), (L.1.26), (L.1.27), (L.2.2); (L.3.2), (L.3.3), (L.3.4), (L.3.5), (L.3.6), (L.3.7), (L.3.8), (L.3.10), (L.3.11), (L.3.12), (L.3.13), (L.3.14), (L.3.15), (L.3.18), (L.3.19); (L.4.2), even more preferably selected from (L.1.2), (L.1.7), (L.1.11), (L.1.13), (L.1.14), (L.1.15), (L.1.18), (L.1.23), (L.3.3), (L.3.4), (L.3.6), (L.3.7), (L.3.8), (L.3.10), (L.3.11), (L.3.12), (L.3.15), and (L.4.2). These mixtures are particularly suitable for foliar treatment. These mixtures for foliar treatment are particularly suitable for vegetables, fruits, vines, cereals, corn, leguminous crops such as soybeans.

The mixtures of active substances can be prepared as compositions comprising besides the active ingredients at least one inert ingredient (auxiliary) by usual means, e. g. by the means given for the compositions of compounds I. Concerning usual ingredients of such compositions reference is made to the explanations given for the compositions containing compounds I.

According to one embodiment, the microbial pesticides selected from groups L1), L3) and L5) embrace not only the isolated, pure cultures of the respective microorganism as defined herein, but also its cell-free extract, its suspensions in a whole broth culture or as a metabolite-containing culture medium or a purified metabolite obtained from a whole broth culture of the microorganism.

When living microorganisms, such as pesticides II from groups L1), L3) and L5), form part of the compositions, such compositions can be prepared as compositions comprising besides the active ingredients at least one auxiliary by usual means (e. g. H.D. Burges: Formulation of Micobial Biopesticides, Springer, 1998). Suitable customary types of such compositions are suspensions, dusts, powders, pastes, granules, pressings, capsules, and mixtures thereof. Examples for composition types are suspensions, capsules, pastes, pastilles, wettable powders or dusts, pressings, granules, insecticidal articles, as well as gel formulations. Herein, it has to be taken into account that each formulation type or choice of auxiliary should not influence the viability of the microorganism during storage of the composition and when finally applied to the soil, plant or plant propagation material. Suitable formulations are e. g. mentioned in WO 2008/002371, US 6955,912, US 5,422,107.

Accordingly, the present invention furthermore relates to compositions comprising one compound I (component 1) and one pesticide II (component 2), which pesticide II is selected from the column "Co. 2" of the lines C-1 to C-584 of Table C.

A further embodiment relates to the compositions C-1 to C-348 listed in Table C, where a row of Table C corresponds in each case to a fungicidal composition comprising as active components one of the in the present specification individualized compounds of formula (I) (component 1) and the respective pesticide II from groups A) to O) (component 2) stated in the row in question. Preferably, the compositions described comprise the active components in synergistically effective amounts.

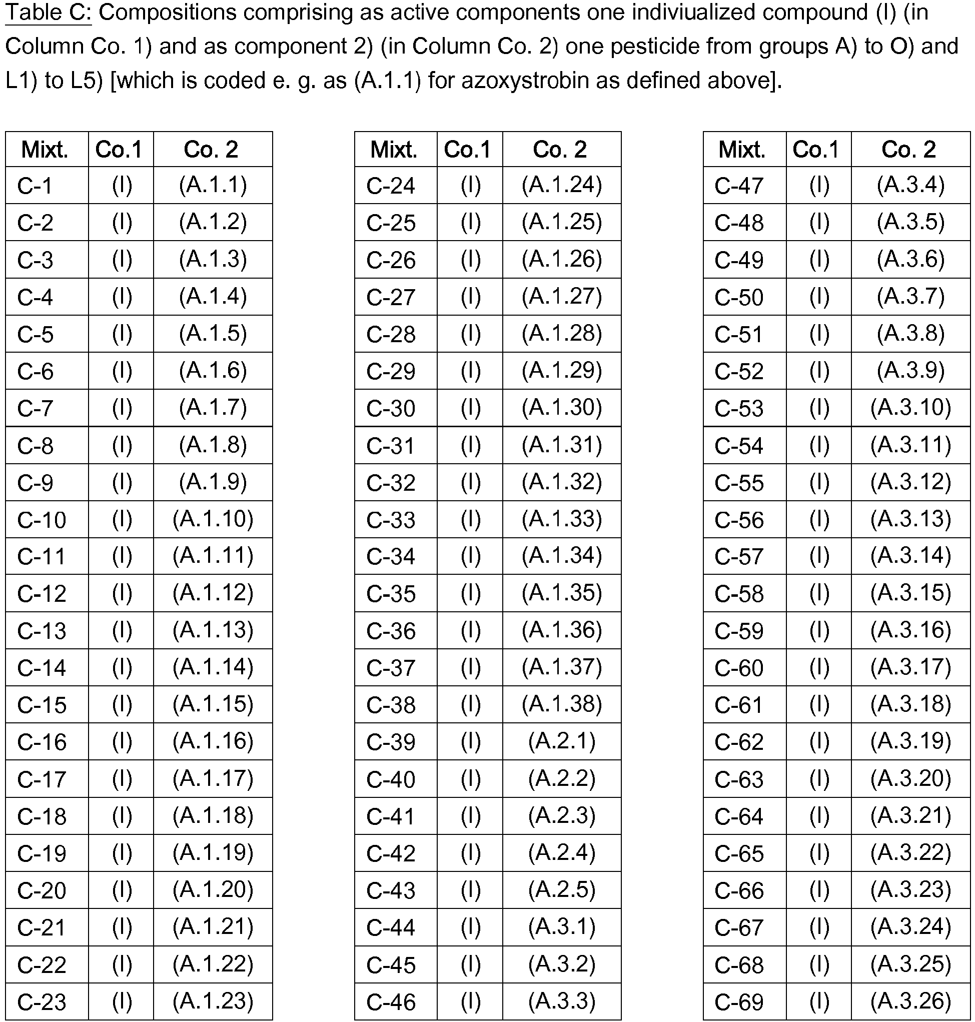

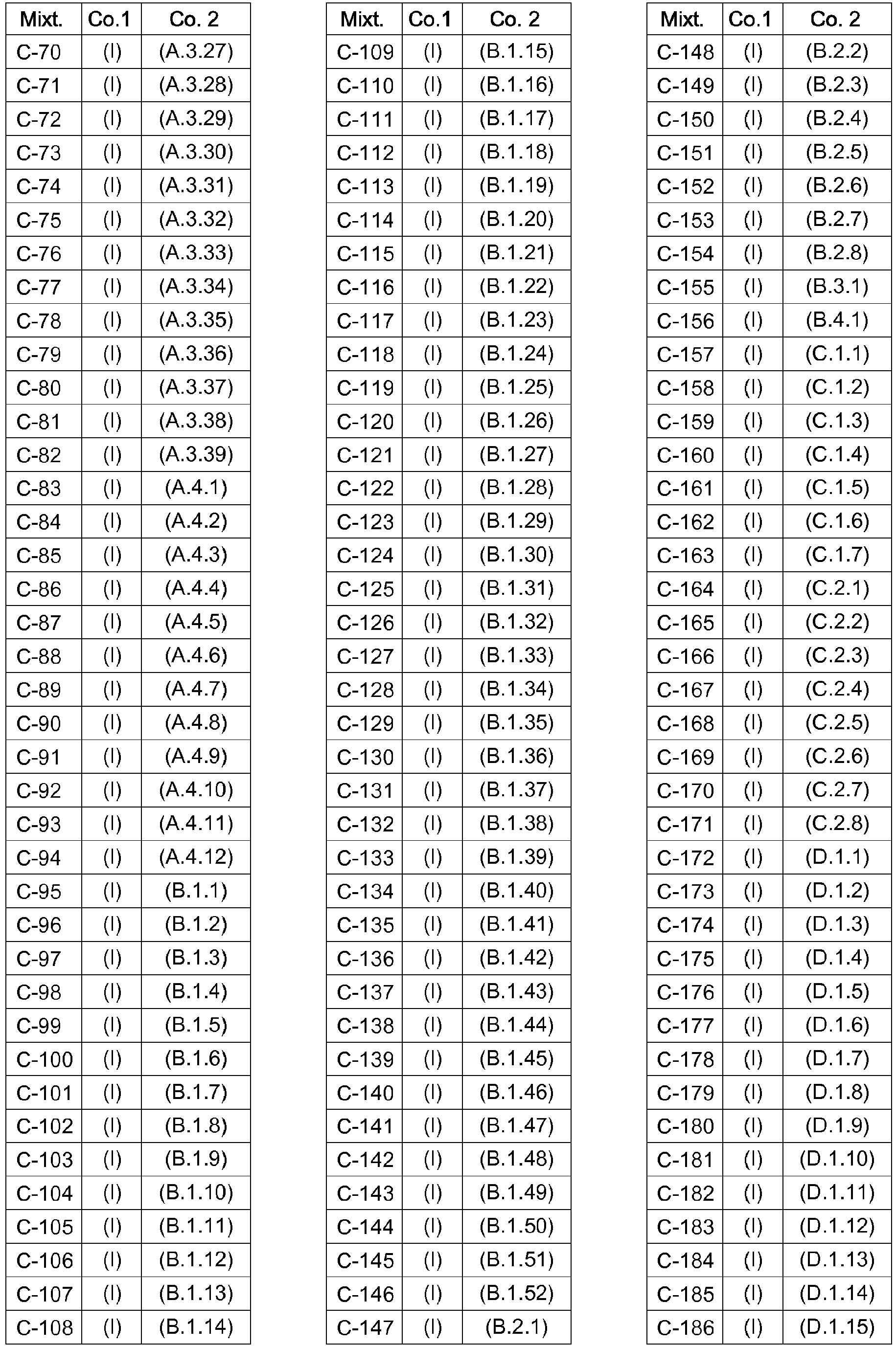

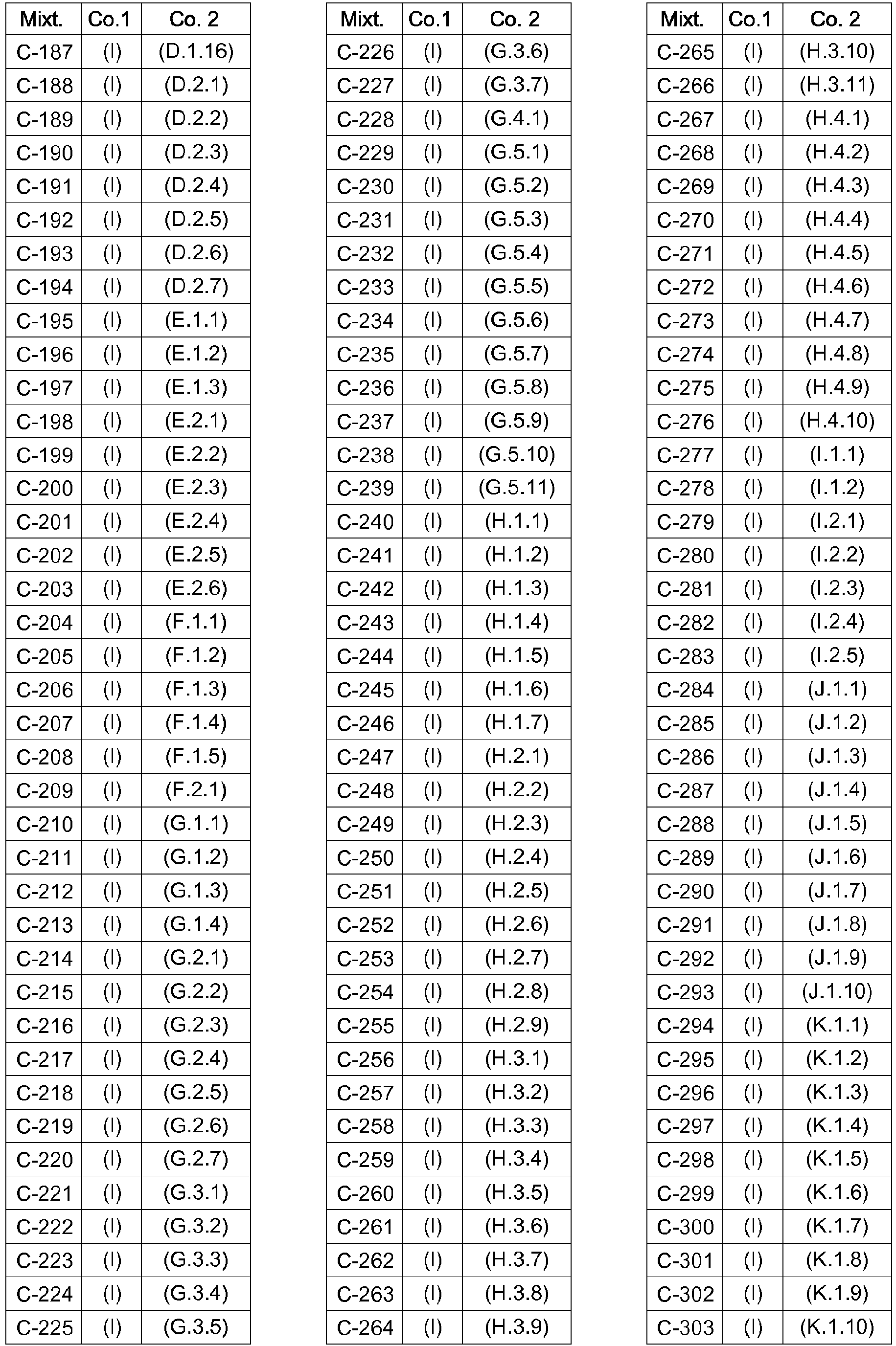

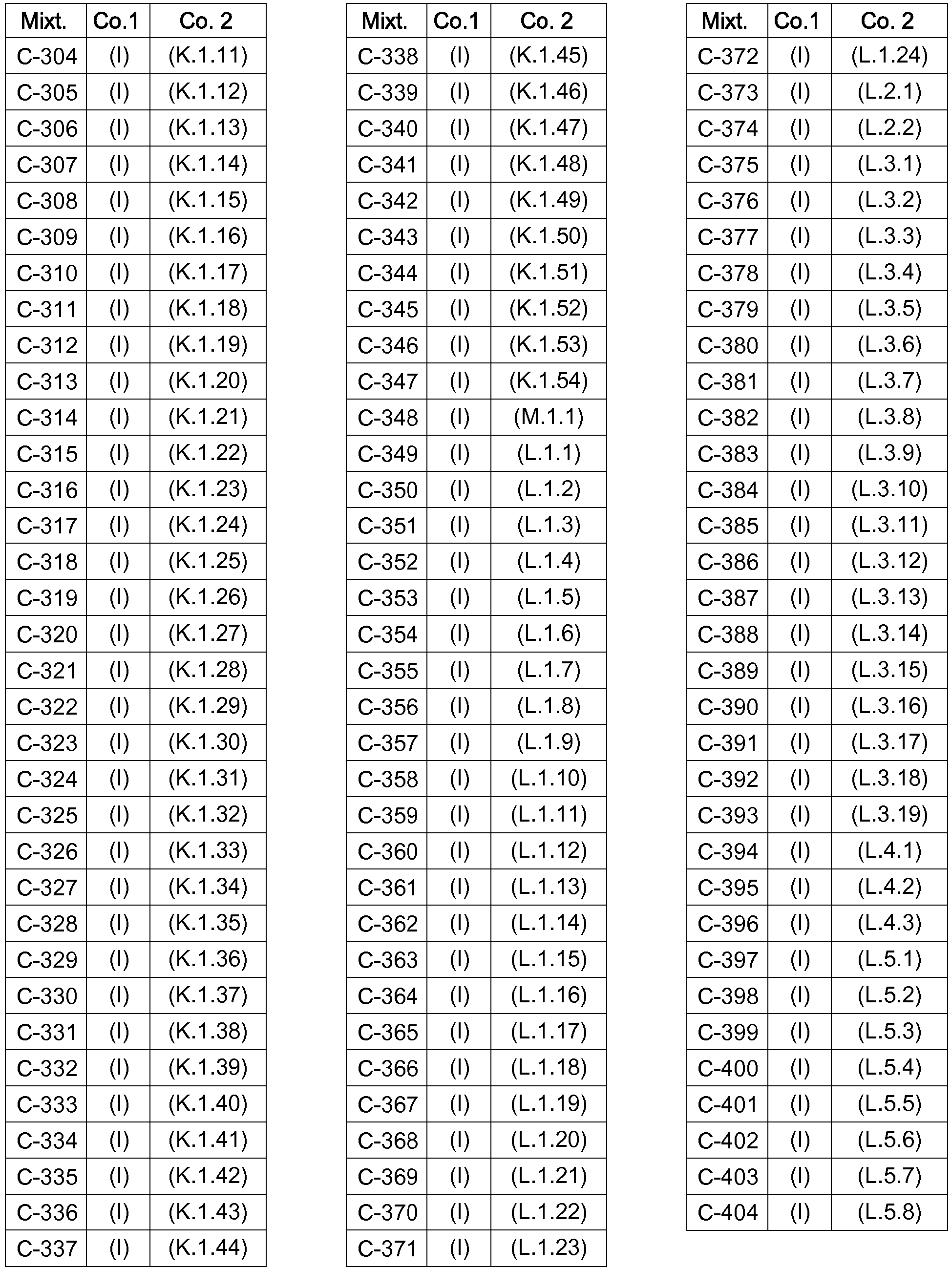

The mixtures of active substances can be prepared as compositions comprising besides the active ingredients at least one inert ingredient (auxiliary) by usual means, e. g. by the means given for the compositions of compounds I.

Concerning usual ingredients of such compositions reference is made to the explanations given for the compositions containing compounds I.

The mixtures of active substances according to the present invention are suitable as fungicides, as are the compounds of formula (I). They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

### I. Synthesis examples

### Example 1. Synthesis of 1,3-bis[(triethylsilyl)oxy]-2-propanone (III-1)

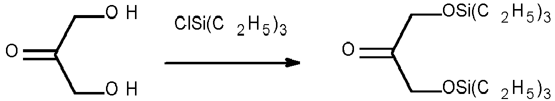

To a solution of dihydroxyacetone (200g, 2.2mol) and imidazole (302g, 4,4 mol) in DMF (2L) at 10 °C was added triethylsilylchloride (676g, 4.5mol) dropwise over 2 hours. The reaction was stirred at room temperature overnight, poured into 5 L ice water and extracted with methyl-tert-butyl ether (three times). The combined organic layers were washed with water (two times), brine, and dried over Na₂SO₄. The crude product was purified by distillation to provide the desired product (III-1) in 67% yield.

### Example 2. Synthesis of 2-[2-chloro-4-(difluoromethoxy)-phenyl]-1,2,3-propanetriol

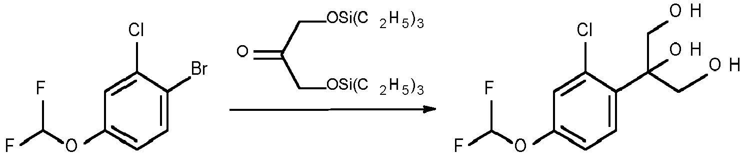

### Step a) Preparation of a Grignard solution

1-bromo-2-chloro-4-(difluoromethoxy)-benzene (200 g, 0,77mol) was dissolved in THF (100mL) and cooled to 5°C. A solution of turbo-Grignard (Aldrich, 1,3M, F2HCO 650 mL) was added rapidly via a dropping funnel during 10 min. Thereafter, the solution was stirred at 5°C for 30 min.

### Step b) Grignard reaction

A solution of 1,3-bis[(triethylsilyl)oxy]- 2-propanone prepared as described in the Example 1 (149g) in THF(200ml) was cooled to 0°C. Dry AlCl₃ (2,5g) was added to the solution and immediately afterwards, the Grignard solution from step a) was added during 15 min to the solution, maintaining the temperature below 10°C. The reaction mixture was warmed to room temperature and stirred for 1h. Thereafter, the reaction mixture was added to 1,5 kg ice and 500ml concentrated HCI, whereby the pH was kept below 1, and stirred for 20 min at room temperature. The reaction mixtures was extracted (3 times) eith methyl-tert-butyl ether. Th combined organic layers were washed with saturated aqueous solution of NaHCO₃ (1L) and dried over Na₂SO₄. The product was crystallized from diisopropylether/heptane and obtained in 71% yield (150g).

### Example 3. Synthesis of 2,2-dimethyl-4-hydroxymethyl-4-[2-chloro-4-(difluoromethoxy)-phenyl]-1,3-dioxolane

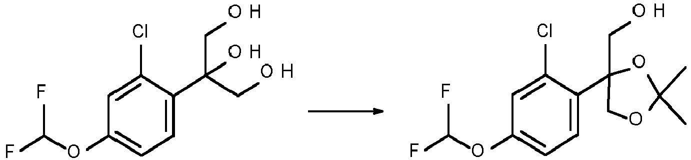

2-[2-chloro-4-(difluoromethoxy)-phenyl]-1,2,3-propanetriol (480g, 1,6 mol) was dissolved in acetone (2.2L) and then p-toluenesulfonic acid (3g) and Na₂SO₄ (50g) were added. The reaction mixture was stirred over night at room temperature. Thereafter, 50g of Na₂CO₃ were added and after 30 min, the reaction mixture was filtered. The combined organic phases were evaporated and the crude product crystallized from diisopropylether/heptane to obtain the target compound in 90% yield (540g).

### Example 4. Synthesis of 2,2-dimethyl-4-difluoromethoxymethyl-4-[2-chloro-4-(difluoromethoxy)-phenyl]-1,3-dioxolane

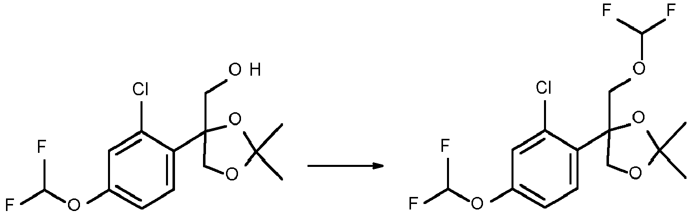

To a solution of 2,2-dimethyl-4-hydroxymethyl-4-[2-chloro-4-(difluoromethoxy)-phenyl]-1,3-dioxolane (318 g, 0,97 mol) in acetonitrile (2L) was added copper (I) iodide (56 g, 0,3 mol). The reaction mixture was heated to 65 °C, then fluorosulfonyldifluoroacetic acid (261 g, 1,47 mol) was added dropwise. The reaction was stirred at 60 °C for 30 min. The reaction mixture was then carefully added into a sodium bicarbonate solution and stirred for 30 min. The suspension was extracted twice with methyl-tert-butyl ether. The combined organic layers were washed with LiCI solution, dried over Na₂SO₄, and concentrated. The residue was taken up in 1 L methanol and 20 mL 30% sodium methoxide solution. The mixture was stirred for 5 min, then water was added and extraction was carried out with methyl-tert-butyl ether. The combined organic layers were washed with water, dried over Na₂SO₄, and concentrated. The product was purified by flash silica column chromatography. The product was obtained in a 69 % yield (248 g).

### Example 5. Synthesis of 2-[2-chloro-4-(difluoromethoxy)-phenyl]-3-difluoromethoxy-1,2-propanediol

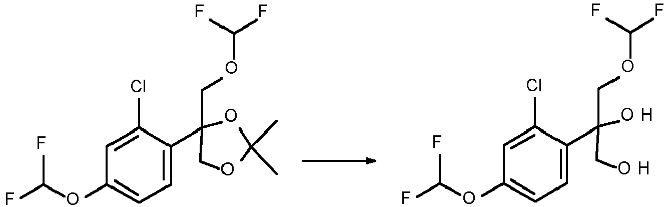

To a solution of 2,2-dimethyl-4-difluoromethoxymethyl-4-[2-chloro-4-(difluoromethoxy)-phenyl]-1,3-dioxolane (248g, 0,69mol) in water (750mL) and THF (2,2L) was added trifluoroacetic acid (750 mL). The reaction solution was stirred at 70 °C for 7 hours, then it was poured into a sodium carbonate solution and extracted with methyl-tert-butyl ether. The combined organic layers were dried over Na₂SO₄ and concentrated. The product was purified by flash silica column chromatography (product eluted with 1:4 methyl-tert-butyl ether /heptane). The crude product was provided in 45 % (112 g) which was used directly in the next reaction

### Example 6. Synthesis of α-[4-(difluoromethoxy)-2-chloro-phenyl]-α-difluoromethoxymethyl-1H-1,2,4-triazole-1-ethanol (I.A.D2-1.D1-170)

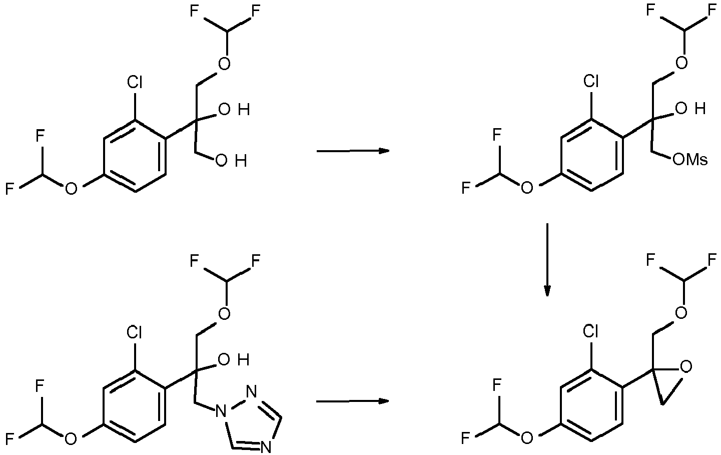

### Step a) Mesylate formation

To a solution of 2-[2-chloro-4-(difluoromethoxy)-phenyl]-3-difluoromethoxy-1,2-propanediol (112g, 0,32mol) in THF (1,1 L) was added Et₃N (240g, 1,6mol). The solution was cooled to 0 °C and methanesulfonylchloride (36,2g, 0,32mol) was added dropwise. The solution was stirred for 5 min then ammonium chloride solution was added. Thereafter the rection mixture was extracted with methyl-tert-butyl ether. The combined organic layers were washed with brine, dried over Na₂SO₄, and concentrated to provide crude which was used directly in the next reaction.

### Step b) Epoxide formation

To a solution of mesylate from Step a) in DMF (1L) at 0 C was added NaH (10g, 0,42 mol). The reaction was stirred for 30 min at room temperature. Then ammonium chloride solution was added and extraction was carried out with methyl-tert-butyl ether. The combined organic layers were washed with LiCI solution, dried over Na₂SO₄, and concentrated to provide 56g of crude which was used directly in the next reaction.

### Step c) Azole formation

The epoxide from step b) was dissolved in MeOH (600mL) and 1,2,4 triazole (61,1 g) and Na-OMe-solution (30%in MeOH, 79,9g) were added. The reaction mixture was heated to 50°C for 15h, HPLC control showed complete conversion. The reaction mixture was added to a mixture of saturated aqueous NH₄Cl-solution (1,5L), methyl-tert-butyl ether (1L) and ice (500g). After phase separation, the aqueous phase was extracted with methyl-tert-butyl ether (twice). The combined organic layers were died with Na₂SO₄. The product was precipated from diisopropylether/heptanes. The target compound was obtained as a white solid (57g).
MP: 67,3°C
HPLC: 2,836min [M+]

### Example 7. Synthesis of 2,2-dimethyl-4-[1,1,2,2-tetrafluoro-2-bromoethoxymethyl]-4-[2-chloro-4-(difluoromethoxy)-phenyl]-1,3-dioxolane

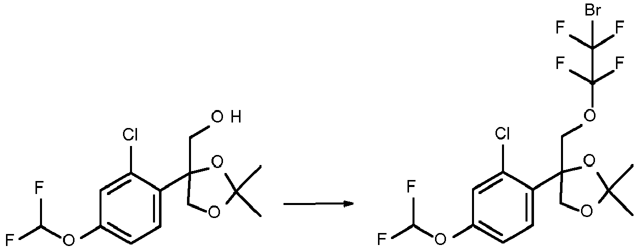

To a solution of 2,2-dimethyl-4-hydroxymethyl-4-[2-chloro-4-(difluoromethoxy)-phenyl]-1,3-dioxolane (240g, 0.7 mol) and 1,2-dibromotetrafluoroethane (272g, 1,05mol) in DMF (1,6L) at 10-20 °C was carefully added sodium hydride (42g, 1.75mol). The addition was carried out over 3 hours. Then, the reaction mixture was stirred at room temperature for 1 hour and thereafter poured carefully into ice water and extracted with methyl-tert-butyl ether. The combined organic layers was were washed with LiCI solution and brine, then dried over Na₂SO₄, and concentrated to provide the desired product in 88% yield (300 g).

### Example 8. Synthesis 2,2-dimethyl-4-[1,1,2,2-tetrafluoroethoxymethyl]-4-[2-chloro-4-(difluoromethoxy)-phenyl]-1,3-dioxolane

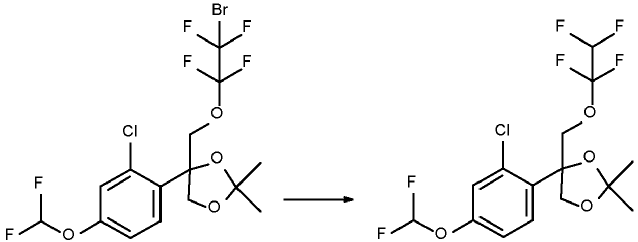

To a solution of 2,2-dimethyl-4-[1,1,2,2-tetrafluoro-2-bromoethoxymethyl]-4-[2-chloro-4-(difluoromethoxy)-phenyl]-1,3-dioxolane (300g) in methanol (1L) under argon was carefully added 10% palladium on activated carbon (15g) followed by ammonium formate (175g). The reaction was slowly heated to 50 °C and stirred for 2 hours. The catalyst was filtered off and MeOH was evaporated. The residue was redissolved in methyl-tert-butyl ether and water, then extracted with methyl-tert-butyl ether. The combined organic layers were washed with water, dried over Na₂SO₄, and concentrated to provide 250 g of crude product (90% purity) which was used directly in the next reaction.

### Example 9. Synthesis of 2-[2-chloro-4-(difluoromethoxy)-phenyl]-3-(1,1,2,2-tetrafluoroethoxy)-1,2-propanediol

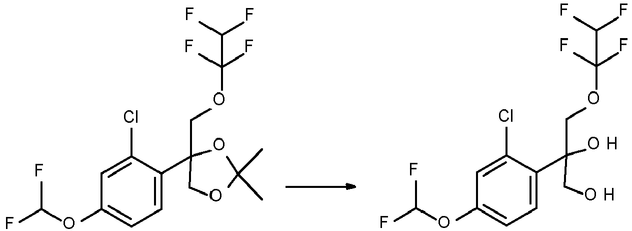

A solution of 2,2-dimethyl-4-(1,1,2,2-tetrafluoroethoxymethyl)-4-[2-chloro-4-(difluoromethoxy)-phenyl]-1,3-dioxolane (250g) and 15% HCl (2L) in isopropanol (1L) was heated to reflux until HPLC indicated full conversion. The reaction mixture was evaporated and the residue was taken up in ethyl ether, washed with sodium bicarbonate solution, dried over Na₂SO₄, and concentrated to provide 137g of the crude product (95% purity) that was used directly in the next reaction.

### Example 10. Synthesis of α-[4-(difluoromethoxy)-2-chloro-phenyl]-α-(1,1,2,2-tetrafluoroethoxymethyl)-1H-1,2,4-triazole-1-ethanol (I.A.D2-1.D1-82)

### Step a) Mesylate formation

To a solution of 2-[2-chloro-4-(difluoromethoxy)-phenyl]-3-difluoromethoxy-1,2-propanediol (137g) in THF (1L) was added trithylamine (179g). The solution was cooled to 0 °C and methanesulfonylchloride (40g) was added dropwise. The solution was stirred for 5 min then ammonium chloride solution was added followed by extraction with methyl-tert-butyl ether. The combined organic layers were washed with brine, dried over Na₂SO₄, and concentrated to provide 170g of crude product which was used directly in the next reaction.

### Step b) Epoxide formation

To a solution of mesylate from Step a) (170g) in DMF (1L) at 0 C was carefully added NaH (9,9g). The reaction was stirred for 30 min at room temperature, then ammonium chloride solution was added and extraction was carried out with methyl-tert-butyl ether. The combined organic layers were washed with LiCl solution, dried over Na₂SO₄, and concentrated to provide 145g of crude product which was used directly in the next reaction.

### Step c) Azole formation

The epoxide from step b) was dissolved in MeOH (1,2L) and 1,2,4 triazole (117g) and NaOMe-solution (30% in MeOH, 153g) were added. The reaction mixture was heated to 50°C for 15h, HPLC control showed complete conversion. The reaction mixture was added to a mixture of saturated aqueous NH₄Cl-solution (1,5L), methyl-tert-butyl ether (1L) and ice (500g). After phase separation, the aqueous phase was extracted with methyl-tert-butyl ether (twice). The combined organic layers were died with Na₂SO₄. The product was precipated from diisopropylether/heptanes. The target compound was obtained as a white solid (82g).
MP: 127°C
HPLC: 3,146 min [M+]

Examples 11-22. Further compounds of the Formula (I.A)were prepared according to the procedures described in the examples 1 to 10.

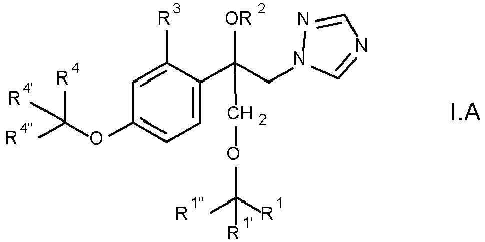

| **Example** | **Compound No.** | **R¹** | **R^{1'}** | **R^{1"}** | **R²** | **R³** | **R⁴** | **R^{4'}** | **R^{4"}** |
|---|---|---|---|---|---|---|---|---|---|
| 11 | I.A.D2-1.D1-75 | H | F | F | H | Cl | F | F | F |
| 12 | I.A.D2-1.D1-45 | H | F | F | H | Cl | H | H | CF₃ |
| 13 | I.A.D2-1.D1-170 | CHF₂ | F | F | H | Cl | F | F | H |
| 14 | I.A.D2-1.D1-163 | CHF₂ | F | F | H | Cl | F | F | F |
| 15 | I.A.D2-1.D1-133 | CHF₂ | F | F | H | Cl | H | H | CF₃ |
| 16 | I.A.D2-2.D1-163 | CHF₂ | F | F | H | F | F | F | F |
| 17 | I.A.D2-2.D1-170 | CHF₂ | F | F | H | F | F | F | H |
| 18 | I.A.D2-2.D1-133 | CHF₂ | F | F | H | F | H | H | CF₃ |
| 19 | I.A.D2-1.D1-159 | CHF₂ | F | F | H | Cl | H | H | SCH₃ |
| 20 | I.A.D2-1.D1-176 | CHF₂ | F | F | H | Cl | =HCl | | Cl |
| 21 | I.A.D2-1.D1-160 | CHF₂ | F | F | H | Cl | H | H | S(O)CH₃ |
| 22 | I.A.D2-1.D1-175 | CHF₂ | F | F | H | Cl | =CFCl | | F |

### Example 23. Synthesis of 2,2-difluoro-2-(2,2,2-trifluoroethoxy)-1-[2-chloro-4-difluoromethoxyphenyl]-ethanone

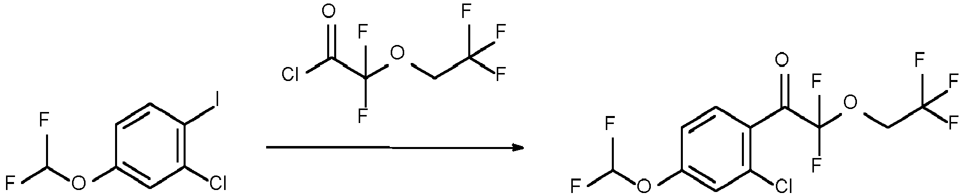

To a solution of 1-iodo-2-chloro-4-(difluoromethoxy)-benzene (1.9 g, 6.3 mmol) in THF (20 mL) was added iPrMgCl·LiCl (6.2 mL, 8.1 mmol) dropwise at -40°C under N₂ and the mixture was stirred for 20 min. The reaction mixture was quickly added to the solution of 2,2-difluoro-2-(2,2,2-trifluoroethoxy)-acetyl chloride (2 g, 9.4 mmol), CuCI (92 mg, 0.93 mmol) and LiCI (78 mg, 1.86 mmol) in THF (20 mL) at -20 °C and the mixture was stirred at 25°C for 30 min. The reaction mixture was quenched with aq. NH₄Cl (50 mL), extracted with methyl-tert-butyl ether (20 mLx2) and the organic layer was washed with aq. NaHCO₃ (100 mL ×2) and brine (100 mL x2), dried over Na₂SO₄, concentrated and the residue was purified by column chromatography (petrol ether: methyl-tert-butyl ether =33:1) to give the product (460 mg, 27%) as yellow oil.

### Example 24. Synthesis of α-[4-(difluoromethoxy)-2-chloro-phenyl]-α-[1,1-difluoro-1-(2,2,2-trifluoroethoxy)-methyl]-1H-1,2,4-triazole-1-ethanol (I.B.D2-1.D1-38)

### Step a) Epoxide formation

To a solution of 2,2-difluoro-2-(2,2,2-trifluoroethoxy)-1-[2-chloro-4-difluoromethoxyphenyl]-ethanone (446 mg, 2 mmol) in DMSO (14 mL) was added t-BuOK (227 mg, 2 mmol) at 20 °C under N₂ and the mixture was stirred at 20°C for 30 min. Trimethylsulfonium iodide (360 mg, 1 mmol) in DMSO was added dropwise and the mixture was stirred at 20 °C for 16 h. The reaction mixture was diluted with brine (50 mL), extracted with methyl-tert-butyl ether (30 mLx2) and the organic layer was washed with brine (50 mLx2), dried over Na₂SO₄ and concentrated. The residue was purified by column (petrol ether: methyl-tert-butyl ether = 50:1) to give the epoxide (170 mg, 46 %) as yellow oil.

### Step c) Azole formation

The solution of epoxide from step b) (170 mg, 0.46 mmol) in DMF (11 mL) was added 1,2,4 triazole (64 mg, 0.92 mmol) and CS₂CO₃ (118 mg, 0.69 mmol) under N₂ at 25 °C and the mixture was heated to 80 °C for 16 h. The reaction mixture was quenched with brine (50mL), extracted with EtOAc (30 mL x 2) and the organic layer was washed with brine (80 mLx2), dried over Na₂SO₄, concentrated and the residue was purified by column (petrol ether:EtOAc =1:1) to give the product (163 mg, 81%) as yellow solid.

### Examples 23-27

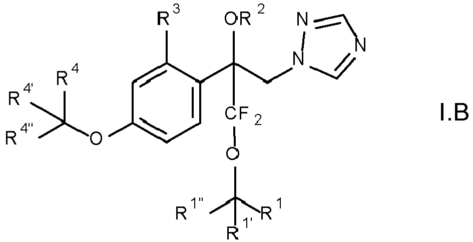

The following compounds of the formula (I.B) were prepared as described in the examples 23 and 24.

| **Example** | **Compound No.** | **R¹** | **R^{1'}** | **R^{1"}** | **R²** | **R³** | **R⁴** | **R^{4'}** | **R^{4"}** |
|---|---|---|---|---|---|---|---|---|---|
| 23 | I.B.D2-1.D1-31 | H | CF₃ | H | H | Cl | F | F | F |
| 25 | I.B.D2-1.D1-119 | CHF₂ | H | H | H | Cl | F | F | F |
| 26 | I.B.D2-1.D1-126 | CHF₂ | H | H | H | Cl | F | F | H |
| 27 | I.B.D2-1.D1-1 | H | CF₃ | H | H | Cl | H | H | CF₃ |

### II. Biological examples

### Microtest

The active compounds were formulated separately as a stock solution having a concentration of 10000 ppm in dimethyl sulfoxide.

### Green House

A mixture of acetone and/or dimethylsulfoxide and the wetting agent/emulsifier wettol, which is based on ethoxylated alkylphenoles, in a relation (volume) solvent-emulsifier of 99 to 1 was added to the compound to give a total of 5 ml. Water was then added to the total volume of 100 ml. This stock solution was diluted with a solvent-emulsifier-water mixture to the given concentration.

### 1. Control of culm rot on pearl millet caused by Fusarium culmorum (Fusacu K1)

Pot-grown pearl millet seedlings were inoculated with a spore suspension of *Fusarium culmorum* in an aqueous biomalt solution. Then the trial plants were immediately transferred to a humid chamber with 23-25°C and a relative humidity close to 100%. The next day the plants were sprayed to run-off with an aqueous suspension, containing the concentration of active ingredient or their mixture as described below. The plants could air-dry and were transferred to the humid chamber After 5 days the extent of fungal attack on the leaves was visually assessed as % diseased leaf area.

### 2. Curative control of soy bean rust on soy beans caused by Phakopsora pachyrhizi (Phakpa K4)

Leaves of pot-grown soy bean seedlings were inoculated with spores of *Phakopsora pachyrhizi.* To ensure the success of the artificial inoculation, the plants were transferred to a humid chamber with a relative humidity of about 95% and 20 to 24°C for 24 h. The next day the plants were cultivated for 3 days in a greenhouse chamber at 23-27°C and a relative humidity between 60 and 80 %. Then the plants were sprayed to run-off with an aqueous suspension, containing the concentration of active ingredient or their mixture as described below. The plants were allowed to air-dry. Then the trial plants were cultivated for 14 days in a greenhouse chamber at 23-27°C and a relative humidity between 60 and 80%. The extent of fungal attack on the leaves was visually assessed as % diseased leaf area.

### 3. Protective control of soy bean rust on soy beans caused by Phakopsora pachyrhizi (Phakpa P6)

Leaves of pot-grown soy bean seedlings were sprayed to run-off with an aqueous suspension, containing the concentration of active ingredient or their mixture as described below. The plants were allowed to air-dry. The trial plants were cultivated for 6 days in a greenhouse chamber at 23-27°C and a relative humidity between 60 and 80%. Then the plants were inoculated with spores of *Phakopsora pachyrhizi.* To ensure the success the artificial inoculation, the plants were transferred to a humid chamber with a relative humidity of about 95 % and 20 to 24°C for 24 h. The trial plants were cultivated for fourteen days in a greenhouse chamber at 23-27°C and a relative humidity between 60 and 80%. The extent of fungal attack on the leaves was visually assessed as % diseased leaf area.

### 4. Protective control of soy bean rust on soy beans caused by Phakopsora pachyrhizi(Phakpa P10)

Leaves of pot-grown soy bean seedlings were sprayed to run-off with an aqueous suspension, containing the concentration of active ingredient or their mixture as described below. The plants were allowed to air-dry. The trial plants were cultivated for 10 days in a greenhouse chamber at 23-27°C and a relative humidity between 60 and 80 %. Then the plants were inoculated with spores of *Phakopsora pachyrhizi.* To ensure the success the artificial inoculation, the plants were transferred to a humid chamber with a relative humidity of about 95 % and 20 to 24°C for 24 h. The trial plants were cultivated for fourteen days in a greenhouse chamber at 23-27°C and a relative humidity between 60 and 80%. The extent of fungal attack on the leaves was visually assessed as % diseased leaf area.

### 5. Curative control of brown rust on wheat caused by Puccinia recondita (Puccrt K4)

The first two developed leaves of pot-grown wheat seedling were dusted with spores of *Puccinia recondita.* To ensure the success the artificial inoculation, the plants were transferred to a humid chamber without light and a relative humidity of 95 to 99 % and 20 to 24°C for 24 h. The next day the plants were cultivated for 3 days in a greenhouse chamber at 20-24°C and a relative humidity between 65 and 70%. Then the plants were sprayed to run-off with an aqueous suspension, containing the concentration of active ingredient or their mixture as described below. The plants could air-dry. Then the trial plants were cultivated for 8 days in a greenhouse chamber at 20-24°C and a relative humidity between 65 and 70%. The extent of fungal attack on the leaves was visually assessed as % diseased leaf area.

**Table Z. Biological activity**

| **Compound** | **Disease (%) at 16ppm** | | | | |
|---|---|---|---|---|---|
| | **Fusacu K1** | **Phakpa K4** | **Phakpa P6** | **Phakpa P10** | **Puccrt K4** |
| I.A.D2-1.D1-82 | 7 | 7 | 2 | 6 | 0 |
| I.A.D2-1.D1-75 | - | 0 | 0 | 0 | 0 |
| I.A.D2-1.D1-45 | 17 | - | 5 | 5 | 16 |
| I.A.D2-1.D1-170 | 3 | 1 | 0 | 0 | 1 |
| I.A.D2-1.D1-163 | 7 | 0 | 0 | 0 | 0 |
| I.A.D2-1.D1-133 | 5 | - | 1 | 0 | 1 |
| I.A.D2-2.D1-163 | 15 | 1 | 0 | 0 | 7 |
| I.A.D2-2.D1-170 | - | 3 | 1 | 1 | - |
| I.A.D2-2.D1-133 | - | - | - | 8 | - |
| I.B.D2-1.D1-31 | - | 2 | 1 | 0 | 0 |
| I.B.D2-1.D1-38 | - | 0 | 0 | 0 | 0 |
| I.B.D2-1.D1-126 | - | 0 | 0 | 0 | 0 |
| I.B.D2-1.D1-1 | - | - | 1 | 1 | 13 |
| I.A.D2-1.D1-176 | - | - | 2 | 1 | - |
| I.A.D2-1.D1-175 | - | 14 | 0 | 0 | - |
| Untreated control | 80 | 100 | 80 | 80 | 90 |

## Claims

1. Compounds of the formula I 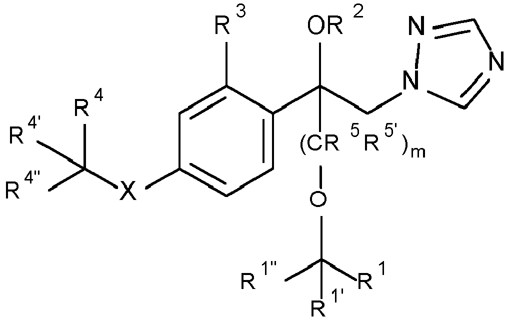 wherein
R¹, R^{1'} and R^{1"} are independently of one another selected from hydrogen, halogen, C₁-C₄-alkyl, C₁-C₄-halogenalkyl, C₂-C₄-alkenyl, C₂-C₄-halogenalkenyl, C₂-C₄-alkynyl, or C₂-C₄-halogen-alkynyl,
provided that if any two of R¹, R^{1'} and R^{1"} are hydrohens, the third one is not hydrogen or alkyl; and
provided that if any two of R¹, R^{1'} and R^{1"} are halogens, the third one is not halogen;
R² is hydrogen, C₁-C₄-alkyl, C₂-C₄-alkenyl or C₂-C₄-alkynyl; wherein the aliphatic moieties of R² are unsubstituted or carry one, two, three or up to the maximum possible number of identical or different groups R^{2a}:
R^{2a} is independently of one another selected from halogen, OH, CN, C₁-C₄-alkoxy, C₃-C₆-cycloalkyl, C₃-C₆-halogencycloalkyl and C₁-C₄-halogenalkoxy;
R³ is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein p is 0, 1 or 2, and
wherein each of R³ is unsubstituted or further substituted by one, two, three or four R^{3a}:
R^{3a} is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
R⁴, R^{4'}, and R^{4"} are independently of one another selected from hydrogen, halogen, C₁-C₆-alkyl, C₂-C₆-alkenyl, C₂-C₆-alkynyl, C₁-C₄-alkoxy, C₃-C₆-cycloalkyl, C₃-C₆-cycloalkenyl, C₃-C₆-cycloalkyl-C₁-C₄-alkyl, -N(R^{A})₂, C₃-C₆-halogencycloalkyl, aryl and aryloxy; wherein the aliphatic moieties of R⁴, R^{4'}, and R^{4"} are unsubstituted or further substituted by one, two, three or four of identical or different groups R^{4a}:
R^{4a} is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is C₁-C₄-alkyl;
wherein the cycloalkyl moieties of R⁴, R^{4'}, and R^{4"} are unsubstituted or carry one, two, three, four, five or up to the maximum number of identical or different groups R^{4b}:
R^{4b} is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy; wherein the aryl and aryloxy moieties of R⁴, R^{4'}, and R^{4"} are unsubstituted or further substituted by one, two, three or four of identical or different groups R^{4c}:
R^{4c} is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and wherein
R^{A} is independently of one another selected from C₁-C₄-alkyl, C₁-C₄-halogenalkyl and -C(O)O-C₁-C₄-alkyl;
or
R⁴ and R^{4'} together are =C(R^{4a})₂, and R" is as defined above;
R⁵ and R^{5'} are independently of one another selected from hydrogen or halogen;
m is 1 or 2; X is O, S(O)n, wherein n is 0, 1 or 2, or NR^{N};
R^{N} is selected from hydrogen, C₁-C₆-alkyl, C₂-C₆-alkenyl, C₂-C₆-alkynyl, C₁-C₆-alkoxy,-C(O)C₁-C₆-alkyl, C₃-C₆-cycloalkyl, C₃-C₆-cycloalkyl-C₁-C₄-alkyl, -S(O)2- C₁-C₆-alkyl and -S(O)₂-aryl,
wherein R^{N} is unsubstituted or further substituted by one, two, three or four of identical or different groups R^{Na}:
R^{Na} is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and the N-oxides and the agriculturally acceptable salts thereof.

2. The compounds of claim 1, weherein m is 1.

3. The compounds of claims 1 or 2, wherein R⁵ and R^{5'} both are hydrogens.

4. The compounds of any one of claims 1 to 3, wherein R¹ is hydrogen or C₁-C₄-halogenalkyl.

5. The compounds of any one of claims 1 to 4, wherein R¹ is selected from hydrogen, CHF₂, CF₂H, or CF₃.

6. The compounds of any one of claims 1 to 5, wherein R^{1'} and R^{1"} both are halogens.

7. The compounds of any one of claims 1 to 6, wherein R² is hydrogen.

8. The compounds of any one of claims 1 to 7, wherein R³ is selected from halogen or C₁-C₄-halogenalkyl.

9. The compounds of any one of claims 1 to 8, wherein X is O.

10. The compounds of any one of claims 1 to 9, wherein R⁴, R^{4'}, and R^{4"} are halogens.

11. A composition, comprising a compound of formula (I), as defined in any of the claims 1 to 10, an N-oxide or an agriculturally acceptable salt thereof.

12. The composition of claim 11, comprising additionally a further active substance.

13. A use of a compound of the formula (I), as defined in any of the claims 1 to 10, and/or of an agriculturally acceptable salt thereof or of the compositions, as defined in any of the claims 11 or 12, for combating phytopathogenic fungi.

14. A method for combating harmful fungi, comprising treating the fungi or the materials, plants, the soil or seeds to be protected against fungal attack with an effective amount of at least one compound of formula (I), as defined in any of the claims 1 to 10, or with a composition, as defined in any of the claims 11 or 12.

15. Seed, coated with at least one compound of the formula (I), as defined in any of the claims 1 to 10, and/or an agriculturally acceptable salt thereof or with a composition, as defined in any of the claims 11 or 12, in an amount of from 0.1 to 10 kg per 100 kg of seed.
